# EUROPEAN PATENT SPECIFICATION

(11) **EP 3 715 350 B1**
(45) Date of publication and mention of the grant of the patent: **08.05.2024**
(21) Application number: 18891652.2
(22) Date of filing: 10.12.2018
(51) Int. Cl.: C07F 9/6558, C07F 9/6561, A61K 31/675, A61P 35/00, A61P 35/02

(54) **ARYLPHOSPHINE OXIDES FOR INHIBITING KINASE ACTIVITY**
ARYLPHOSPHINOXIDE ZUR HEMMUNG DER KINASEAKTIVITÄT
OXYDES D'ARYLPHOSPHINE AGISSANT EN TANT QU'INHIBITEURS DE L'ACTIVITÉ KINASE

(30) Priority: 21.12.2017 CN 201711394508
(43) Date of publication of application: 30.09.2020
(73) Proprietor: Shenzhen TargetRx, Inc., Shenzhen, Guangdong 518057 (CN)
(72) Inventor: WANG, Yihan, Shenzhen, Guangdong 518057 (CN); LI, Huanyin, Shenzhen, Guangdong 518057 (CN)
(74) Representative: Novitas Patent AB
(86) International application number: PCT/CN2018/120089
(87) International publication number: WO 2019/120094

(56) References cited:
- EP-A1- 3 165 530
- WO-A1-2016/000581
- CN-A- 1 788 001
- CN-A- 101 687 822
- CN-A- 102 105 150
- CN-A- 106 699 810

## Description

### Field of the invention

The present disclosure belongs to the technical filed of medicine. Specifically, the present disclosure relates to arylphosphine oxides with inhibitory activity against the protein tyrosine kinase, pharmaceutical compositions containing the same as well as the preparation method and the use thereof.

### Background of the invention

Anaplastic lymphoma kinase (ALK) is a receptor-type protein tyrosine kinase that belongs to the insulin receptor superfamily. It was discovered by Morris and Shiota et al. in 1994 as a product of chromosome rearrangement in anaplastic large cell lymphoma (ALCL). The most frequently found fusion is the one that the NPM (Nucleophosmin) gene on chromosome 5 fuses with the ALK gene on chromosome 2. NPM-ALK fusion protein was detected in nearly 75% of ALK-positive ALCL patients. It was found in subsequent studies that different forms of ALK fusion were present in various cancers, including inflammatory myofibroblastoma and diffuse large B-cell lymphoma. Nevertheless, the importance of ALK kinase as an effective target for anti-tumor drugs has not been fully recognized until in 2007 Soda et al. discovered that the occurrence rate of the EML4-ALK fusion protein in non-small-cell lung cancer (NSCLC) is 5%, and then the importance of ALK kinase as a target for anti-tumor drugs was highlighted. This is because among tremendous cancer patients worldwide, the lung cancer ranks the first. There are more than 8,000 new ALK-positive lung cancer cases each year in the United States, while the new cases each year in China are more than 65,000. The global 5-year survival rate for lung cancer is only 15%. It is interesting to note that EML4-ALK gene-positive patients generally do not carry epidermal growth factor receptor (EGFR) or Kirsten rat sarcoma virus (KRAS) mutations, which makes EML4-ALK fusion gene the unique molecular target for non-small cell lung cancer. In addition, amplification or point mutations of the ALK gene have been found in neuroblastomas, anaplastic thyroid cancer and ovarian cancer.

The first small molecule inhibitor Crizotinib (Xalkori), developed by Pfizer, targets the ALK fusion gene and is the first-generation ALK inhibitors. However, although Crizotinib achieved an objective response rate of 60-74% and a good median progression-free survival (8-11 months) in patients with ALK + NSCLC, most patients experienced disease relapse after 1 year of treatment, that is acquired resistance was generated. The mechanism of the acquired resistance to Crizotinib has also been identified, including the gene gain of the ALK fusion gene, activation of the signaling pathway, secondary mutations in the ALK kinase region, and other mechanisms. About 40% of ALK-positive patients have no objective response at the beginning of receiving Crizotinib therapy, and 1/3 of Crizotinib-resistant patients will undergo secondary mutations that induce secondary resistance.

There are several second-generation ALK inhibitors that may effectively overcome the shortcomings of resistance to Crizotinib therapy, such as Ceritinib (Zykadia, Novartis) and Alectinib (Alecensa, Roche). However, although these second-generation inhibitors may effectively overcome most of Crizotinib-resistant mutations, they are still not effective for some mutations, for example, Ceritinib still has no effect on F 1174C/V, Alectinib has no effect on I1171N/T/S, and neither of them has effect on G1202R. So there is an urgent need for developing novel, more effective and safe ALK inhibitors. Some spirocyclic aryl phosphorus oxide compounds intended for use in treating cancers caused by ALK enzyme abnormalities are disclosed in EP3165530.

### Summary of the invention

The present disclosure provides a new arylphosphine oxide and a pharmaceutical composition including the same as well as the preparation method and use thereof, which has better ALK kinase inhibitory activity, and high selectivity for drug resistance mutation L1196M, and thus is useful in treating, preventing and alleviating diseases mediated by ALK kinase.

The present invention relates to the compounds presented in the appended claims.

In the first aspect of the present disclosure, the compound of formula (I) is provided: 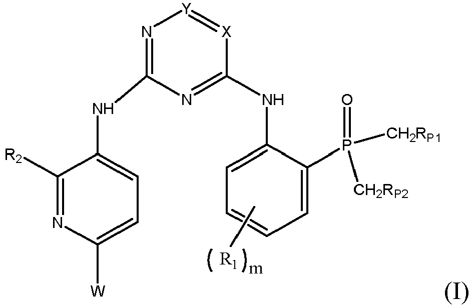 wherein,
X is selected from CRx or NR_{X1};
Y is selected from CR_{Y} or NR_{Y1};
m is selected from 0, 1, 2, 3 or 4;
R₁, R_{X} and R_{Y} are independently selected from:
   1) H, halogen, -CN, -OH, -NO₂ or -NH₂;
   2) C₁-C₆ alkyl, C₁-C₆ haloalkyl, C₁-C₆ alkoxy, C₁-C₆ haloalkoxy, C₁-C₆ alkylthio, C₁-C₆ alkylamino, C₁-C₆ alkanoyl, 3- to 7-membered carbocyclyl or 3- to 8-membered heterocyclyl; wherein the said group is optionally substituted by one or more Rₐ; or
   3) R_{X1} and R_{Y1} are absent; or two adjacent substituents selected from R_{X}, R_{X1}, R_{Y} and R_{Y1}, or two adjacent R₁ groups, together with the atoms to which they are attached may form a fused 5- to 7-membered ring, which contains 0 to 4 heteroatoms selected from N, O and S and may be substituted by one or more Rₐ;
R_{P1} and R_{P2} are independently H;
R₂ is selected from C₁-C₆ alkoxy, C₁-C₆ haloalkoxy, C₂-C₆ alkenyloxy or 3- to 7-membered cycloalkyloxy; wherein the said group is optionally substituted by one or more Rₐ;
W is selected from -R₃, -NR₃R₄, or 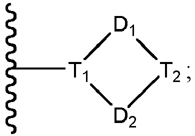
T₁ is selected from N or CR₃;
T₂ is selected from -NR₃, -CR₃(NR₃R₄), O or S;
D₁ and D₂ are independently selected from -(CR₃R₄)₁₋₃-;
R₃ and R₄ are each independently selected from H, C₁-C₆ alkyl, C₁-C₆ alkoxy, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S; wherein the said group is optionally substituted by one or more Rₐ;
each Rₐ is independently selected from H, -R, -NRR, or -OR, as long as the chemistry permits; wherein each R is independently H, C₁₋₆ alkyl, C₁₋₆ haloalkyl, C₁₋₆ alkoxy, C₁₋₆ haloalkoxy, 3- to 10-membered carbocyclyl, 3- to 10-membered heterocyclyl,; two adjacent R could be taken together to form optionally substituted 3- to 10-membered carbocyclyl, or optionally substituted 5- to 8-membered heterocyclyl;
or pharmaceutically acceptable salts, crystal forms, hydrates, solvates, stereoisomers or isotopic derivatives thereof.

In another aspect, the present disclosure provides a pharmaceutical composition containing a compound of the present disclosure and pharmaceutically acceptable excipient(s). In a specific embodiment, the compound of the present disclosure is provided in the pharmaceutical composition in an effective amount. In a specific embodiment, the compound of the present disclosure is provided in a therapeutically effective amount. In specific embodiments, the compound of the present disclosure is provided in a prophylactically effective amount.

In another aspect, the present disclosure provides a compound for use in a method for preparing a pharmaceutical composition as described above, including the following steps: mixing pharmaceutically acceptable excipient(s) with the compound of the present disclosure to form a pharmaceutical composition.

In another aspect, the present disclosure provides a pharmaceutical composition containing a compound of the present disclosure and pharmaceutically acceptable excipient(s), and other therapeutic agent(s).

In another aspect, the present disclosure provides a compound for use in a method of treating cancer-related disorders caused by ALK mutations in a subject in need thereof, comprising administering to the subject an effective amount of a compound of the present disclosure. In a specific embodiment, the cancer is selected from the group consisting of non-small cell lung cancer, breast cancer, neural tumor (such as glioblastoma and neuroblastoma); esophageal cancer, soft tissue cancer (such as rhabdomyosarcoma and the like); various forms of lymphoma, such as non-Hodgkin's lymphoma (NHL) known as anaplastic large cell lymphoma (ALCL); various forms of leukemia. In an alternative embodiment, the non-small cell lung cancer is ALK positive non-small cell lung cancer. In a specific embodiment, the compound is administered orally, subcutaneously, intravenously, or intramuscularly. In a specific embodiment, the compound is administered for a prolonged period of time.

Other objects and advantages of the present disclosure will be apparent to those skilled in the art from the following specific embodiments, examples, and claims.

### Definitions

### Chemical definitions

Definitions of specific functional groups and chemical terms are described in more detail below.

When a range of values is listed, it is intended to encompass each value and sub-range within the range. For example "C₁-C₆ alkyl" is intended to encompass, C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅ and C₅-C₆ alkyl.

It should be understood that when described herein any of the moieties defined forth below may be substituted by a variety of substituents, and that the respective definitions are intended to include such substituted moieties within their scope as set out below. Unless otherwise stated, the term "substituted" is to be defined as set out below.

"C₁-C₆ alkyl" refers to a radical of a straight-chain or branched saturated hydrocarbon group having from 1 to 6 carbon atoms, and it is also referred to herein as "lower alkyl". In some embodiments, C₁-C₄ alkyl is particularly preferred. Examples of alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Unless otherwise specified, each instance of an alkyl group is independently optionally substituted, i.e., unsubstituted (an "unsubstituted alkyl") or substituted (a "substituted alkyl") with one or more substituents; *e.g.,* for instance from 1 to 5 substituents, 1 to 3 substituents, or 1 substituent. In certain embodiments, the alkyl group is unsubstituted C₁₋₆ alkyl (*e.g.,* -CH₃). In certain embodiments, the alkyl group is substituted C₁₋₆ alkyl.

"C₂-C₆ alkenyl" refers to a radical of a straight-chain or branched hydrocarbon group having from 2 to 6 carbon atoms, one or more carbon-carbon double bonds *(e.g.,* 1, 2, or 3 carbon-carbon double bonds). The one or more carbon-carbon double bonds may be internal (such as in 2-butenyl) or terminal (such as in 1-butenyl). In some embodiments, C₂₋₄ alkenyl is particularly preferred. Examples of alkenyl groups include, but are not limited to, ethenyl (C₂), 1-propenyl (C₃), 2-propenyl (C₃), 1-propen-2-yl (C₃), 1-butenyl (C₄), 2-butenyl (C₄), butadienyl (C₄), pentenyl (C₅), pentadienyl (C₅), hexenyl (C₆), and the like. Unless otherwise specified, each instance of an alkenyl group is independently optionally substituted, *i.e.,* unsubstituted (an "unsubstituted alkenyl") or substituted (a "substituted alkenyl") with one or more substituents e.g., for instance from 1 to 5 substituents, 1 to 3 substituents, or 1 substituent. In certain embodiments, the alkenyl group is unsubstituted C₂₋₆ alkenyl. In certain embodiments, the alkenyl group is substituted C₂₋₆ alkenyl.

"C₂-C₆ alkynyl" refers to a radical of a straight-chain or branched hydrocarbon group having from 2 to 6 carbon atoms, one or more carbon-carbon triple bonds (*e.g.,* 1, 2, or 3 carbon-carbon triple bonds), and optionally one or more carbon-carbon double bonds (*e.g.,* 1, 2, or 3 carbon-carbon double bonds). In some embodiments, C₂₋₄ alkynyl is particularly preferred. In certain embodiments, alkynyl does not contain any double bonds. The one or more carbon-carbon triple bonds may be internal (such as in 2-butynyl) or terminal (such as in 1-butynyl). Examples of the alkynyl groups include, without limitation, ethynyl (C₂), 1-propynyl (C₃), 2-propynyl (C₃), 1-butynyl (C₄), 2-butynyl (C₄), pentynyl (C₅), 3-methylbut-1-ynyl (C₅), hexynyl (C₆), and the like. Unless otherwise specified, each instance of an alkynyl group is independently optionally substituted, *i*.*e*., unsubstituted (an "unsubstituted alkynyl") or substituted (a "substituted alkynyl") with one or more substituents; *e*.*g*., for instance from 1 to 5 substituents, 1 to 3 substituents, or 1 substituent. In certain embodiments, the alkynyl group is unsubstituted C₂₋₆ alkynyl. In certain embodiments, the alkynyl group is substituted C₂₋₆ alkynyl.

"C₁-C₆ alkoxy" refers to the group -OR wherein R is a substituted or unsubstituted C₁-C₆ alkyl group. In some embodiments, C₁-C₄ alkoxy group is particularly preferred. Specific alkoxy groups include, but are not limited to, methoxy, ethoxy, n-propoxy, isopropoxy, n-butoxy, tert-butoxy, sec-butoxy, n-pentyloxy, n-hexyloxy and 1,2-dimethylbutoxy.

"C₁-C₆ alkylthio" refers to the group -SR wherein R is optionally substituted C₁-C₆ alkyl. In some embodiments, C₁-C₄ alkylthio group is particularly preferred. Specifically, the C₁-C₆ alkylthio group includes, but is not limited to, methylthio, ethylthio, n-propylthio, isopropylthio, n-butylthio, tert-butylthio, sec-butylthio, n-pentylthio, n-hexylthio and 1,2-dimethylbutylthio.

"C₁-C₆ alkylamino" refers to the group -NHR or -NR₂, wherein R is optionally substituted C₁-C₆ alkyl. In some embodiments, C₁-C₄ alkylamino group is particularly preferred. Specifically, the C₁-C₆ alkylamino group includes, but is not limited to, methylamino, ethylamino, n-propylamino, isopropylamino, n-butylamino, t-butylamino, dimethylamino, methylethylamino and diethylamino.

"C₁-C₆ alkanoyl" refers to the group -(=O)R, wherein R is optionally substituted C₁-C₆ alkyl. In some embodiments, C₁-C₄ alkanoyl group is particularly preferred. Exemplary C₁-C₆ alkanoyl groups include, but are not limited to, -(=O)CH₃, -(=O)CH₂CH₃, -(=O)CH₂CH₂CH₃ and -(=O)CH(CH₃)₂.

"Halo" or "halogen" means fluorine (F), chlorine (Cl), bromine (Br) and iodine (I). In some embodiments, the halo group is F, -Cl or Br. In some embodiments, the halogen group is F or Cl. In some embodiments, the halogen group is F.

Thus, "C₁-C₆ haloalkyl" and "C₁-C₆ haloalkoxy" refer to the above "C₁-C₆ alkyl" and "C₁-C₆ alkoxy", which are substituted by one or more halo groups. In some embodiments, C₁-C₄ haloalkyl group is particularly preferred, and more preferably C₁-C₂ haloalkyl group. In some embodiments, C₁-C₄ haloalkoxy group is particularly preferred, and more preferably C₁-C₂ haloalkoxy group. Exemplary haloalkyl groups include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. Exemplary haloalkoxy groups include, but are not limited to: -OCH₂F, -OCHF₂, -OCF₃, and the like.

"C₃-C₈ carbocyclyl" or "3- to 8-membered carbocyclyl" refers to a radical of a non-aromatic cyclic hydrocarbon group having from 3 to 8 ring carbon atoms and zero heteroatoms. In some embodiments, C₅-C₈ carbocyclyl is preferred, which is a radical of a non-aromatic cyclic hydrocarbon group having from 5 to 8 ring carbon atoms and zero heteroatoms. In some embodiments, C₃-C₆ carbocyclyl is preferred, which is a radical of a non-aromatic cyclic hydrocarbon group having from 3 to 6 ring carbon atoms and zero heteroatoms. In some embodiments, C₅ carbocyclyl is preferred, which is a radical of a non-aromatic cyclic hydrocarbon group having 5 ring carbon atoms and zero heteroatoms. Carbocyclyl also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Exemplary carbocyclyl groups include, but is not limited to, cyclopropyl (C₃), cyclopropenyl (C₃), cyclobutyl (C₄), cyclobutenyl (C₄), cyclopentyl (C₅), cyclopentenyl (C₅), cyclopentadienyl (C₅), cyclohexyl (C₆), cyclohexenyl (C₆), cyclohexadienyl (C₆), as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Unless otherwise specified, each instance of a carbocyclyl group is independently optionally substituted, i.e., unsubstituted (an "unsubstituted carbocyclyl") or substituted (a "substituted carbocyclyl") with one or more substituents. In certain embodiments, the carbocyclyl group is unsubstituted C₃₋₈ carbocyclyl. In certain embodiments, the carbocyclyl group is a substituted C₃₋₈ carbocyclyl.

"3- to 8-membered heterocyclyl" refers to a radical of a 3- to 8-membered non-aromatic ring system having ring carbon atoms and 1 to 3 ring heteroatoms, wherein each heteroatom is independently selected from nitrogen, oxygen, sulfur, boron, phosphorus, and silicon, wherein the carbon, nitrogen, sulfur and phosphorus atoms may be present in the oxidation state, such as C(O), S(O), S(O)₂, P(O), and the like. In heterocyclyl groups that contain one or more nitrogen atoms, the point of attachment may be a carbon or nitrogen atom, as valency permits. In some embodiments, 4- to 7-membered heterocyclyl is preferred, which is a radical of a 4- to 7-membered non-aromatic ring system having ring carbon atoms and 1 to 3 ring heteroatoms. In some embodiments, 5- to 8-membered heterocyclyl is preferred, which is a radical of a 5- to 8-membered non-aromatic ring system having ring carbon atoms and 1 to 3 ring heteroatoms. In some embodiments, 4- to 6-membered heterocyclyl is preferred, which is a radical of a 4- to 6-membered non-aromatic ring system having ring carbon atoms and 1 to 3 ring heteroatoms. In some embodiments, 5- to 6-membered heterocyclyl is preferred, which is a radical of a 5- to 6-membered non-aromatic ring system having ring carbon atoms and 1 to 3 ring heteroatoms. In some embodiments, 5-membered heterocyclyl is more preferred, which is a radical of a 5-membered non-aromatic ring system having ring carbon atoms and 1 to 3 ring heteroatoms. In some embodiments, the 3- to 8-membered heterocyclyl, 4- to 7-membered heterocyclyl, 5- to 8-membered heterocyclyl, 4- to 6-membered heterocyclyl, 5- to 6-membered heterocyclyl and 5-membered heterocyclyl contain 1 to 3 (more preferably 1 or 2) ring heteroatoms selected from nitrogen, oxygen and sulfur (preferably nitrogen and oxygen). Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-8 membered heterocyclyl. In certain embodiments, the heterocyclyl group is substituted 3-8 membered heterocyclyl. Heterocyclyl also includes ring systems wherein the heterocyclyl ring, as defined above, is fused with one or more carbocyclyl groups wherein the point of attachment is on the carbocyclyl ring, or ring systems wherein the heterocyclyl ring, as defined above, is fused with one or more aryl or heteroaryl groups, wherein the point of attachment is on the heterocyclyl ring; and in such instances, the number of ring members continue to designate the number of ring members in the heterocyclyl ring system. Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxathiolanyl, oxathiolyl (1,2-oxathiolyl, 1,3-oxathiolyl), dithiolanyl, dihydropyrazolyl, dihydroimidazolyl, dihydrothiazolyl, dihydroisothiazolyl, dihydrooxazolyl, dihydroisoxazolyl, dihydrooxadiazolyl and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, tetrahydropyridinyl, and thianyl. Exemplary 6-membered heterocyclyl groups containing two heteroatoms include, without limitation, dihydropyrazinyl, piperazinyl, morpholinyl, dithianyl, dioxanyl. Exemplary 6-membered heterocyclyl groups containing three heteroatoms include, without limitation, triazinanyl. Exemplary 7-membered heterocyclyl groups containing one or two heteroatoms include, without limitation, azepanyl, diazepanyl, oxepanyl and thiepanyl. Exemplary 8-membered heterocyclyl groups containing one or two heteroatoms include, without limitation, azocanyl, oxecanyl, thiocanyl, octahydrocyclopenta[c]pyrrolyl and octahydropyrrolo[3,4-c]pyrrolyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an C₆ aryl ring (also referred to herein as a 6,6-bicyclic heterocyclic ring) include, without limitation, tetrahydroquinolinyl, tetrahydroisoquinolinyl, and the like.

"C₆-C₁₄ aryl" or "6- to 14-membered aromatic radical" refers to a radical of a monocyclic or polycyclic (*e*.*g*., bicyclic or tricyclic) 4n+2 aromatic ring system (*e*.*g*., having 6, 10, or 14 π electrons shared in a cyclic array) having 6-14 ring carbon atoms and zero heteroatoms provided in the aromatic ring system. In some embodiments, an aryl group has six ring carbon atoms ("C₆ aryl"; *e*.*g*., phenyl). In some embodiments, an aryl group has ten ring carbon atoms ("C₁₀ aryl"; *e*.*g*., naphthyl such as 1-naphthyl and 2-naphthyl). In some embodiments, an aryl group has fourteen ring carbon atoms ("C₁₄ aryl"; *e*.*g*., anthracyl). "C₆-C₁₄ aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"5- to 10-membered heteroaryl" refers to a radical of a 5-10 membered monocyclic or bicyclic 4n+2 aromatic ring system (*e*.*g*., having 6 or 10 π electrons shared in a cyclic array) having ring carbon atoms and 1-4 ring heteroatoms provided in the aromatic ring system, wherein each heteroatom is independently selected from nitrogen, oxygen and sulfur. In some embodiments, 5-membered heteroaryl is preferred, which is a radical of a 5-membered monocyclic 4n+2 aromatic ring system (*e*.*g*., having 6 π electrons shared in a cyclic array) having ring carbon atoms and 1-4 ring heteroatoms, wherein each heteroatom is independently selected from nitrogen, oxygen and sulfur. In heteroaryl groups that contain one or more nitrogen atoms, the point of attachment may be a carbon or nitrogen atom, as valency permits. Heteroaryl bicyclic ring systems can include one or more heteroatoms in one or both rings. Heteroaryl includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. Unless otherwise specified, each instance of a heteroaryl group is independently optionally substituted, i.e., unsubstituted (an "unsubstituted heteroaryl") or substituted (a "substituted heteroaryl") with one or more substituents. In certain embodiments, the heteroaryl group is unsubstituted 5- to 10-membered heteroaryl. In certain embodiments, the heteroaryl group is substituted 5- to 10-membered heteroaryl. Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Alkyl, alkenyl, alkynyl, carbocyclyl, heterocyclyl, aryl, and heteroaryl groups, as defined herein, are optionally substituted groups. In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. For purposes of this disclosure, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, perhaloalkyl, alkenyl, alkynyl, carbocyclyl, heterocyclyl, aryl, and heteroaryl, wherein each alkyl, alkenyl, alkynyl, carbocyclyl, heterocyclyl, aryl, and heteroaryl is independently substituted by 0, 1, 2, 3, 4, or 5 R^{dd} groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from alkyl, perhaloalkyl, alkenyl, alkynyl, carbocyclyl, heterocyclyl, aryl, and heteroaryl, or two R^{aa} groups are joined to form a heterocyclyl or heteroaryl ring, wherein each alkyl, alkenyl, alkynyl, carbocyclyl, heterocyclyl, aryl, and heteroaryl is independently substituted by 0, 1, 2, 3, 4, or 5 R^{dd} groups;
each instance of R^{bb} is, independently, selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, perhaloalkyl, alkenyl, alkynyl, carbocyclyl, heterocyclyl, aryl, and heteroaryl, or two R^{bb} groups are joined to form a heterocyclyl or heteroaryl ring, wherein each alkyl, alkenyl, alkynyl, carbocyclyl, heterocyclyl, aryl, and heteroaryl is independently substituted by 0, 1, 2, 3, 4, or 5 R^{dd} groups;
each instance of R^{cc} is, independently, selected from hydrogen, alkyl, perhaloalkyl, alkenyl, alkynyl, carbocyclyl, heterocyclyl, aryl, and heteroaryl, or two R^{cc} groups are joined to form a heterocyclyl or heteroaryl ring, wherein each alkyl, alkenyl, alkynyl, carbocyclyl, heterocyclyl, aryl, and heteroaryl is independently substituted by 0, 1, 2, 3, 4, or 5 R^{dd} groups;
each instance of R^{dd} is, independently, selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, perhaloalkyl, alkenyl, alkynyl, carbocyclyl, heterocyclyl, aryl, heteroaryl, wherein each alkyl, alkenyl, alkynyl, carbocyclyl, heterocyclyl, aryl, and heteroaryl is independently substituted by 0, 1, 2, 3, 4, or 5 R^{gg} groups, or two geminal R^{dd} substituents may be joined to form =O or =S;
each instance of R^{ee} is, independently, selected from alkyl, perhaloalkyl, alkenyl, alkynyl, carbocyclyl, aryl, heterocyclyl, and heteroaryl, wherein each alkyl, alkenyl, alkynyl, carbocyclyl, heterocyclyl, aryl, and heteroaryl is independently substituted by 0, 1, 2, 3, 4, or 5 R^{gg} groups;
each instance of R^{ff} is, independently, selected from hydrogen, alkyl, perhaloalkyl, alkenyl, alkynyl, carbocyclyl, heterocyclyl, aryl and heteroaryl, or two R^{ff} groups are joined to form a heterocyclyl or heteroaryl ring, wherein each alkyl, alkenyl, alkynyl, carbocyclyl, heterocyclyl, aryl, and heteroaryl is independently substituted by 0, 1, 2, 3, 4, or 5 R^{gg} groups; and
each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl) ⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)( C₁₋₆ alkyl), -N(C₁₋₆ alkyl)C(=O)( C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂,-SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, C₃₋₁₀ carbocyclyl, C₆₋₁₀ aryl, 3-10 membered heterocyclyl, 5-10 membered heteroaryl; or two geminal R^{gg} substituents may be joined to form =O or =S; wherein X⁻ is a counterion.

Exemplary nitrogen atom substituents include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, perhaloalkyl, alkenyl, alkynyl, carbocyclyl, heterocyclyl, aryl, and heteroaryl, or two R^{cc} groups attached to a nitrogen atom are joined to form a heterocyclyl or heteroaryl ring, wherein each alkyl, alkenyl, alkynyl, carbocyclyl, heterocyclyl, aryl, and heteroaryl is independently substituted by 0, 1, 2, 3, 4, or 5 R^{dd} groups, and wherein R^{aa}, R^{bb}, R^{cc} and R^{dd} are as defined above.

### Other definitions

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge *et al.,* describes pharmaceutically acceptable salts in detail in J. Pharmaceutical Sciences (1977) 66:1-19. Pharmaceutically acceptable salts of the compounds of the present disclosure include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (*i*.*e*., a male or female of any age group, *e.g.,* a pediatric subject (*e*.*g*., infant, child, adolescent) or adult subject (*e*.*g*., young adult, middle-aged adult or senior adult)) and/or a non-human animal, *e.g.,* a mammal such as primates (*e*.*g*., cynomolgus monkeys, rhesus monkeys), cattle, pigs, horses, sheep, goats, rodents, cats, and/or dogs. In certain embodiments, the subject is a human. In certain embodiments, the subject is a non-human animal.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

In general, the "effective amount" of a compound refers to an amount sufficient to elicit the desired biological response. As will be appreciated by those of ordinary skill in this art, the effective amount of a compound disclosed herein may vary depending on such factors as the desired biological endpoint, the pharmacokinetics of the compound, the disease being treated, the mode of administration, and the age, health, and condition of the subject. An effective amount encompasses therapeutic and prophylactic treatment.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

"Combination" and related terms mean the simultaneous or sequential administration of a therapeutic agent of the present disclosure. For example, a compound disclosed herein may be administered simultaneously or sequentially with another therapeutic agent in separate unit dosage forms, or together with another therapeutic agent in a single unit dosage form.

### Specific embodiments of the disclosure

### Compound

The "compound of the present disclosure" used herein refers to the following compounds of formula (I), and their pharmaceutically acceptable salts, crystal forms, hydrates, solvates, stereoisomers or isotopic derivatives thereof.

In one embodiment, the present disclosure relates to a compound of formula (I): 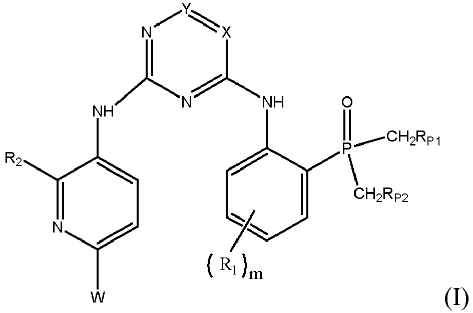 wherein,
X is selected from CRx or NR_{X1};
Y is selected from CR_{Y} or NR_{Y1};
m is selected from 0, 1, 2, 3 or 4;
R₁, R_{X} and R_{Y} are independently selected from:
   1) H, halogen, -CN, -OH, -NO₂ or -NH₂;
   2) C₁-C₆ alkyl, C₁-C₆ haloalkyl, C₁-C₆ alkoxy, C₁-C₆ haloalkoxy, C₁-C₆ alkylthio, C₁-C₆ alkylamino, C₁-C₆ alkanoyl, 3- to 7-membered carbocyclyl or 3- to 8-membered heterocyclyl; wherein the said group is optionally substituted by one or more Rₐ; or
   3) R_{X1} and R_{Y1} are absent; or two adjacent substituents selected from R_{X}, R_{X1}, R_{Y} and R_{Y1}, or two adjacent R₁ groups, together with the atoms to which they are attached may form a fused 5- to 7-membered ring, which contains 0 to 4 heteroatoms selected from N, O and S and may be substituted by one or more Rₐ;
R_{P1} and R_{P2} are independently H;
R₂ is selected from C₁-C₆ alkoxy, C₁-C₆ haloalkoxy, C₂-C₆ alkenyloxy or 3- to 7-membered cycloalkyloxy; wherein the said group is optionally substituted by one or more Ra;
W is selected from -R₃, - -NR₃R₄, or 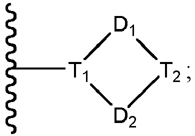
T₁ is selected from N or CR₃;
T₂ is selected from -NR₃, -CR₃(NR₃R₄), O or S;
D₁ and D₂ are independently selected from -(CR₃R₄)₁₋₃-;
each R₃ and R₄ is independently selected from H, C₁-C₆ alkyl, C₁-C₆ alkoxy, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S; wherein the said group is optionally substituted by one or more Rₐ;
each Rₐ is independently selected from H, -R, -NRR, or -OR, , as long as the chemistry permits; wherein each R is independently H, C₁₋₆ alkyl, C₁₋₆ haloalkyl, C₁₋₆ alkoxy, C₁₋₆ haloalkoxy, 3- to 10-membered carbocyclyl, or 3- to 10-membered heterocyclyl; two adjacent R could be taken together to form optionally substituted 3- to 10-membered carbocyclyl, or optionally substituted 5- to 8-membered heterocyclyl;
or pharmaceutically acceptable salts, crystal forms, hydrates, solvates, stereoisomers or isotopic derivatives thereof.

In such embodiment, alternatively, X is selected from CRx or NR_{X1}, Y is selected from CR_{Y} or NR_{Y1}; alternatively, X is CR_{X} and Y is CR_{Y}.

In the above embodiments of X, alternatively, Rx is selected from H, halogen, -CN, -OH, -NO₂, -NH₂, C₁-C₆ alkyl, C₁-C₆ haloalkyl, C₁-C₆ alkoxy, C₁-C₆ haloalkoxy, C₁-C₆ alkylthio, C₁-C₆ alkylamino, C₁-C₆ alkanoyl, 3- to 7-membered carbocyclyl or 3- to 8-membered heterocyclyl; alternatively, Rx is selected from H, halogen, -CN, C₁-C₆ alkyl, C₁-C₆ haloalkyl, C₁-C₆ alkoxy, C₁-C₆ haloalkoxy, 3- to 6-membered carbocyclyl or 3- to 6-membered heterocyclyl; alternatively, Rx is selected from halogen, C₁-C₆ alkyl or C₁-C₆ haloalkyl; yet alternatively, Rx is halogen; yet alternatively, Rx is selected from -F, -Cl or -Br; still alternatively, Rx is -Cl; the aforementioned groups are optionally substituted by one or more Rₐ; and Rₐ is as defined above, as long as the valency permits.

In the above embodiments of Y, alternatively, R_{Y} is selected from H, halogen, -CN, -OH, -NO₂, -NH₂, C₁-C₆ alkyl, C₁-C₆ haloalkyl, C₁-C₆ alkoxy, C₁-C₆ haloalkoxy, C₁-C₆ alkylthio, C₁-C₆ alkylamino, C₁-C₆ alkanoyl, 3- to 7-membered carbocyclyl or 3- to 8-membered heterocyclyl; alternatively, R_{Y} is selected from H, halogen, -CN, -OH, -NO₂, -NH₂, C₁-C₆ alkyl, C₁-C₆ haloalkyl, 3- to 6-membered carbocyclyl or 3- to 6-membered heterocyclyl; alternatively, R_{Y} is selected from H, halogen, -CN, -OH, -NO₂ or -NH₂; yet alternatively R_{Y} is H; the aforementioned groups are optionally substituted by one or more Rₐ; and Rₐ is as defined above, as long as the valency permits.

In the above embodiments of X and Y, alternatively, R_{X} and R_{Y} together with the carbon atom to which they are attached may form a fused 5- to 7-membered ring, which contains 0 to 4 heteroatoms selected from N, O and S; alternatively, R_{X} and R_{Y} together with the carbon atom to which they are attached may form a fused 5- to 7-membered aromatic ring, which contains 0 to 2 heteroatoms selected from N, O and S; alternatively, R_{X} and R_{Y} together with the carbon atom to which they are attached may form a fused 5-membered aromatic ring, which contains 0 to 2 heteroatoms selected from N and S; yet alternatively, R_{X} and R_{Y} together with the carbon atom to which they are attached may form a pyrrole ring, pyrazole ring, imidazole ring, thiophene ring or furan ring; still alternatively, Rx and R_{Y} together with the carbon atom to which they are attached may form a pyrrole ring or thiophene ring.

In such embodiment, R_{P1} and R_{P2} are independently H.

In such embodiment, alternatively, R₁ is selected from H, halogen, -CN, -OH, -NO₂, -NH₂, C₁-C₆ alkyl, C₁-C₆ haloalkyl, C₁-C₆ alkoxy, C₁-C₆ haloalkoxy, C₁-C₆ alkylthio, C₁-C₆ alkylamino, C₁-C₆ alkanoyl, 3- to 7-membered carbocyclyl or 3- to 8-membered heterocyclyl, or two adjacent R₁ groups, together with the atoms to which they are attached may form a fused 5- to 7-membered ring, which contains 0 to 4 heteroatoms selected from N, O and S; alternatively, R₁ is selected from H, halogen, -CN, -OH, -NO₂, C₁-C₆ alkyl, C₁-C₆ haloalkyl, 3- to 6-membered carbocyclyl or 3- to 6-membered heterocyclyl; alternatively, R₁ is selected from H, halogen, -CN or C₁-C₆ alkyl; yet alternatively, R₁ is H; the aforementioned groups are optionally substituted by one or more Rₐ; and Rₐ is as defined above, as long as the valency permits.

In such embodiment, alternatively, m is selected from 0, 1 or 2, alternatively, m is selected from 0 or 1.

In such embodiment, alternatively, R₂ is selected from C₁-C₆ alkoxy, C₁-C₆ haloalkoxy, C₂-C₆ alkenyloxy or 3- to 7-membered cycloalkyloxy; alternatively, R₂ is selected from C₁-C₆ alkoxy or C₁-C₆ haloalkoxy; alternatively, R₂ is selected from -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, -OCF₃, -OCHF₂, -OCH₂F, -OCH₂CF₃, -OCHF₂, -OCH₂F, -OCCl₃, -OCHCl₂, -OCH₂Cl, -OCH₂CCl₃, -OCHCl₂, -OCH₂Cl; yet alternatively, R₂ is selected from -OCH₃, -OCH(CH₃)₂ or -OCH₂CF₃; the aforementioned groups are optionally substituted by one or more Rₐ; and Rₐ is as defined above, as long as the valency permits.

In such embodiment, alternatively, W is selected from -R₃, -NR₃R₄, , or

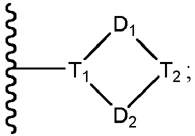

In the above embodiment of W, alternatively, T₁ is selected from N or CR₃;

In the above embodiment of W, alternatively, T₂ is selected from -NR₃, -CR₃(NR₃R₄), O or S; alternatively, T₂ is selected from -NR₃, or -CR₃(NR₃R₄);

In the above embodiment of W, D₁ and D₂ are independently -(CR₃R₄)₁₋₃;

In the above embodiment of W, alternatively, R₃ and R₄ are independently selected from H, C₁-C₆ alkyl, C₁-C₆ alkoxy, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S; the aforementioned groups are optionally substituted by one or more Rₐ; and Rₐ is as defined above, as long as the valency permits.

In the above embodiment of W, alternatively, W is selected from the following groups: 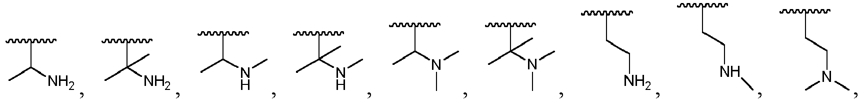 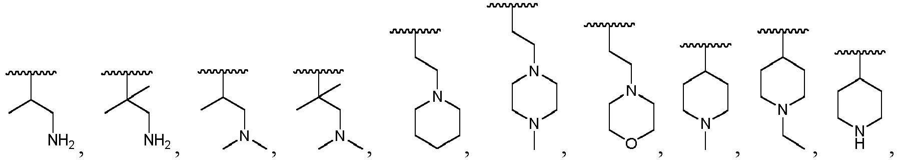 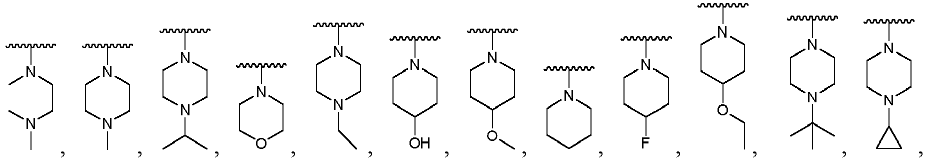 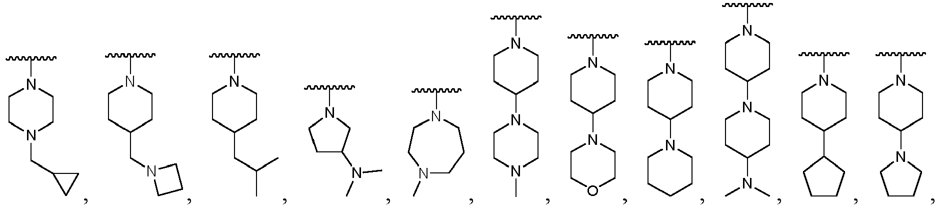 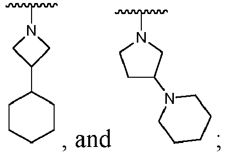 alternatively, W is selected from the following groups:

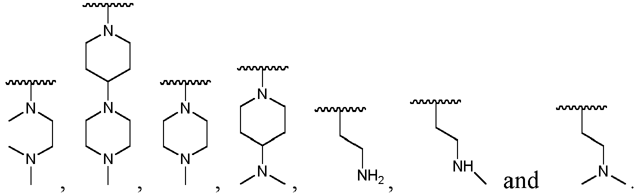

In another embodiment, the present disclosure relates to a compound of formula (II): 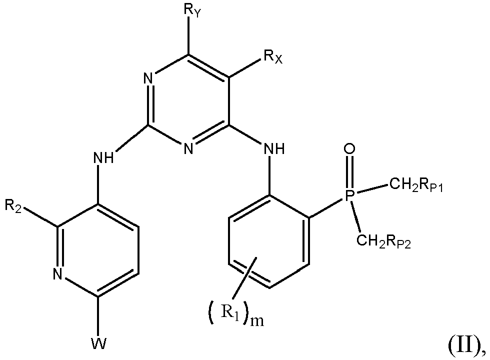 wherein Rx, R_{Y}, R₁, R_{P1}, R_{P2}, R₂, Wand m are as defined above.

In another alternative embodiment, the present disclosure relates to a compound of formula (II): 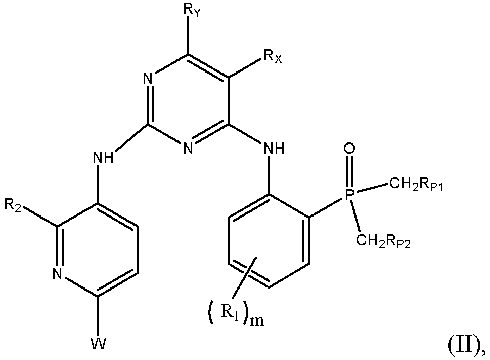 wherein,
R_{X} and R_{Y} are each independently selected from H, halogen or C₁-C₆ alkyl, R_{X} and R_{Y} together with the carbon atom to which they are attached may form a fused 5- to 7-membered aromatic ring, which contains 0 to 2 heteroatoms selected from N, O and S;
m is selected from 0, 1 or 2;
R₁ is selected from H, halogen or C₁-C₆ alkyl, wherein the C₁-C₆ alkyl is optionally substituted by one or more Rₐ;
R_{P1} and R_{P2} are each independently H;
R₂ is selected from C₁-C₆ alkoxy, C₁-C₆ haloalkoxy or 3- to 7-membered cycloalkyloxy; wherein the said group is optionally substituted by one or more Rₐ;
W is selected from -R₃, -NR₃R₄ or 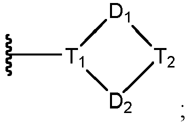
T₁ is selected from N or CR₃;
T₂ is selected from -NR₃, -CR₃(NR₃R₄), O or S;
D₁ and D₂ are independently selected from -(CR₃R₄)₁₋₃-;
R₃ and R₄ are independently selected from H, C₁-C₆ alkyl, C₁-C₆ alkoxy, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S; wherein the said group is optionally substituted by one or more Rₐ;
each Rₐ is independently selected from H, -R, -NRR, or -OR, as long as the chemistry permits; wherein each R is independently H, C₁₋₆ alkyl, C₁₋₆ haloalkyl, C₁₋₆ alkoxy, C₁₋₆ haloalkoxy, 3- to 10-membered carbocyclyl, 3- to 10-membered heterocyclyl,; two adjacent R could be taken together to form optionally substituted 3- to 10-membered carbocyclyl, or optionally substituted 5- to 8-membered heterocyclyl; or pharmaceutically acceptable salts, crystal forms, hydrates, solvates, stereoisomers or isotopic derivatives thereof.

In some embodiments of formula (II), alternatively, Rx is selected from H, halogen or C₁-C₄ alkyl; alternatively, Rx is H; alternatively, Rx is methyl; alternatively, Rx is selected from F, Cl or Br; yet alternatively, Rx is Cl.

In some embodiments of formula (II), alternatively, R_{Y} is selected from H, halogen or C₁-C₄ alkyl; yet alternatively, R_{Y} is H.

In some embodiments of formula (II), alternatively, R_{X} and R_{Y} together with the carbon atom to which they are attached may form a fused 5-membered aromatic ring, which contains 0 to 2 heteroatoms selected from N and S; yet alternatively, R_{X} and R_{Y} together with the carbon atom to which they are attached may form a pyrrole ring, pyrazole ring, imidazole ring, thiophene ring or furan ring; still alternatively, Rx and R_{Y} together with the carbon atom to which they are attached may form a pyrrole ring or thiophene ring; the aforementioned groups are optionally substituted by one or more Rₐ; and Rₐ is as defined above, as long as the valency permits.

In some embodiments of formula (II), alternatively, R₁ is selected from H, halogen or C₁-C₄ alkyl; yet alternatively, R₁ is H.

In some embodiments of formula (II), alternatively, m is selected from 0 or 1.

In some embodiments of formula (II), alternatively, R₂ is selected from C₁-C₄ alkoxy, C₁-C₄ haloalkoxy or 3- to 7-membered cycloalkyloxy;
alternatively, R₂ is selected from C₁-C₄ alkoxy or C₁-C₄ haloalkoxy ;
alternatively, R₂ is selected from -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, -OCF₃, -OCHF₂, -OCH₂F, -OCH₂CF₃, -OCHF₂, -OCH₂F, -OCCl₃, -OCHCl₂, -OCH₂Cl, -OCH₂CCl₃, -OCHCl₂, -OCH₂Cl;
yet alternatively, R₂ is selected from -OCH₃, -OCH(CH₃)₂ or -OCH₂CF₃;
the aforementioned groups are optionally substituted by one or more Rₐ; and Rₐ is as defined above, as long as the valency permits.

In some embodiments of formula (II), alternatively, W is selected from -R₃, -NR₃R₄ or 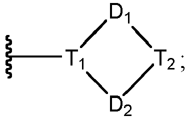 wherein,
T₁ is selected from N or CR₃;
T₂ is selected from -NR₃, or -CR₃(NR₃R₄);
D₁ and D₂ are independently -(CR₃R₄)₁₋₃;
R₃ and R₄ are independently selected from C₁-C₆ alkyl, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S;
alternatively, W is selected from the following groups: 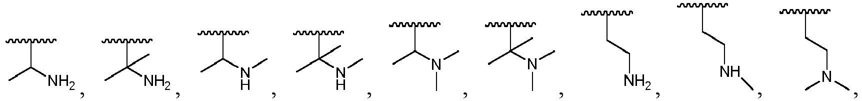 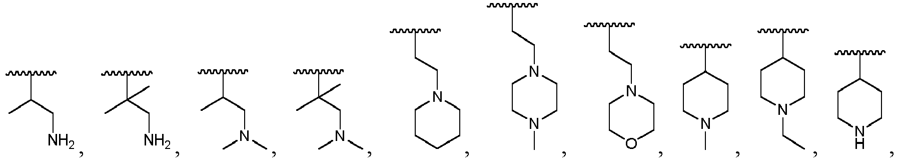 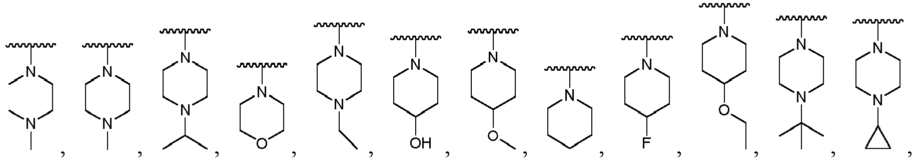 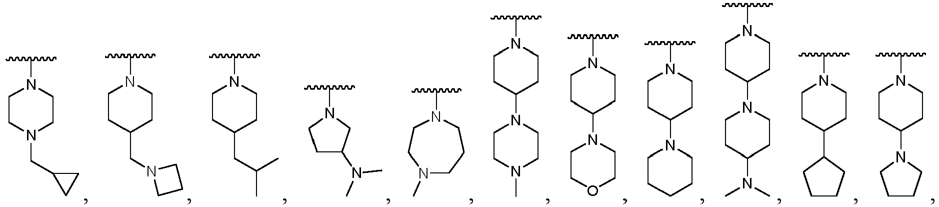 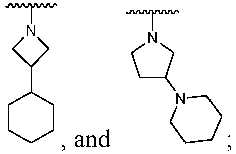
alternatively, W is selected from the following groups: 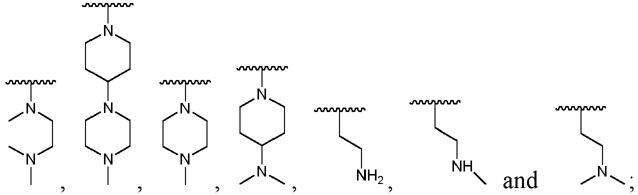
the aforementioned groups are optionally substituted by one or more Rₐ, as long as the valency permits; wherein, each Rₐ is independently selected from H, -R, -NRR or -OR, as long as the chemistry permits; wherein, each R is independently selected from H, C₁₋₆ alkyl, C₁₋₆ haloalkyl, C₁₋₆ alkoxy, C₁₋₆ haloalkoxy, 3- to 10-membered carbocyclyl, 3- to 10-membered heterocyclyl; two adjacent R could be taken together to form optionally substituted 3- to 10-membered carbocyclyl or optionally substituted 5- to 8-membered heterocyclyl.

In another embodiment, the disclosure relates to a compound of formulae (IIIa) to (IIIm): 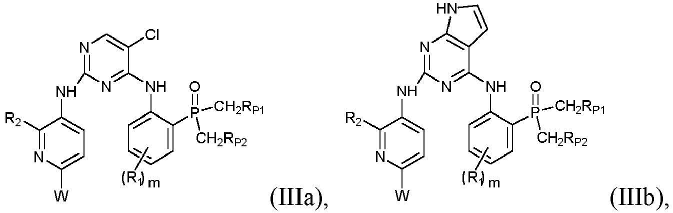 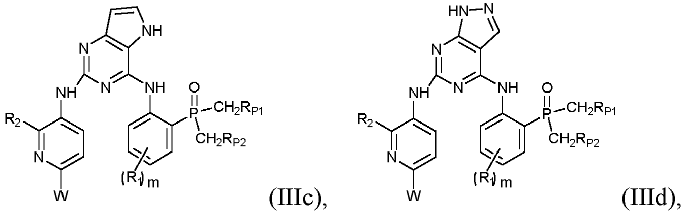 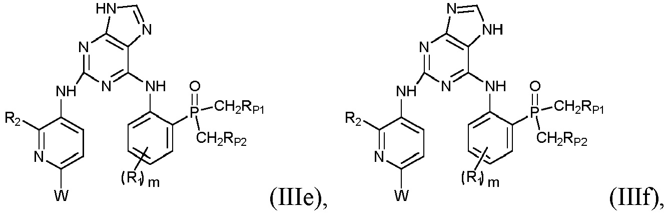 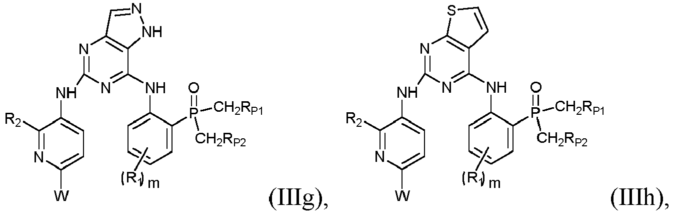 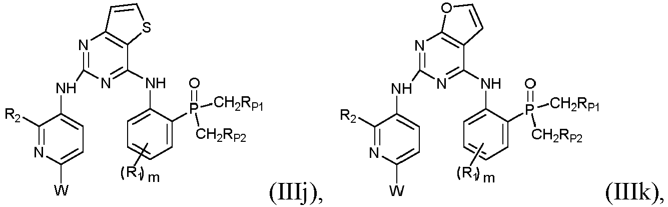 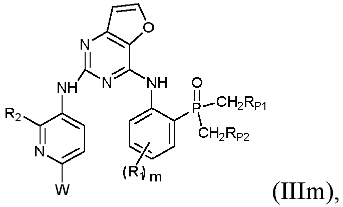 wherein, R₁, R_{P1}, R_{P2}, R₂, Wand m are as defined above.

In another alternative embodiment, the present disclosure relates to a compound of formulae (IIIa) to (IIIm): 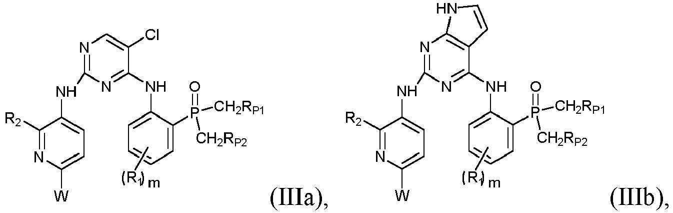 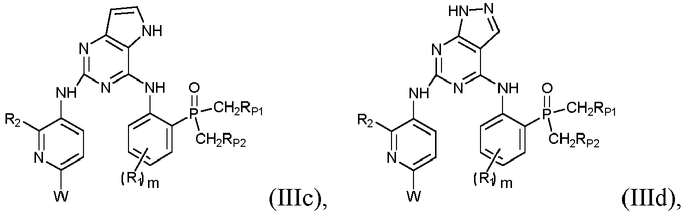 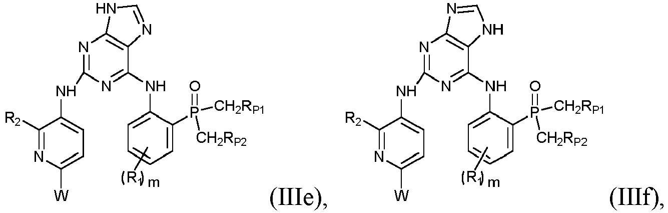 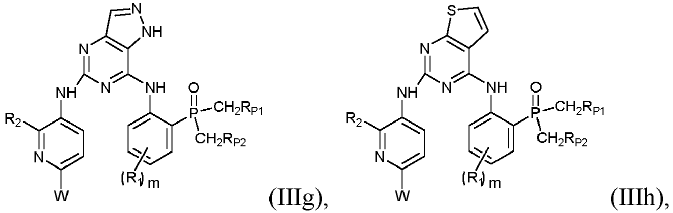 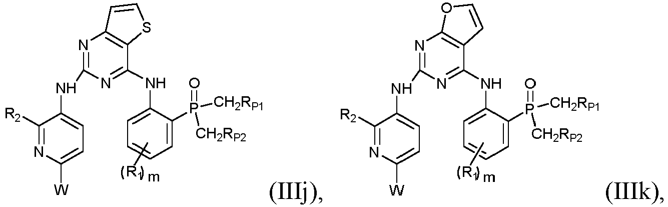 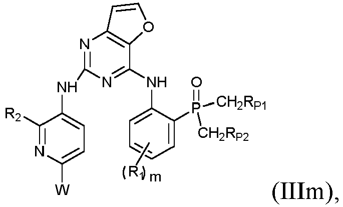 wherein,
m is selected from 0, 1 or 2;
R₁ is selected from H, halogen or C₁-C₆ alkyl, wherein C₁-C₆ alkyl is optionally substituted by one or more Rₐ;
R_{P1} and R_{P2} are each independently H;
R₂ is selected from C₁-C₆ alkoxy, C₁-C₆ haloalkoxy or 3- to 7-membered cycloalkyloxy; wherein the said group is optionally substituted by one or more Rₐ;
W is selected from -R₃, -NR₃R₄ or 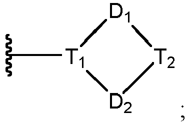
T₁ is selected from N or CR₃;
T₂ is selected from -NR₃, -CR₃(NR₃R₄), O or S;
D₁ and D₂ are independently selected from -(CR₃R₄)₁₋₃-;
R₃ and R₄ are independently selected from H, C₁-C₆ alkyl, C₁-C₆ alkoxy, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S; wherein the said group is optionally substituted by one or more Rₐ;
each Rₐ is independently selected from H, -R, -NRR, or -OR, as long as the chemistry permits; wherein each R is independently H, C₁₋₆ alkyl, C₁₋₆ haloalkyl, C₁₋₆ alkoxy, C₁₋₆ haloalkoxy, 3- to 10-membered carbocyclyl, 3- to 10-membered heterocyclyl; two adjacent R could be taken together to form optionally substituted 3- to 10-membered carbocyclyl, optionally substituted 5- to 8-membered heterocyclyl; or pharmaceutically acceptable salts, crystal forms, hydrates, solvates, stereoisomers or isotopic derivatives thereof.

In some embodiments of formulae (IIIa) to (IIIm), alternatively, R₁ is selected from H, halogen or C₁-C₄ alkyl; yet alternatively, R₁ is H.

In some embodiments of formulae (IIIa) to (IIIm), alternatively, m is selected from 0 or 1.

In some embodiments of formulae (IIIa) to (IIIm), alternatively, R₂ is selected from C₁-C₄ alkoxy, C₁-C₄ haloalkoxy or 3- to 7-membered cycloalkyloxy;
alternatively, R₂ is selected from C₁-C₄ alkoxy or C₁-C₄ haloalkoxy ;
alternatively, R₂ is selected from -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, -OCF₃, -OCHF₂, -OCH₂F, -OCH₂CF₃, -OCHF₂, -OCH₂F, -OCCl₃, -OCHCl₂, -OCH₂Cl, -OCH₂CCl₃, -OCHCl₂, -OCH₂Cl;
yet alternatively, R₂ is selected from -OCH₃, -OCH(CH₃)₂ or -OCH₂CF₃;
the aforementioned groups are optionally substituted by one or more Rₐ; and Rₐ is as defined above, as long as the valency permits.

In some embodiments of formulae (IIIa) to (IIIm), alternatively, W is selected from -R₃, -NR₃R₄ or 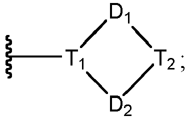 wherein,
T₁ is selected from N or CR₃;
T₂ is selected from -NR₃, or -CR₃(NR₃R₄);
D₁ and D₂ are independently -(CR₃R₄)₁₋₃;
R₃ and R₄ are independently selected from C₁-C₆ alkyl, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S;
alternatively, W is selected from the following groups: 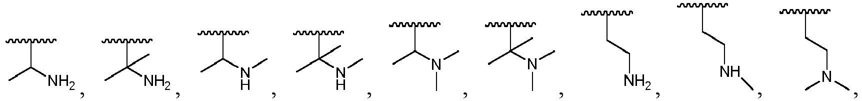 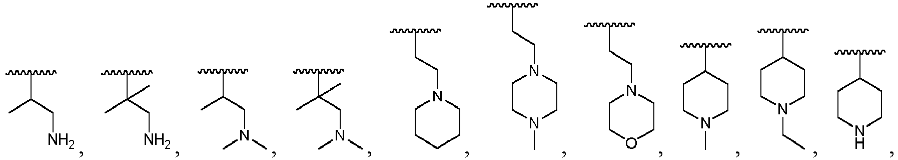 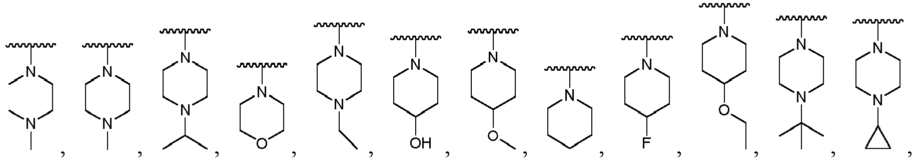 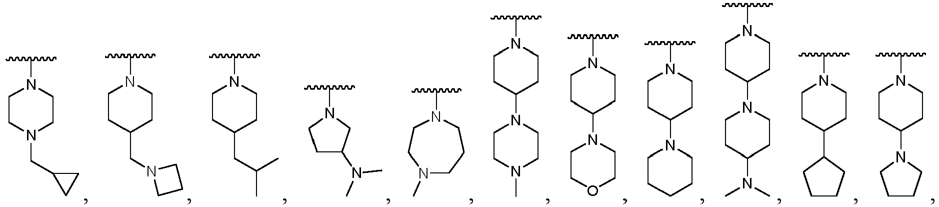 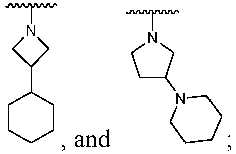
alternatively, W is selected from the following groups: 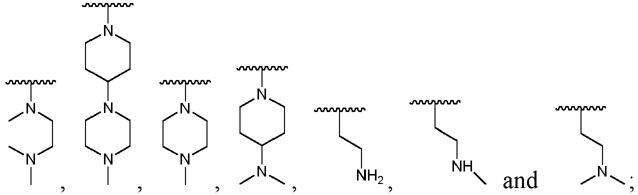
as long as the valency permits, the aforementioned groups are optionally substituted by one or more Rₐ; wherein, each Rₐ is independently selected from H, -R, -NRR or -OR, as long as the chemistry permits; wherein each R is independently selected from H, C₁₋₆ alkyl, C₁₋₆ haloalkyl, C₁₋₆ alkoxy, C₁₋₆ haloalkoxy, 3- to 10-membered carbocyclyl, 3- to 10-membered heterocyclyl; two adjacent R could be taken together to form optionally substituted 3- to 10-membered carbocyclyl or optionally substituted 5- to 8-membered heterocyclyl.

In another embodiment, the present disclosure relates to a compound of formula (IIIa): 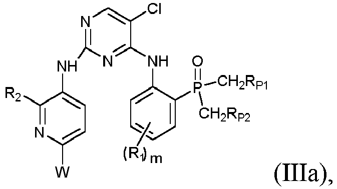 wherein,
R_{P1} and R_{P2} are each independently H;
m is selected from 0 or 1;
R₁ is H;
R₂ is selected from C₁-C₆ alkoxy or C₁-C₆ haloalkoxy ;
W is selected from the following groups: 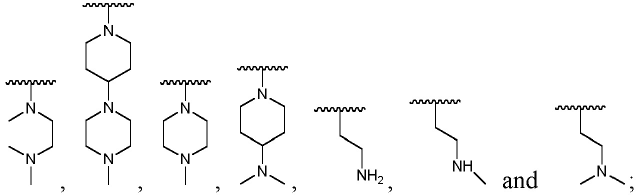
or pharmaceutically acceptable salts, crystal forms, hydrates, solvates, stereoisomers or isotopic derivatives thereof.

In another preferred embodiment, the present disclosure relates to a compound of formula (IIIa): 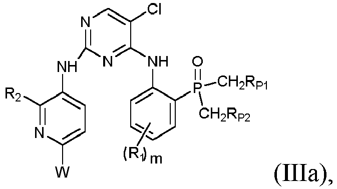 wherein,
R_{P1} and R_{P2} are each independently H;
m is selected from 0 or 1;
R₁ is H;
R₂ is selected from -OCH₃, -OCH(CH₃)₂ or -OCH₂CF₃;
W is selected from the following groups: 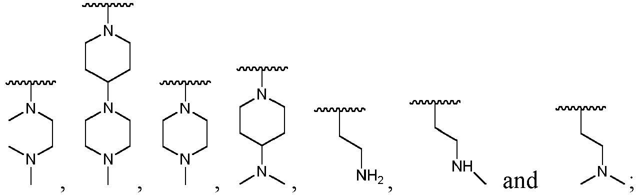
or pharmaceutically acceptable salts, crystal forms, hydrates, solvates, stereoisomers or isotopic derivatives thereof.

In another embodiment, the present disclosure relates to a compound of formula (IIIb): 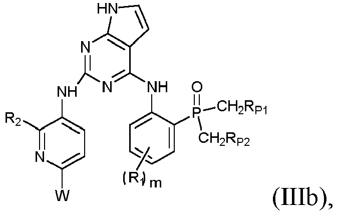 wherein,
R_{P1} and R_{P2} are each independently H;
m is selected from 0 or 1;
R₁ is H;
R₂ is selected from C₁-C₆ alkoxy or C₁-C₆ haloalkoxy ;
W is selected from the following groups: 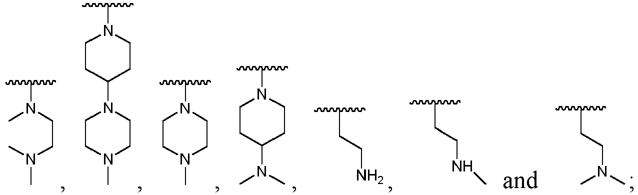
or pharmaceutically acceptable salts, crystal forms, hydrates, solvates, stereoisomers or isotopic derivatives thereof.

In another preferred embodiment, the present disclosure relates to a compound of formula (IIIb): 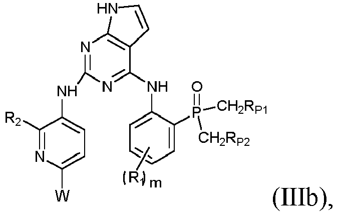 wherein,
m is selected from 0 or 1;
R₁ is H;
R₂ is -OCH₃;
W is selected from the following groups: 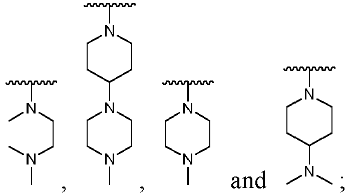
or pharmaceutically acceptable salts, crystal forms, hydrates, solvates, stereoisomers or isotopic derivatives thereof.

In another embodiment, the present disclosure relates to a compound of formula (IIIh) : 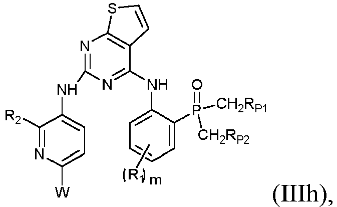 wherein,
R_{P1} and R_{P2} are each independently H;
m is selected from 0 or 1;
R₁ is H;
R₂ is selected from C₁-C₆ alkoxy or C₁-C₆ haloalkoxy ;
W is selected from the following groups: 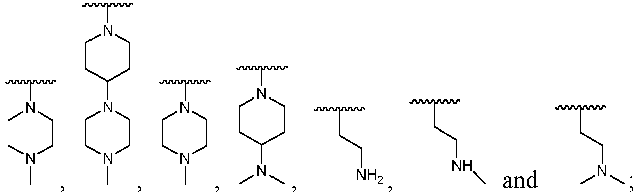
or pharmaceutically acceptable salts, crystal forms, hydrates, solvates, stereoisomers or isotopic derivatives thereof.

In another embodiment, the present disclosure relates to a compound of formula (IIIj): 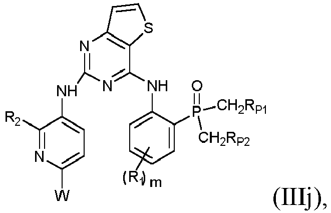 wherein,
R_{P1} and R_{P2} are each independently H;
m is selected from 0 or 1;
R₁ is H;
R₂ is selected from C₁-C₆ alkoxy or C₁-C₆ haloalkoxy ;
W is selected from the following groups: 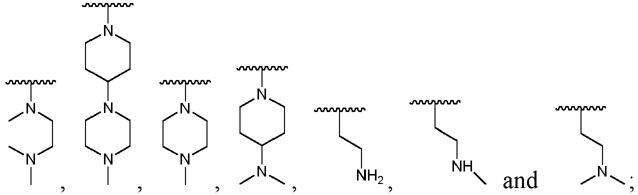
or pharmaceutically acceptable salts, crystal forms, hydrates, solvates, stereoisomers or isotopic derivatives thereof.

In some embodiments, the present disclosure provides a compound selected from:

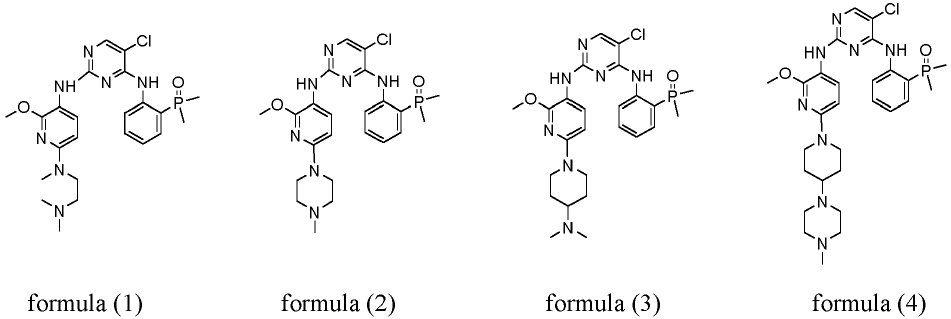

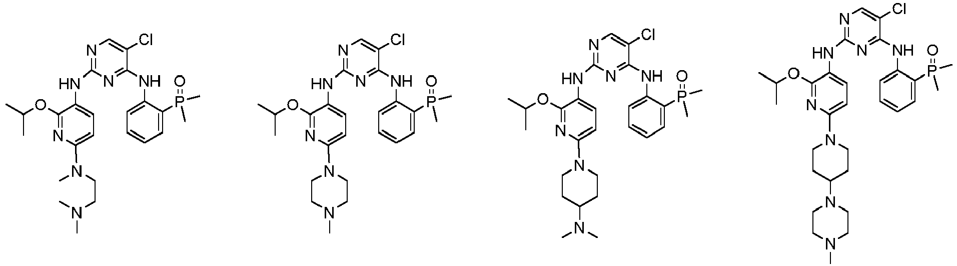

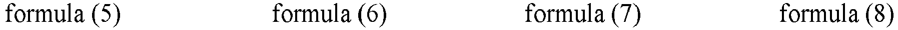

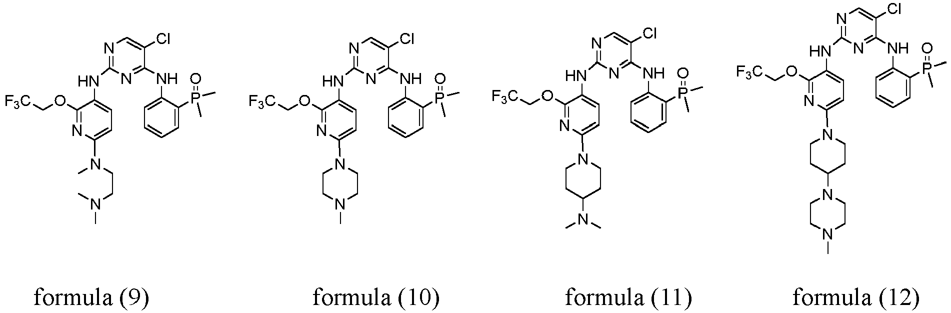

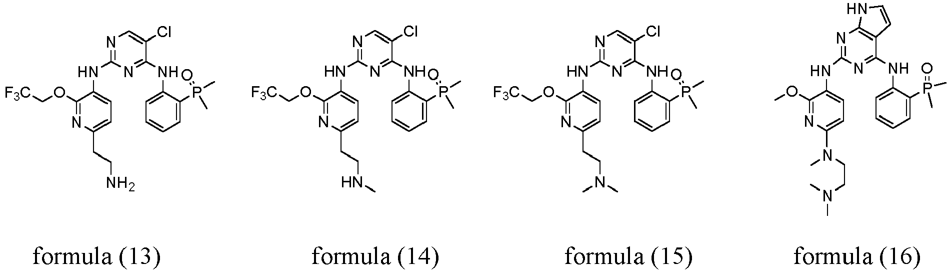

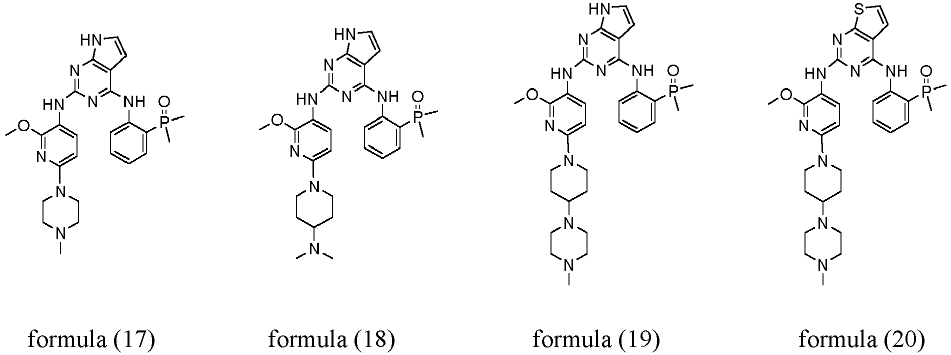

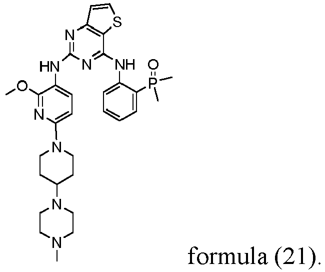

Compounds described herein can comprise one or more asymmetric centers, and thus can exist in various stereoisomeric forms, *e*.*g*., enantiomers and/or diastereomers. For example, the compounds described herein may be in the form of an individual enantiomer, diastereomer or geometric isomer (such as cis- and trans-isomer), or may be in the form of a mixture of stereoisomers, including racemic mixtures and mixtures enriched in one or more stereoisomer. Isomers may be isolated from mixtures by methods known to those skilled in the art, including chiral high pressure liquid chromatography (HPLC) and the formation and crystallization of chiral salts; or preferred isomers may be prepared by asymmetric syntheses.

Also disclosed herein are all suitable isotopic derivatives of the compounds disclosed herein. An isotope derivative of a compound disclosed herein is defined as wherein at least one atom is replaced by an atom having the same atomic number but differing in atomic mass from the atomic mass typically found in nature. Examples of isotopes that may be listed as compounds disclosed herein include hydrogen, carbon, nitrogen, oxygen, fluorine, and chlorine isotopes, such as ²H, ³H, ¹³C, ¹⁴C, ¹⁵N, ¹⁷O, ¹⁸O, ¹⁸F, ³¹P, ³²P, ³⁵S and ³⁶Cl, respectively. Certain isotopic derivatives of the compounds disclosed herein, such as the radioisotopes of ³H and ¹⁴C, are also among them and are useful in the tissue distribution experiments of drugs and substrates. Tritium, i.e., ³H, and carbon-14, i.e., ¹⁴C, are easier to prepare and detect and are the first choice for isotopes. In addition, substitution with isotopes such as deuterium, i.e., ²H, has advantages in some therapies due to its good metabolic stability, for example, increased half-life in vivo or reduced dosage, and thus priority may be given in some cases. Isotopic derivatives of the compounds disclosed herein may be prepared by conventional procedures, for example by descriptive methods or by the preparations described in the Examples below, using appropriate isotopic derivatives of the appropriate reagents.

The compound of the present disclosure or a pharmaceutically acceptable salt thereof may be in an amorphous or crystalline form. Furthermore, the compounds of the present disclosure may exist in one or more crystalline forms. Accordingly, the disclosure includes within its scope all amorphous or crystalline forms of the compounds disclosed herein. The term "polymorph" refers to a crystalline form (or a salt, hydrate or solvate thereof) of a compound in a particular crystal packing arrangement. All polymorphs have the same elemental composition. Different crystalline forms typically have different X-ray diffraction patterns, infrared spectra, melting points, density, hardness, crystal shape, photoelectric properties, stability, and solubility. Recrystallization solvents, crystallization rates, storage temperatures, and other factors can result in a crystalline form that predominates. Various polymorphs of the compounds may be prepared by crystallization under different conditions.

Those skilled in the art will appreciate that many organic compounds can form complexes with solvents that react in or precipitate or crystallize from the solvent. These complexes are referred to as "solvates." When the solvent is water, the complex is referred to as a "hydrate." The disclosure encompasses all solvates of the compounds disclosed herein.

The term "prodrug" if used herein refers to a compound which is converted in vivo to an active form thereof having a medical effect by, for example, hydrolysis in blood. Pharmaceutically acceptable prodrugs are described in T. Higuchi and V Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130.

A prodrug is any covalently bonded carrier which, when administered to a patient, releases the compound of formula (I) in vivo. Prodrugs are typically prepared by modifying functional groups in such a way that the modifications may be cleaved by routine manipulation or in vivo to yield the parent compound. Prodrugs include, for example, compounds disclosed herein wherein a hydroxyl, amine or sulfhydryl group is bonded to any group which, when administered to a patient, may be cleaved to form a hydroxyl, amine or sulfhydryl group. Thus, representative examples of prodrugs include, but are not limited to, acetate, formate and benzoate derivatives of alcohol, mercapto and amine functional groups of the compounds of formula (I). Further, in the case of a carboxylic acid (-COOH), an ester such as a methyl ester, an ethyl ester or the like may be used. The ester itself may be active and/or may hydrolyze under conditions in human bodies. Suitable pharmaceutically acceptable hydrolysable in vivo ester groups include those groups which readily decompose in the human body to release the parent acid or a salt thereof.

### Pharmaceutical compositions, formulations and kits

In another aspect, the disclosure provides a pharmaceutical composition comprising a compound of the present disclosure (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other materials) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

The following formulation examples illustrate representative pharmaceutical compositions that may be prepared in accordance with this disclosure. The present disclosure, however, is not limited to the following pharmaceutical compositions.

*Exemplary Formulation 1* - *Tablets: A compound of the present disclosure may be admixed as a dry powder with a dry gelatin binder in an approximate 1:2 weight ratio. A minor amount of magnesium stearate is added as a lubricant. The mixture is formed into 0.3-30 mg tablets (0.1-10 mg of active compound per tablet) in a tablet press.*

*Exemplary Formulation 2* - *Tablets: A compound of the present disclosure may be admixed as a dry powder with a dry gelatin binder in an approximate 1:2 weight ratio. A minor amount of magnesium stearate is added as a lubricant. The mixture is formed into 30-90 mg tablets (10-30 mg of active compound per tablet) in a tablet press.*

*Exemplary Formulation 3* - *Tablets: A compound of the present disclosure may be admixed as a dry powder with a dry gelatin binder in an approximate 1:2 weight ratio. A minor amount of magnesium stearate is added as a lubricant. The mixture is formed into 90-150 mg tablets (30-50 mg of active compound per tablet) in a tablet press.*

*Exemplary Formulation 4* - *Tablets: A compound of the present disclosure may be admixed as a dry powder with a dry gelatin binder in an approximate 1:2 weight ratio. A minor amount of magnesium stearate is added as a lubricant. The mixture is formed into 150-240 mg tablets (50-80 mg of active compound per tablet) in a tablet press.*

*Exemplary Formulation 5* - *Tablets:* A compound of the present disclosure may be admixed as a dry powder with a dry gelatin binder in an approximate 1:2 weight ratio. A minor amount of magnesium stearate is added as a lubricant. The mixture is formed into 240-270 mg tablets (80-90 mg of active compound per tablet) in a tablet press.

*Exemplary Formulation 6* - *Tablets: A compound of the present disclosure may be admixed as a dry powder with a dry gelatin binder in an approximate 1:2 weight ratio. A minor amount of magnesium stearate is added as a lubricant. The mixture is formed into 270-450 mg tablets (90-150 mg of active compound per tablet) in a tablet press.*

*Exemplary Formulation 7* - *Tablets: A compound of the present disclosure may be admixed as a dry powder with a dry gelatin binder in an approximate 1:2 weight ratio. A minor amount of magnesium stearate is added as a lubricant. The mixture is formed into 450-900 mg tablets (150-300 mg of active compound) in a tablet press.*

*Exemplary Formulation 8* - *Capsules: A compound of the present disclosure may be admixed as a dry powder with a starch diluent in an approximate 1:1 weight ratio. The mixture is filled into 250 mg capsules (125 mg of active compound per capsule).*

*Exemplary Formulation 9* - *Liquid: A compound of the present disclosure (125 mg) may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color are diluted with water and added with stirring. Sufficient water may then be added to produce a total volume of 5 mL.*

*Exemplary Formulation 10* - *Injection: A compound of the present disclosure may be dissolved or suspended in a buffered sterile saline injectable aqueous medium to a concentration of approximately 5 mg*/*mL.*

### Administration

The pharmaceutical composition provided by the present disclosure may be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, *e.g*., within the therapeutic window over the extended period of time.

The pharmaceutical compositions of the present disclosure may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g*., through an IV drip) allows a much faster delivery which quickly raises the concentration of the active ingredient in the blood to an effective level. In other embodiments, the pharmaceutical composition may be administered as a continuous infusion, *e.g*., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with preferred doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and yet alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration may be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington 's Pharmaceutical Sciences, 17th edition, 1985, Mack Publishing Company, Easton, Pennsylvania.

The compounds of the present disclosure can also be administered in sustained release forms or from sustained release drug delivery systems. A description of representative sustained release materials may be found in Remington's Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are α-, β- and γ- cyclodextrins consisting of 6, 7 and 8 α-1 ,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, *e.g*., for example, sulfobutyl ether β-cyclodextrin, also known as Captisol. See, *e.g*., U.S. 5,376,645. In certain embodiments, the formulation comprises hexapropyl-β-cyclodextrin (*e.g*., 10-50% in water).

### Treatment method

The present disclosure provides the administration of a compound of the present disclosure, or a pharmaceutically acceptable salt, stereoisomer, solvate, hydrate, crystalline form, prodrug or isotopic derivative thereof, or the pharmaceutical composition according to the disclosure, to a subject in need of treatment, for use in the treatment of cancer. In some embodiments, the cancer is ALK-driven cancer. In some embodiments, the cancer is non-small cell lung cancer.

A "therapeutically effective amount" is that amount effective for detectable killing or inhibition of the growth or spread of cancer cells; the size or number of tumors; or other measure of the level, stage, progression or severity of the cancer. The exact amount required can vary from subject to subject, depending on the species, age, and general condition of the subject, the severity of the disease, the particular anticancer agent, its mode of administration, combination treatment with other therapies, and the like.

Disclosed herein are compounds having biological properties which make them of interest for treating or modulating disease in which kinases can be involved, symptoms of such disease, or the effect of other physiological events mediated by kinases. For instance, a number of compounds as disclosed herein have been shown to inhibit tyrosine kinase activity of ALK, FAK and c-Met, among other tyrosine kinases which are believed to mediate the growth, development and/or metastasis of cancer. A number of compounds as disclosed herein have also been found to possess potent in vitro activity against cancer cell lines. Such compounds are thus of interest for the treatment of cancers, including solid tumors as well as lymphomas and including cancers which are resistant to other therapies.

In some embodiments, the cancer is an ALK driven cancer. In some embodiments, the cancer is non-small cell lung cancer (NSCLC). In some embodiments, the cancer is ALK-positive NSCLC. In some embodiments, the cancer is locally advanced or metastatic ALK-positive NSCLC. In some embodiments, the cancer/patient has previously been treated with crizotinib or another tyrosine kinase inhibitor. In some embodiments, the cancer/patient has not previously been treated with an ALK inhibitor.

Such cancers include, but are not limited to, cancers of the breast, non small cell lung cancer (NSCLC), neural tumors such as glioblastomas and neuroblastomas; esophageal carcinomas, soft tissue cancers such as rhabdomyosarcomas, among others; various forms of lymphoma such as a non-Hodgkin's lymphoma (NHL) known as anaplastic large-cell lymphoma (ALCL), various forms of leukemia; and including cancers which are ALK or c-met mediated.

Anaplastic Lymphoma Kinase (ALK) is a cell membrane-spanning receptor tyrosine kinase, which belong to the insulin receptor subfamily. ALK receptor tyrosine kinase (RTK) was initially identified due to its involvement in the human non-Hodgkin lymphoma subtype known as anaplastic large-cell lymphoma (ALCL). ALK normally has a restricted distribution in mammalian cells, being found at significant levels only in nervous system during embryonic development, suggesting a possible role for ALK in brain development.

In addition to its role in normal development, the expression of full-length normal ALK has been detected in cell lines derived from various tumors, such as glioblastoma, neuroectodermal tumors and glioblastoma and breast cancer and melanoma lines.

In common with other RTKs, translocations affect the ALK gene, resulting in expression of oncogenic fusion kinases, the most common of which is NPM-ALK. For example, approximately sixty percent of anaplastic large cell lymphomas (ALCL) are associated with a chromosome mutation that generates a fusion protein consisting of nucleophosmin (NMP) and the intracellular domain of ALK. This mutant protein, NPM-ALK, possesses a constitutively active tyrosine kinase domain that is responsible for its oncogenic property through activation of downstream effectors. Experimental data have demonstrated that the aberrant expression of constitutively active ALK is directly implicated in the pathogenesis of ALCL and that inhibition of ALK can markedly impair the growth of ALK positive lymphoma cells. The constitutively activated chimeric ALK has also been demonstrated in about 60% of inflammatory myofibroblastic tumors (IMTs), a slow growing sarcoma that mainly affects children and young adults. Furthermore, recent reports have also described the occurrence of a variant ALK fusion, TPM4-ALK, in cases of squamous cell carcinoma (SCC) of the esophagus. Thus, ALK is one of the few examples of an RTK implicated in oncogenesis in both non-hematopoietic and hematopoietic malignancies. More recently, it has been shown that a small inversion within chromosome 2p results in the formation of a fusion gene comprising portions of the echinoderm microtubule-associated protein-like 4 (EML4) gene and the anaplastic lymphoma kinase (ALK) gene in non-small-cell lung cancer (NSCLC) cells.

In some embodiments, an ALK inhibitor can create durable cures when used as a single therapeutic agent or combined with current chemotherapy for ALCL, IMT, proliferative disorders, glioblastoma and other possible solid tumors cited herein, or, as a single therapeutic agent, could be used in a maintenance role to prevent recurrence in patients in need of such a treatment.

Compounds as disclosed herein can be administered as part of a treatment regimen in which the compound is the sole active pharmaceutical agent, or used in combination with one or more other therapeutic agents as part of a combination therapy. When administered as one component of a combination therapy, the therapeutic agents being administered can he formulated as separate compositions that are administered at the same time or sequentially at different times (e.g., within 72 hours, 48 hours, or 24 hours of one another), or the therapeutic, agents can be formulated together in a single pharmaceutical composition and administered simultaneously.

Thus, the administration of compounds as disclosed herein in a form disclosed herein can be in conjunction with at least one additional therapeutic agent known to those skilled in the art in the prevention or treatment of cancer, such as radiation therapy or cytostatic agents, cytotoxic agents, other anti-cancer agents and other drugs to ameliorate symptoms of the cancer or side effects of any of the drugs. Non-limiting examples of additional therapeutic agents include agents suitable for immunotherapy (such as, for example, PD-1 and PDL-1 inhibitors), antiangiogenesis (such as, for example, bevacizumab), and/or chemotherapy.

If formulated as a fixed dose, such combination products employ compounds as disclosed herein within the accepted dosage ranges. Compounds as disclosed herein can also be administered sequentially with other anticancer or cytotoxic agents when a combination formulation is appropriate. Compounds as disclosed herein can be administered prior to, simultaneously with, or after administration of the other anticancer or cytotoxic agent,

Currently, standard treatment of primary tumors consists of surgical excision, when appropriate, followed by either radiation or chemotherapy, and typically administered intravenously. The typical chemotherapy regime consists of either DNA alkylating agents, DNA intercalating agents. CDK inhibitors, or microtubule poisons. The chemotherapy doses used are just below the maximal tolerated dose and therefore dose limiting toxicities typically include, nausea, vomiting, diarrhea, hair loss, neutropenia and the like.

There are large numbers of antineoplastic agents available in commercial use, in clinical evaluation and in pre-clinical development, which would be selected for treatment of cancer by combination drug chemotherapy. And there are several major categories of such antineoplastic agents, namely, antibiotic-type agents, alkylating agents, antimetabolite agents, anti-hormonal agents, immunological agents, interferon-type agents and a category of miscellaneous agents.

Examples of other therapeutic agents include but not limited to one or more of the following: an anti-cancer alkylating or intercalating agent (e.g., mechlorethamine, chlorambucil, Cyclophosphamide, Melphalan, and Ifosfamide); antimetabolite (e.g., Methotrexate); purine antagonist or pyrimidine antagonist (e.g., 5-Fluorouracil, Cytarabile, and Gemcitabine); spindle poison (e.g., Vinblastine, Vincristine, Vinorelbine and Paclitaxel); podophyllotoxin (e.g., Etoposide, Irinotecan, Topotecan); antibiotic (e.g., Doxorubicin, Bleomycin and Mitomycin); nitrosourea (e.g., Carmustine, Lomustine); inorganic ion (e.g., Cisplatin, Carboplatin, Oxaliplatin or oxiplatin); enzyme (e.g., Asparaginase); hormone (e.g., Tamoxifen, Leuprolide, Flutamide or Megestrol); mTOR inhibitor (e.g., Sirolimus (rapamycin), Temsirolimus (CCI779), Everolimus (RAD00l), AP23573 or other compounds disclosed in US Patent No. 7,091,213); proteasome inhibitor (such as Velcade, other proteasome inhibitors (e.g., an inhibitor of Src, Bcr/Abl, kdr, flt3, aurora-2, glycogen synthase kinase 3 (GSK-3), EGFR kinase (e.g., Iressa, Tarceva, etc.), VEGF-R kinase, PDGF-R kinase, etc.); an antibody, soluble receptor or other receptor antagonist against a receptor or hormone implicated in a cancer (including receptors such as EGFR, ErbB2, VEGFR, PDGFR, and IGF-R; and agents such as Herceptin, Avastin, Erbitux, etc.); etc. Examples of other therapeutic agents include among others, Zyloprim, alemtuzmab, altretamine, amifostine, nastrozole, antibodies against prostate-specific membrane antigen (such as MLN-591, MLN591RL and MLN2704), arsenic trioxide, bexarotene, bleomycin, busulfan, capecitabine, Gliadel Wafer, celecoxib, chlorambucil, cisplatin-epinephrine gel, cladribine, cytarabine liposomal, daunorubicin liposomal, daunorubicin, daunomycin, dexrazoxane, docetaxel, doxorubicin, Elliott's B Solution, epirubicin, estramustine, etoposide phosphate, exemestane, fludarabine, 5-FU, fulvestrant, gemcitabine, gemtuzumab-ozogamicin, goserelin acetate, hydroxyurea, idarubicin, Idamycin, ifosfamide, imatinib mesylate, irinotecan (or other topoisomerase inhibitor, including antibodies such as MLN576 (XRl1576)), letrozole, leucovorin, leucovorin levamisole, liposomal daunorubicin, melphalan, L-PAM, mesna, methotrexate, methoxsalen, mitomycin C, mitoxantrone, MLN518 or MLN608 (or other inhibitors of the flt-3 receptor tyrosine kinase, PDFG-R or c-kit), itoxantrone, paclitaxel, Pegademase, pentostatin, Rituximab, talc, tamoxifen, temozolamide, teniposide, VM-26 , topotecan, toremifene, 2C4 (or other antibody which interferes with HER2-mediated signaling), tretinoin, ATRA, valrubicin, vinorelbine, or pamidronate, zoledronate or other bisphosphonate.

### Examples

The present disclosure will be further described below in combination with specific embodiments. It should be understood that these examples are only used to illustrate the present disclosure and not to limit the scope of the present disclosure. The experimental methods without specific conditions in the following examples generally follow the conventional conditions or the conditions recommended by the manufacturer. Unless otherwise stated, parts and percentages are parts by weight and weight percent.

Generally, in the preparation process, each reaction is usually carried out in an inert solvent at a temperature from room temperature to reflux temperature (such as 0 °C to 100 °C, alternatively 0 °C to 80 °C). The reaction time is usually 0.1-60 hours, alternatively 0.5-24 hours.

### Example 1 Preparation of (2-((5-chloro-2-((6-(4-(dimethylamino)piperidin-1-yl)-2-methoxypyridin-3-yl)amino)pyr imidin-4-yl)amino)phenyl)dimethylphosphine oxide (compound T-1)

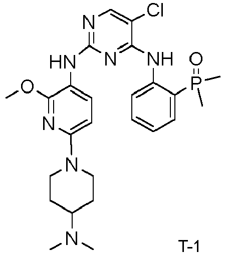

The following route was used for the synthesis:

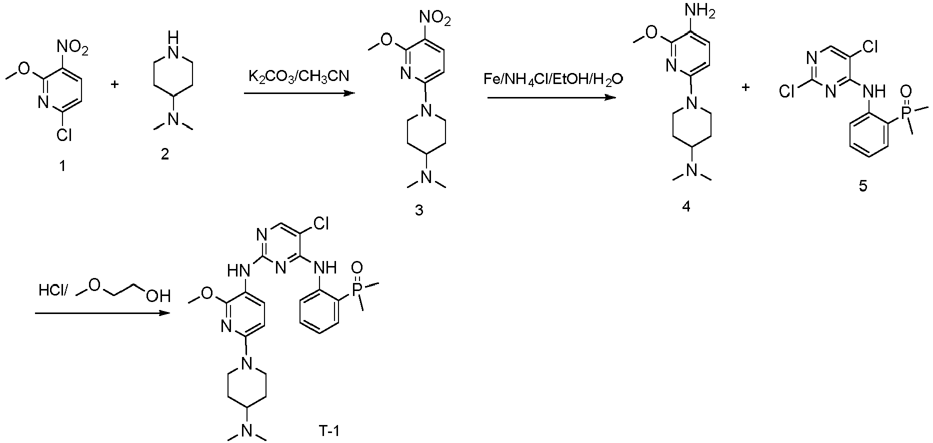

### Step 1 Synthesis of compound 3

To a 50 mL single-necked flask equipped with a magnetic stirrer compound 1 (1.0 g, 5.3 mmol) and acetonitrile (10 mL) were added in sequence, and the mixture was stirred to form a solution. The hydrochloride salt of compound 2 (1.31 g, 7.95 mmol) and potassium carbonate (3.66 g, 26.5 mmol) were added, and the reaction mixture was heated to 80 °C under a nitrogen atmosphere and reacted overnight with stirring at the same temperature. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to afford 1.3 g of a yellow solid. Yield: 87.5%. LC-MS(APCI): m/z=281.2(M+1)⁺.

### Step 2 Synthesis of compound 4

To a 50 mL single-necked flask equipped with a magnetic stirrer and a condenser tube compound 3 (1.3 g, 4.64 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and stirred at this temperature for 1 hour. The reaction was cooled to room temperature, filtered, and the filter cake was washed with ethanol (5 mL). The resulting solution was concentrated to remove the organic solvent, extracted with dichloromethane (40 mLx3). The organic phases were combined, dried over anhydrous sodium sulfate, filtered, and concentrated to give 970 mg of a brown solid. Yield: 83.6%. LC-MS(APCI): m/z=251.3(M+1)⁺.

### Step 3 Synthesis of compound T-1

To a 25 mL single-necked flask equipped with a magnetic stirrer and a condenser tube compound 4 (150 mg, 0.6 mmol), compound 5 (189 mg, 0.6 mmol) and ethylene glycol monomethyl ether (3 mL) were added, and the solution was stirred to form a solution. A solution of hydrogen chloride in isopropanol (0.9 mmol, 0.18 mL, 5M) was added dropwise, and the temperature of the mixture was raised to 120 °C under a nitrogen atmosphere and the reaction was stirred at the same temperature overnight. The reaction was cooled to room temperature, water (10 mL) and saturated sodium bicarbonate (5 mL) were added, and extracted with dichloromethane (15 mLx3). The organic phases were combined, wash with saturated brine, dried over anhydrous sodium sulfate, filtered, and concentrated. The residue was passed through a silica gel column to afford 200 mg of a white solid. Yield: 63.0%. LC-MS(APCI): m/z=530.2(M+1)⁺. ¹H NMR(300 MHz, CDCl₃) δ (ppm): 11.07(s, 1H), 8.84(dd, J=6.3 Hz, J=3.3 Hz, 1H), 8.47(d, J=6.3 Hz, 1H), 8.33(s, 1H), 7.74(t, J=5.7 Hz, 1H), 7.57-7.52(m, 2H), 7.38(t, J=5.7 Hz, 1H), 6.44(t, J=6.3 Hz, 1H), 4.53(d, J=9.6 Hz, 2H), 4.21(s, 3H), 3.03(t, J=9.3 Hz, 2H), 2.67(t, J=8.4 Hz, 1H), 2.61(s, 6H), 2.21(d, J=9.3 Hz, 2H), 2.10(d, J=9.6 Hz, 6H), 1.86-1.84(m, 2H).

### Example 2 Preparation of (2-((5-chloro-2-((2-methoxy-6-(4-methylpiperazin-1-yl)pyridin-3-yl)amino)pyrimidin-4-yl)amino)phenyl)dimethylphosphine oxide (compound T-2)

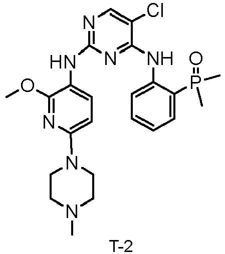

The following route was used for the synthesis:

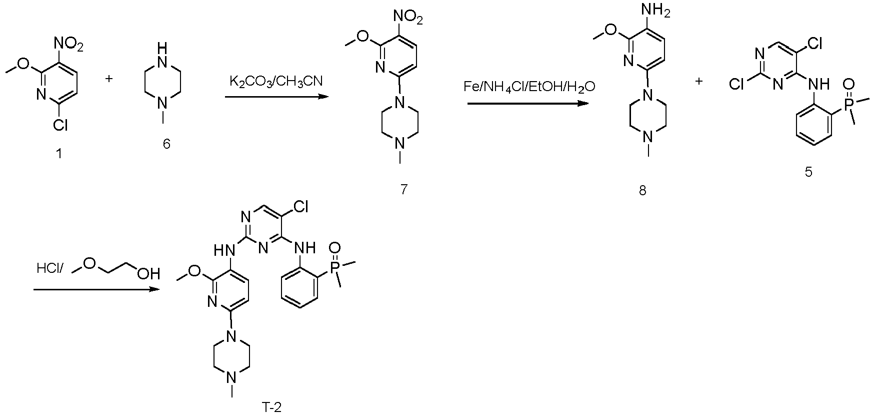

### Step 1 Synthesis of compound 7

To a 50 mL single-necked flask equipped with a magnetic stirrer compound 1 (1.0 g, 5.3 mmol) and acetonitrile (10 mL) were added in sequence, and the mixture was stirred to form a solution. N-methylpiperazine (compound 6, 0.8 g, 7.95 mmol) and potassium carbonate (1.46 g, 10.6 mmol) were added, and the reaction mixture was heated to 80 °C under a nitrogen atmosphere and the reaction was stirred at this temperature overnight. The reaction was cooled to room temperature, the resulting solution was diluted with ethyl acetate (50 mL), the inorganic salt was filtered off, and the filter cake was washed with ethyl acetate (10 mL). The filtrate was combined, concentrated, and the residue was passed through a silica gel column to give 1.1 g of a yellow solid. Yield: 82.4%. LC-MS(APCI): m/z=253.2(M+1)⁺.

### Step 2 Synthesis of compound 8

To a 50 mL single-necked flask equipped with a magnetic stirrer and a condenser tube compound 7 (1.1 g, 4.36 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The resulting solution was concentrated to remove the organic solvent, and extracted with dichloromethane (40 mLx3). The organic phases were combined, dried over anhydrous sodium sulfate, filtered, and concentrated to give 800 mg of a brown solid. Yield: 79.1%. LC-MS(APCI): m/z=223.3(M+1)⁺.

### Step 3 Synthesis of compound T-2

To a 25 mL single-necked flask equipped with a magnetic stirrer and a condenser tube compound 8 (133 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filtered, and concentrated. The residue was passed through a silica gel column to give 160 mg of white solid. Yield: 50.1%. LC-MS(APCI): m/z=502.2(M+1)⁺. ¹H NMR(300 MHz, CDCl₃) δ (ppm): 10.35(s, 1H), 8.06(dd, J=6.0 Hz, J=3.0 Hz, 1H), 7.82(d, J=6.0 Hz, 1H), 7.60(s, 1H), 7.00(t, J=5.7 Hz, 1H), 6.87(s, 1H), 6.72(d, J=2.7 Hz, 1H), 6.53(d, J=2.7 Hz, 1H), 6.17(d, J=6.6 Hz, 1H), 3.94(s, 3H), 3.56(t, J=3.6 Hz, 4H), 2.70(t, J=3.6 Hz, 4H), 2.44(s, 3H), 2.65(s, 6H), 1.81(d, J=9.6 Hz, 6H).

### Example 3 Preparation of (2-((5-chloro-2-((6-((2-(dimethylamino)ethyl)(methyl)amino)-2-methoxypyridin-3-yl)am ino)pyrimidin-4-yl)amino)phenyl)dimethylphosphine oxide (compound T-3)

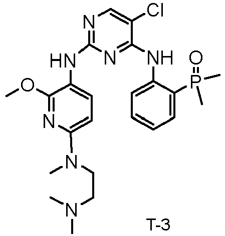

The following route was used for the synthesis:

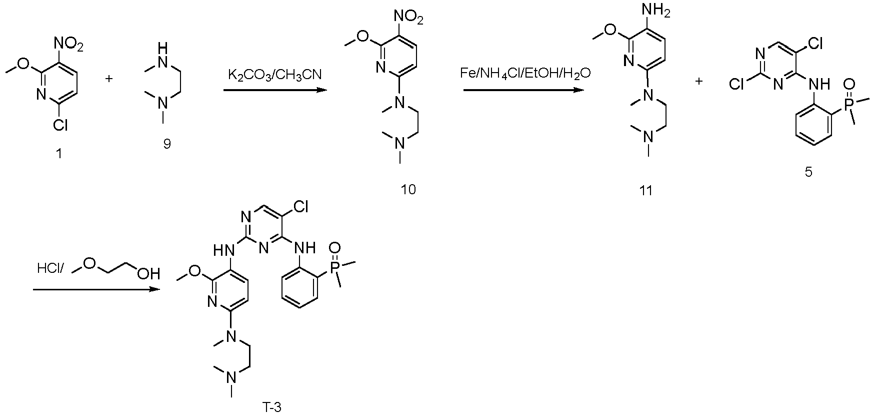

### Step 1 Synthesis of compound 10

To a 50 mL single-necked flask equipped with a magnetic stirrer compound 1 (1.0 g, 5.3 mmol) and acetonitrile (10 mL) were added in sequence, and the mixture was stirred to form a solution. Compound 9 (0.8 g, 7.95 mmol) and potassium carbonate (1.46 g, 10.6 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to give 1.1 g of a yellow solid. Yield: 82.4%. LC-MS(APCI): m/z=255.2(M+1)⁺.

### Step 2 Synthesis of compound 11

To a 50 mL single-necked flask equipped with a magnetic stirrer and a condenser tube compound 10 (1.1 g, 4.36 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The resulting solution was concentrated to remove the organic solvent, and extracted with dichloromethane (40 mLx3). The organic phases were combined, dried over anhydrous sodium sulfate, filtered, and concentrated to give 800 mg of a brown solid. Yield: 79.1%. LC-MS(APCI): m/z=225.3(M+1)⁺.

### Step 3 Synthesis of compound T-3

To a 25 mL single-necked flask equipped with a magnetic stirrer and a condenser tube compound 11 (133 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filtered, and concentrated. The residue was passed through a silica gel column to afford 160 mg of a white solid. Yield: 50.1%. LC-MS(APCI): m/z=504.2(M+1)⁺. ¹H NMR(300 MHz, CDCl₃) δ (ppm): 11.84(s, 1H), 8.57(dd, J=6.3 Hz, J=3.3 Hz, 1H), 8.21(d, J=6.6 Hz, 1H), 8.06(s, 1H), 7.48(t, J=6.0 Hz, 1H), 7.31-7.26(m, 1H), 7.12(t, J=5.7 Hz, 1H), 6.91(s, 1H), 6.05(d, J=6.6 Hz, 3H), 4.03(t, J=5.4 Hz, 2H), 3.94(s, 3H), 3.13(t, J=5.1 Hz, 2H), 3.06(s, 3H), 2.75(s, 6H), 1.83(d, J=9.6 Hz, 6H).

### Example 4 Preparation of (2-((5-chloro-2-((2-methoxy-6-(4-(4-methylpiperazin-1-yl)piperidin-1-yl)pyridin-3-yl) mino)pyrimidin-4-yl)amino)phenyl)dimethylphosphine oxide (compound T-4)

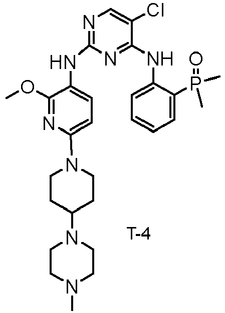

The following route was used for the synthesis:

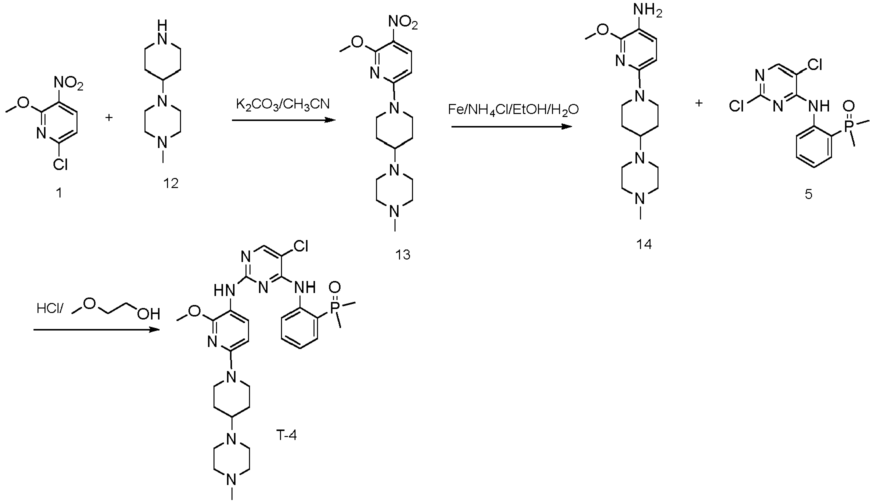

### Step 1 Synthesis of compound 13

To a 50 mL single-necked flask equipped with a magnetic stirrer compound 1 (1.0 g, 5.3 mmol) and acetonitrile (10 mL) were added in sequence, and the mixture was stirred to form a solution. Compound 12 (0.97 g, 7.95 mmol) and potassium carbonate (1.46 g, 10.6 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to afford 1.2 g of a yellow solid. Yield: 67.6%. LC-MS(APCI): m/z=336.2(M+1)⁺.

### Step 2 Synthesis of compound 14

To a 50 mL single-necked flask equipped with a magnetic stirrer and a condenser tube compound 13 (1.2 g, 3.58 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The solution was concentrated to remove the organic solvent, extracted with dichloromethane (40 mLx3), and the organic phases were combined, dried over anhydrous sodium sulfate, filtered, and concentrated to give 800 mg of a brown solid. Yield: 71.3%. LC-MS(APCI): m/z=306.3(M+1)⁺.

### Step 3 Synthesis of compound T-4

To a 25 mL single-necked flask equipped with a magnetic stirrer and a condenser tube compound 14 (183 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filtered, and concentrated. The residue was passed through a silica gel column to afford 160 mg of a white solid. Yield: 45.6%. LC-MS(APCI): m/z=585.2(M+1)⁺. ¹H NMR(400 MHz, DMSO-D₆) δ (ppm): 11.19(s, 1H), 8.45(br s, 1H), 8.21(s, 1H), 8.04(s, 1H), 7.56-7.52(m, 2H), 7.26-7.23(m, 1H), 7.08(t, J=8.0 Hz, 1H), 6.35(d, J=8.0Hz, 1H), 4.29(d, J=13.2 Hz, 2H), 3.78(s, 3H), 3.35-2.89(m, 7H), 2.83-2.77(m, 4H), 2.56(s, 3H), 1.91-1.88(m, 2H), 1.77(s, 3H), 1.74(s, 3H), 1.44-1.39(m, 2H).

### Example 5 Preparation of (2-((5-chloro-2-((6-(4-(dimethylamino)piperidin-1-yl)-2-isopropoxypyridin-3-yl)amino) pyrimidin-4-yl)amino)phenyl)dimethylphosphine oxide (compound T-5)

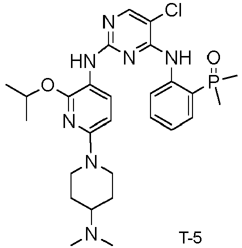

The following route was used for the synthesis:

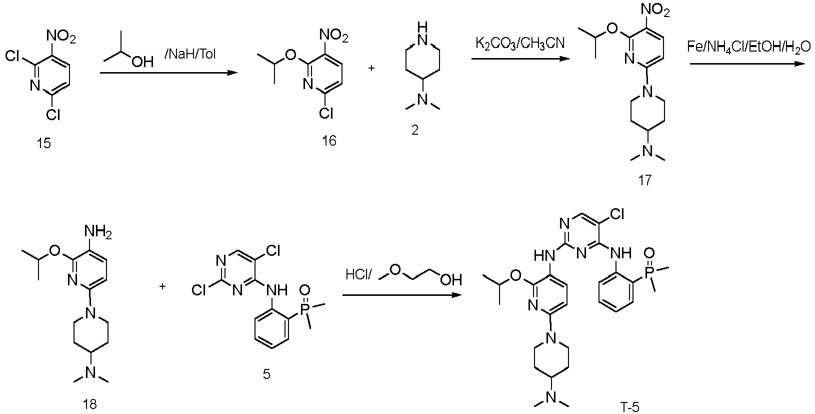

### Synthetic step 1 Synthesis of compound 16

To a 250 mL single-necked flask equipped with a magnetic stirrer compound 15 (5.79 g, 30 mmol) and dry toluene (50 mL) were added, and the mixture was stirred to form a solution. Isopropanol (2.16 g, 36 mmol) was added, and the mixture was cooled to 0 °C before NaH (1.56 g, 39 mmol, 60%) was added, and the solution was stirred for half an hour under a nitrogen atmosphere. Then the ice bath was removed, and the reaction was stirred at room temperature overnight. The reaction was quenched by adding water (50 mL), the organic layer was separated, and the aqueous phase was extracted with ethyl acetate (40 mL × 2). The organic phases were combined, dried over anhydrous sodium sulfate, and filtered, and the filtrate was concentrated and the residue was passed through a silica gel column to afford 3.11 g of a white solid. Yield: 48.2%. LC-MS(APCI): m/z=217.2(M+1)⁺. ¹H NMR(400 MHz, CDCl₃) δ ppm: 8.25(d, J=8.0 Hz, 1H), 7.01(d, J=8.0 Hz, 1H), 5.56-5.52(m, 1H), 1.47(d, J=6.0 Hz, 6H).

### Step 2 Synthesis of compound 17

To a 50 mL single-necked flask equipped with a magnetic stirrer compound 16 (1.1 g, 5.3 mmol) and acetonitrile (10 mL) were added in sequence, and the mixture was stirred to form a solution. The hydrochloride salt of compound 2 (1.31 g, 7.95 mmol) and potassium carbonate (3.66 g, 26.5 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to give 1.1 g of a yellow solid. Yield: 67.4%. LC-MS(APCI): m/z=309.2(M+1)⁺.

### Step 3 Synthesis of compound 18

To a 50 mL single-necked flask equipped with a magnetic stirrer and a condenser tube compound 17 (1.1 g, 3.57 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The solution was concentrated to remove the organic solvent, and extracted with dichloromethane (40 mLx3). The organic phases were combined, dried over anhydrous sodium sulfate, filtered, and concentrated to give 800 mg of a brown solid. Yield: 80.6%. LC-MS(APCI): m/z=279.3(M+1)⁺.

### Step 4 Synthesis of compound T-5

To a 25 mL single-necked flask equipped with a magnetic stirrer and a condenser tube compound 18 (166 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filtered, and concentrated. The residue was passed through a silica gel column to afford 200 mg of a white solid. Yield: 59.8%. LC-MS(APCI): m/z=558.2(M+1)⁺. ¹H NMR(400MHz, DMSO-D₆) δ ppm: 11.18(s, 1H), 8.44(s, 1H), 8.07(s, 1H), 8.05(s, 1H), 7.64(d, J=8.0Hz, 1H), 7.57-7.51(m, 1H), 7.31(t, J=8.0Hz, 1H), 7.11(t, J=8.0Hz, 1H), 6.38(d, J=8.4Hz, 1H), 5.17-5.13(m, 1H), 4.36(d, J=10.8Hz, 2H), 3.46-3.42(m, 1H), 2.80(t, J=12.8Hz, 2H), 2.71(s, 6H), 2.10(d, J=11.6Hz, 2H), 1.79(s, 3H), 1.75(s, 3H), 2.18-2.15(m, 2H), 1.22(d, J=6.0Hz, 6H).

### Example 6 Preparation of (2-((5-chloro-2-((2-isopropoxy-6-(4-methylpiperazin-1-yl)pyridin-3-yl)amino)pyrimidin -4-yl)amino)phenyl)dimethylphosphine oxide (compound T-6)

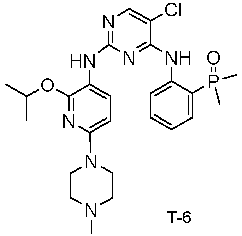

The following route was used for the synthesis:

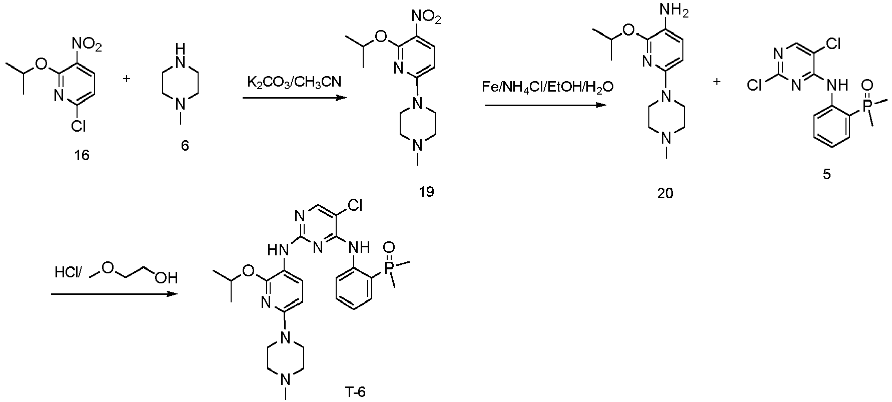

### Step 1 Synthesis of compound 19

To a 50 mL single-necked flask equipped with a magnetic stirrer compound 16 (1.1 g, 5.3 mmol) and acetonitrile (10 mL) were added in sequence, and the mixture was stirred to form a solution. Compound 6 (0.8 g, 7.95 mmol) and potassium carbonate (1.46 g, 10.6 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to give 1.1 g of a yellow solid. Yield: 73.9%. LC-MS(APCI): m/z=281.2(M+1)⁺.

### Step 2 Synthesis of compound 20

To a 50 mL single-necked flask equipped with a magnetic stirrer and a condenser tube compound 19 (1.1 g, 3.93 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The solution was concentrated to remove the organic solvent, and extracted with dichloromethane (40 mLx3). The organic phases was combined, dried over anhydrous sodium sulfate, filtered, and concentrated to give 800 mg of a brown solid. Yield: 81.4%. LC-MS(APCI): m/z=251.3(M+1)⁺.

### Step 3 Synthesis of compound T-6

To a 25 mL single-necked flask equipped with a magnetic stirrer and a condenser tube compound 20 (150 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the solution was extracted with dichloromethane (15 mLx3). The organic phases were combined, washed with saturated brine, dried over anhydrous sodium sulfate, filtered, and concentrated. The residue was passed through a silica gel column to afford 160 mg of a white solid. Yield: 50.1%. LC-MS(APCI): m/z=530.2(M+1)⁺. ¹H NMR(400MHz, DMSO-D₆) δ ppm: 11.18(s, 1H), 8.41(s, 1H), 8.08-8.06(m, 2H), 7.64(d, J=8.0Hz, 1H), 7.57-7.51(m, 1H), 7.31(t, J=8.0Hz, 1H), 7.11(t, J=8.0Hz, 1H), 6.38(d, J=8.4Hz, 1H), 5.17-5.13(m, 1H), 3.57-3.52(m, 4H), 3.34-3.28(m, 4H), 2.79(s, 3H), 1.78(s, 3H), 1.75(s, 3H), 1.20(d, J=6.4 Hz, 6H).

### Example 7 Preparation of (2-((5-chloro-2-((6-((2-(dimethylamino)ethyl)(methyl)amino)-2-isopropoxypyridin-3-yl) amino)pyrimidin-4-yl)amino)phenyl)dimethylphosphine oxide (compound T-7)

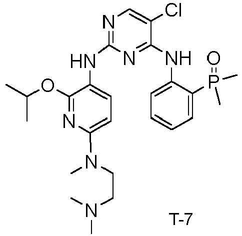

The following route was used for the synthesis:

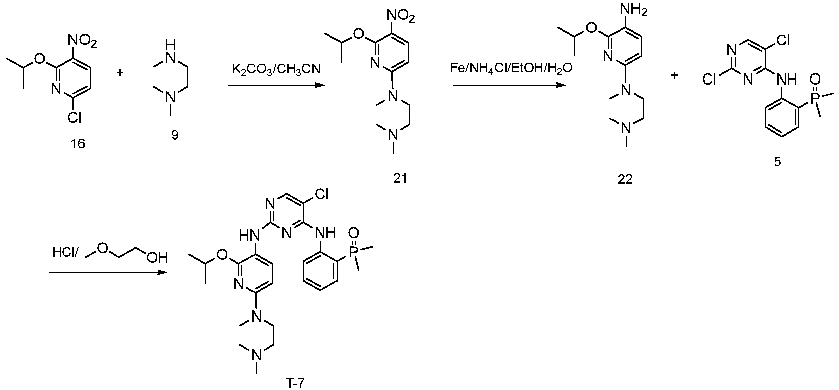

### Step 1 Synthesis of compound 21

To a 50 mL single-necked flask equipped with a magnetic stirrer compound 16 (1.1 g, 5.3 mmol) and acetonitrile (10 mL) were added in sequence, and the mixture was stirred to form a solution. Compound 9 (0.8 g, 7.95 mmol) and potassium carbonate (1.46 g, 10.6 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to give 1.1 g of a yellow solid. Yield: 73.9%. LC-MS(APCI): m/z=283.2(M+1)⁺.

### Step 2 Synthesis of compound 22

To a 50 mL single-necked flask equipped with a magnetic stirrer and a condenser tube compound 21 (1.1 g, 3.93 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The solution was concentrated to remove the organic solvent, and extracted with dichloromethane (40 mLx3). The organic phases were combined, dried over anhydrous sodium sulfate, filtered, and concentrated to give 800 mg of a brown solid. Yield: 81.4%. LC-MS(APCI): m/z=253.3(M+1)⁺.

### Step 3 Synthesis of compound T-7

To a 25 mL single-necked flask equipped with a magnetic stirrer and a condenser tube compound 22 (150 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filtered, and concentrated. The residue was passed through a silica gel column to afford 160 mg of a white solid. Yield: 50.1%. LC-MS(APCI): m/z=532.2(M+1)⁺. ¹H NMR(400 MHz, DMSO-D₆) δ ppm: 11.04(s, 1H), 8.29(s, 1H), 7.93-7.91(m, 2H), 7.47(d, J=8.0 Hz, 1H), 7.42-7.37(m, 1H), 7.16(t, J=8.0 Hz, 1H), 6.96(t, J=8.0 Hz, 1H), 6.04(d, J=8.0 Hz, 1H), 5.09-5.03(m, 1H), 3.69(t, J=7.2 Hz, 2H), 2.95(t, J=7.2Hz, 2H), 2.56(s, 3H), 1.67(s, 3H), 1.61(s, 3H), 1.06(d, J=6.0 Hz, 6H).

### Example 8 Preparation of (2-((5-chloro-2-((2-isopropoxy-6-(4-(4-methylpiperazin-1-yl)piperidin-1-yl)pyridin-3-yl) amino)pyrimidin-4-yl)amino)phenyl)dimethyl phosphine oxide (compound T-8)

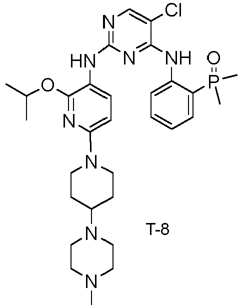

The following route was used for the synthesis:

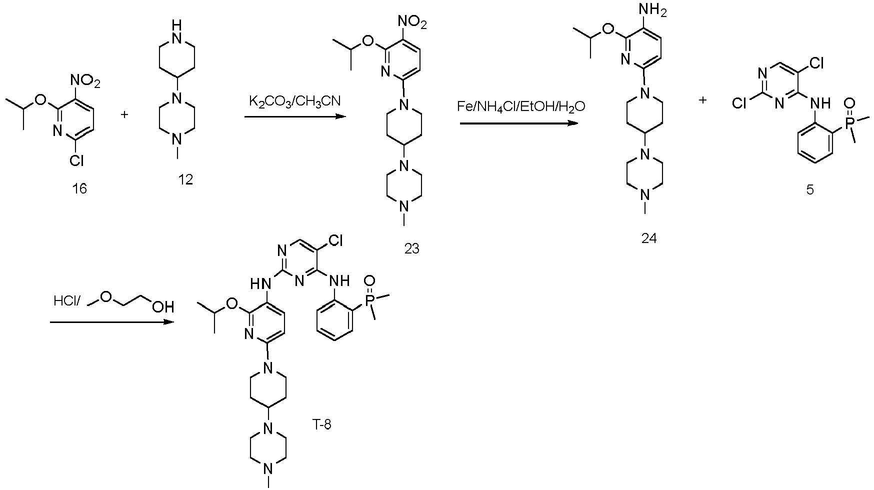

### Step 1 Synthesis of compound 23

To a 50 mL single-necked flask equipped with a magnetic stirrer compound 16 (1.1 g, 5.3 mmol) and acetonitrile (10 mL) were added in sequence, and the mixture was stirred to form a solution. Compound 12 (1.4 g, 7.95 mmol) and potassium carbonate (1.46 g, 10.6 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to afford 1.3 g of a yellow solid. Yield: 67.4%. LC-MS(APCI): m/z=364.2(M+1)⁺.

### Step 2 Synthesis of compound 24

To a 50 mL single-necked flask equipped with a magnetic stirrer and a condenser tube compound 23 (1.4 g, 3.93 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The solution was concentrated to remove the organic solvent, and extracted with dichloromethane (40 mLx3). The organic phases were combined, dried over anhydrous sodium sulfate, filtered, and concentrated to give 980 mg of a brown solid. Yield: 74.9%. LC-MS(APCI): m/z=334.3(M+1)⁺.

### Step 3 Synthesis of compound T-8

To a 25 mL single-necked flask equipped with a magnetic stirrer and a condenser tube compound 24 (200 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the solution was extracted with dichloromethane (15 mLx3). The organic phases were combined, washed with saturated brine, dried over anhydrous sodium sulfate, filtered, and concentrated. The residue was passed through a silica gel column to afford 160 mg of a white solid. Yield: 50.1%. LC-MS(APCI): m/z=613.2(M+1)⁺. ¹H NMR(400 MHz, DMSO-D₆) δ ppm: 11.17(s, 1H), 8.44(s, 1H), 8.06-8.03(m, 2H), 7.61-7.50(m, 2H), 7.29(t, J=8.0 Hz, 1H), 7.10(t, J=8.0 Hz, 1H), 6.33(d, J=8.4 Hz, 1H), 5.15-5.12(m, 1H), 4.25(d, J=11.2 Hz, 2H), 3.35-2.89(m, 7H), 2.83-2.77(m, 4H), 2.56(s, 3H), 1.91-1.88(m, 2H), 1.77(s, 3H), 1.74(s, 3H), 1.44-1.39(m, 2H), 1.20(d, J=6.0Hz, 6H).

### Example 9 Preparation of (2-((5-chloro-2-((6-(4-(dimethylamino)piperidin-1-yl)-2-(2,2,2-trifluoroethoxy)pyridin-3 -yl)amino)pyrimidin-4-yl)amino)phenyl)dimethylphosphine oxide (compound T-9)

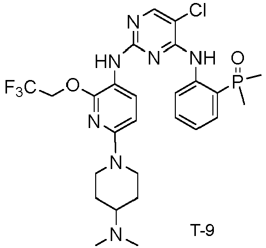

The following route was used for the synthesis:

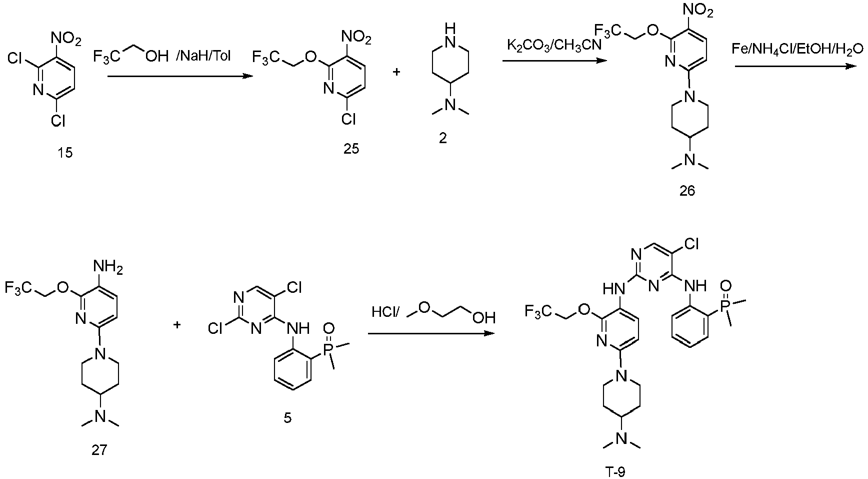

### Synthesis step 1 Synthesis of compound 25

To a 250 mL single-necked flask equipped with a magnetic stirrer compound 15 (5.79 g, 30 mmol) and dry toluene (50 mL) were added, and the mixture was stirred to form a solution. Trifluoroethanol (3.6 g, 36 mmol) was added, the solution was cooled to 0 °C before NaH (1.56 g, 39 mmol, 60%) was added, and the resulting solution was stirred for half an hour under a nitrogen atmosphere. The ice bath was then removed, and the reaction was stirred at room temperature overnight. The reaction was quenched by adding water (50 mL), the organic layer was separated, and the aqueous phase was extracted with ethyl acetate (40 mL × 2). The organic phases were combined, dried over anhydrous sodium sulfate, filtered, and the filtrate was concentrated. The residue was passed through a silica gel column to afford 3.41 g of a white solid. Yield: 44.2%. LC-MS(APCI): m/z=257.0(M+1)⁺. ¹H NMR(400 MHz, CDCl₃) δ ppm: 8.35(d, J=8.4 Hz, 1H), 7.18(d, J=8.4 Hz, 1H), 4.91(q, t=8.0 Hz, 2H).

### Step 2 Synthesis of compound 26

To a 50 mL single-necked flask equipped with a magnetic stirrer compound 25 (1.36 g, 5.3 mmol) and acetonitrile (10 mL) were added in sequence, and the mixture was stirred to form a solution. The hydrochloride salt of compound 2 (1.31 g, 7.95 mmol) and potassium carbonate (3.66 g, 26.5 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to give 1.5 g of a yellow solid. Yield: 81.3%. LC-MS(APCI): m/z=349.2(M+1)⁺.

### Step 3 Synthesis of compound 27

To a 50 mL single-necked flask equipped with a magnetic stirrer and a condenser tube compound 26 (1.24 g, 3.57 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The resulting solution was concentrated to remove the organic solvent, extracted with dichloromethane (40 mLx3), the organic phases were combined, dried over anhydrous sodium sulfate, filtered, and concentrated to get 900 mg of a brown solid. Yield: 79.0%. LC-MS(APCI): m/z=319.3(M+1)⁺.

### Step 4 Synthesis of compound T-9

To a 25 mL single-necked flask equipped with a magnetic stirrer and a condenser tube compound 27 (190 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the solution was extracted with dichloromethane (15 mLx3). The organic phases were combined, washed with saturated brine, dried over anhydrous sodium sulfate, filtered, and concentrated. The residue was passed through a silica gel column to afford 200 mg of a white solid. Yield: 55.8%. LC-MS(APCI): m/z=598.2(M+1)⁺. ¹H NMR(400MHz, DMSO-D₆) δ ppm: 11.19(s, 1H), 8.37(br s, 1H), 8.35(s, 1H), 8.04(s, 1H), 7.59(d, J=8.8Hz, 1H), 7.54-7.49(m, 1H), 7.27-7.24(m, 1H), 7.08(t, J=8.0Hz, 1H), 6.53(d, J=8.4Hz, 1H), 4.83(q, J=9.2Hz, 2H), 4.40(d, J=12.8Hz, 2H), 3.35-3.32(m, 1H), 2.82(t, J=9.0Hz, 2H), 2.69(s, 6H), 2.09(d, J=10.8Hz, 2H), 1.77(s, 3H), 1.74(s, 3H). 1.71-1.55(m, 2H).

### Example 10 Preparation of (2-((5-chloro-2-((6-(4-methylpiperazin-1-yl)-2-(2,2,2-trifluoroethoxy)pyridin-3-yl)amino )pyrimidin-4-yl)amino)phenyl)dimethylphosphine oxide (compound T-10)

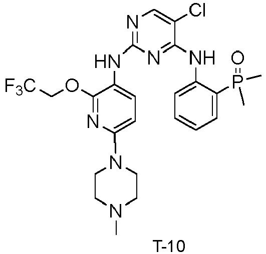

The following route was used for the synthesis:

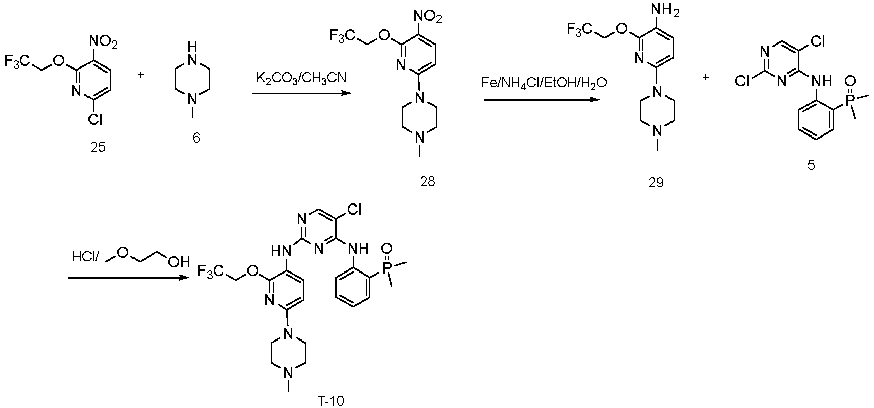

### Step 1 Synthesis of compound 28

To a 50 mL single-necked flask equipped with a magnetic stirrer compound 25 (1.36 g, 5.3 mmol) and acetonitrile (10 mL) were added in sequence, and the mixture was stirred to form a solution. Compound 6 (0.8 g, 7.95 mmol) and potassium carbonate (3.66 g, 26.5 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to give 1.5 g of a yellow solid. Yield: 88.4%. LC-MS(APCI): m/z=321.1(M+1)⁺.

### Step 2 Synthesis of compound 29

To a 50 mL single-necked flask equipped with a magnetic stirrer and a condenser tube compound 28 (1.14 g, 3.57 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the mixture was heated to reflux under a nitrogen atmosphere and reacted at this temperature for 1 hour. The reaction was cooled to room temperature, filtered, and the filter cake was washed with ethanol (5 mL). The resulting solution was concentrated to remove the organic solvent, and extracted with dichloromethane (40 mL x 3). The organic phases were combined, dried over anhydrous sodium sulfate, filtered, and concentrated to give 850 mg of a brown solid. Yield: 82.1%. LC-MS(APCI): m/z=291.1(M+1)⁺.

### Step 3 Synthesis of compound T-10

To a 25 mL single-necked flask equipped with a magnetic stirrer and a condenser tube compound 29 (174 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filtered, and concentrated. The residue was passed through a silica gel column to afford 200 mg of a white solid. Yield: 58.6%. LC-MS(APCI): m/z=570.2(M+1)⁺. ¹H NMR(400MHz, DMSO-D₆) δ ppm: 11.19(s, 1H), 8.39-8.35(m, 2H), 8.05(s, 1H), 7.59 (d, J=8.0 Hz, 1H), 7.54-7.49(m, 1H), 7.26-7.23(m, 1H), 7.07(t, J=8.0 Hz, 1H), 6.49(d, J=8.0 Hz, 1H), 4.83(q, J=9.2 Hz, 2H), 3.55-3.52(m, 4H), 2.65-2.62(m, 4H), 2.38(s, 3H), 1.77(s, 3H), 1.74(s, 3H).

### Example 11 Preparation of (2-((5-chloro-2-((6-((2-(dimethylamino)ethyl)(methyl)amino)-2-(2,2,2-trifluoroethoxy)v yridin-3-yl)amino)pyrimidin-4-yl)amino)phenyl)dimethylphosphine oxide (compound T-11)

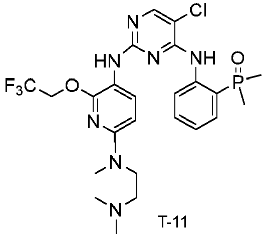

The following route was used for the synthesis:

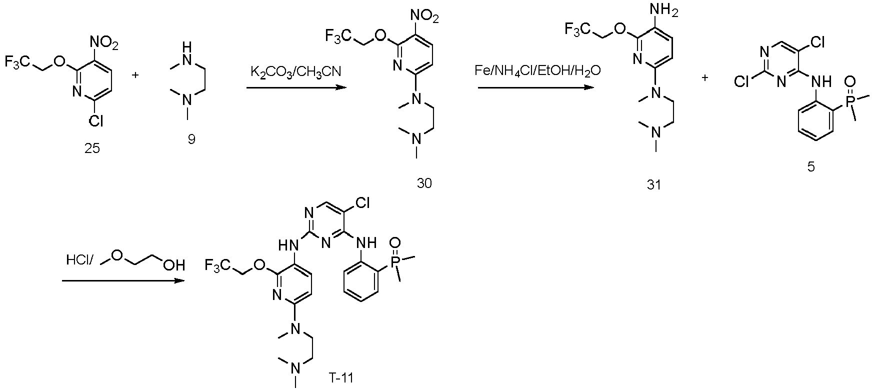

### Step 1 Synthesis of compound 30

To a 50 mL single-necked flask equipped with a magnetic stirrer compound 25 (1.36 g, 5.3 mmol) and acetonitrile (10 mL) were added in sequence, and the mixture was stirred to form a solution. Compound 9 (0.8 g, 7.95 mmol) and potassium carbonate (3.66 g, 26.5 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to give 1.5 g of a yellow solid. Yield: 88.4%. LC-MS(APCI): m/z=321.1(M+1)⁺.

### Step 2 Synthesis of compound 31

To a 50 mL single-necked flask equipped with a magnetic stirrer and a condenser tube compound 30 (1.14 g, 3.57 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the mixture was heated to reflux under a nitrogen atmosphere and reacted at this temperature for 1 hour. The reaction was cooled to room temperature, filtered, and the filter cake was washed with ethanol (5 mL). The resulting solution was concentrated to remove the organic solvent, and extracted with dichloromethane (40 mL × 3). Te organic phases were combined, dried over anhydrous sodium sulfate, filtered, and concentrated to give 850 mg of a brown solid. Yield: 82.1%. LC-MS(APCI): m/z=291.1(M+1)⁺.

### Step 3 Synthesis of compound T-11

To a 25 mL single-necked flask equipped with a magnetic stirrer and a condenser tube compound 31 (174 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filter, and concentrated. The residue was passed through a silica gel column to afford 200 mg of a white solid. Yield: 58.6%. LC-MS(APCI): m/z=572.2(M+1)⁺. ¹H NMR(400MHz, DMSO-D₆) δ ppm: 11.17(s, 1H), 8.40(br s, 1H), 8.33(s, 1H), 8.03(s, 1H), 7.56-7.48(m, 2H), 7.24(br s, 1H), 7.07(t, J=8.0 Hz, 1H), 6.29(d, J=8.4 Hz, 1H), 4.87(q, J=9.2 Hz, 2H), 3.83-3.78(m, 2H), 3.02(s, 3H), 3.00-2.96(m, 2H), 2.59(s, 6H), 1.77(s, 3H), 1.74(s, 3H).

### Example 12 Preparation of (2-((5-chloro-2-((6-(4-(4-methylpiperazin-1-yl)piperidin-1-yl)-2-(2,2,2-trifluoroethoxy)p yridin-3-yl)amino)pyrimidin-4-yl)amino)phenyl)dimethylphosphine oxide (compound T-12)

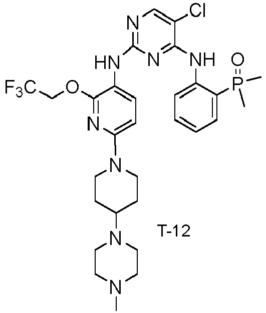

The following route was used for the synthesis:

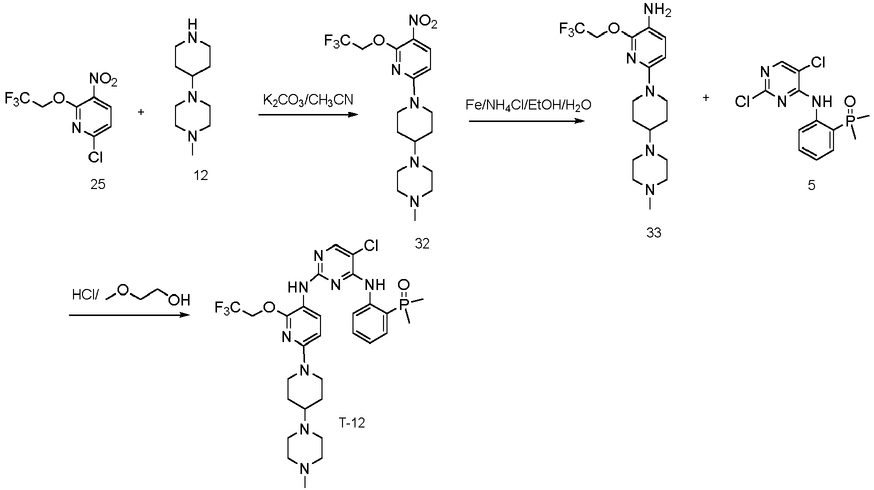

### Step 1 Synthesis of compound 32

To a 50 mL single-necked flask equipped with a magnetic stirrer compound 25 (1.36 g, 5.3 mmol) and acetonitrile (10 mL) were added in sequence, and the mixture was stirred to form a solution. Compound 12 (1.46 g, 7.95 mmol) and potassium carbonate (3.66 g, 26.5 mmol) were added, and the reaction mixture was heated to 80 °C under a nitrogen atmosphere and the reaction was stirred at this temperature overnight. The reaction was cooled to room temperature, diluted with ethyl acetate (50 mL), the inorganic salt was filtered off, and the filter cake was washed with ethyl acetate (10 mL). The filtrate was combined, concentrated, and the residue was passed through a silica gel column to afford 1.6 g of a yellow solid. Yield: 74.9%. LC-MS(APCI): m/z=404.1(M+1)⁺.

### Step 2 Synthesis of compound 33

To a 50 mL single-necked flask equipped with a magnetic stirrer and a condenser tube compound 32 (1.44 g, 3.57 mmol) and ethanol/water (30 mL, 3/1) were added, and the mixture was stirred to form a solution. Reduced iron powder (23.2 mmol, 1.29 g) and ammonium chloride (23.2 mmol, 1.24 g) were added, and the reaction was heated to reflux under a nitrogen atmosphere and reacted at this temperature for 1 hour. The reaction was cooled to room temperature, filtered, and the filter cake was washed with ethanol (5 mL). The solution was concentrated to remove the organic solvent, and extracted with dichloromethane (40 mLx3). The organic phases were combined, dried over anhydrous sodium sulfate, filtered, and concentrated to give 1.1 g of a brown solid. Yield: 82.4%. LC-MS(APCI): m/z=374.1(M+1)⁺.

### Step 3 Synthesis of compound T-12

To a 25 mL single-necked flask equipped with a magnetic stirrer and a condenser tube compound 33 (224 mg, 0.6 mmol), compound 5 (189 mg, 0.6 mmol) and ethylene glycol monomethyl ether (3 mL) were added, and the mixture was stirred to form a solution. A solution of hydrogen chloride in isopropanol (0.9 mmol, 0.18 mL, 5M) was added dropwise, and the temperature of the reaction was raised to 120 °C under a nitrogen atmosphere and the reaction was stirred at this temperature overnight. The reaction was cooled to room temperature, water (10 mL) and saturated sodium bicarbonate (5 mL) were added, and the resulting solution was extracted with dichloromethane (15 mLx3). The organic phases were combined, washed with saturated brine, dried over anhydrous sodium sulfate, filtered, and concentrated. The residue was passed through a silica gel column to afford 220 mg of white solid. Yield: 56.2%. LC-MS(APCI): m/z=653.2(M+1)⁺. ¹H NMR(400 MHz, DMSO-D₆) δ ppm: 11.19(s, 1H), 8.35(br s, 1H), 8.34(s, 1H), 8.04(s, 1H), 7.57-7.49(m, 1H), 7.27-7.24(m, 1H), 7.08(t, J=8.0 Hz, 1H), 6.48(d, J=8.4 Hz, 1H), 4.83(q, J=9.2 Hz, 2H), 4.40(d, J=12.8 Hz, 2H), 4.28(d, J=12.0 Hz, 2H), 3.35-3.33(m, 2H), 3.01-2.54(m, 9H), 2.49(s, 3H), 1.89-1.83(m, 2H), 1.79(s, 3H), 1.74(s, 3H). 1.44-1.41(m, 2H).

### Example 13 Preparation of (2-((2-((6-(4-(dimethylamino)piperidin-1-yl)-2-methoxypyridin-3-yl)amino)-7H-pyrrolo [2,3-d]pyrimidin-4-yl)amino)phenyl)dimethylphosphine oxide (compound T-13)

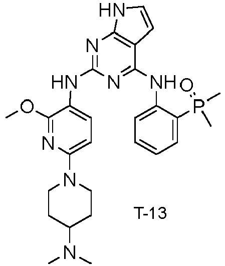

The following route was used for the synthesis:

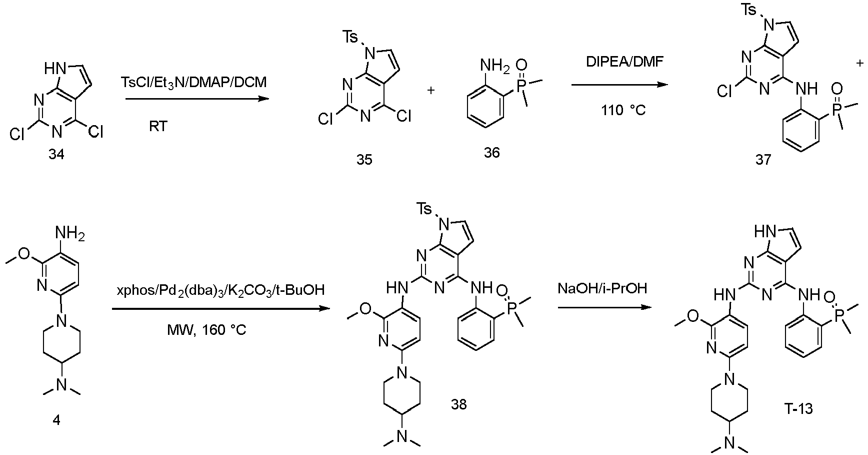

### Step 1 Synthesis of compound 35

To a 250 mL single-necked flask equipped with a magnetic stirrer compound 34 (3.0 g, 16.11 mmol) and dichloromethane (80 mL) were added, and the mixture was stirred to form a solution. P-toluenesulfonyl chloride (TsCl, 3.18 g, 16.92 mmol), triethylamine (3.24 g, 32.22 mmol) and 4-dimethylaminopyridine (DMAP, 60 mg, 0.48 mmol) were added in sequence. After addition, the reaction mixture was stirred at room temperature overnight under a liquid nitrogen atmosphere. The reaction was quenched by adding water (50 mL), the organic layer was separated, and the aqueous layer was extracted with dichloromethane (40 mL × 2). The organic phases were combined, dried over anhydrous sodium sulfate, filtered, and the filtrate was concentrated. The residue was passed through a silica gel column to give 4.8 g of a white solid. Yield: 89.1%. LC-MS(APCI): m/z=342.1(M+1)⁺. ¹H NMR(300 MHz, CDCl₃) δ (ppm): 8.12 (d, J = 5.1 Hz, 1H), 8.03 (d, J= 8.4 Hz, 2H), 7.50 (d, J = 8.4 Hz, 2H), 6.98 (d, J = 4.2 Hz, 1H), 2.38 (s, 3H)

### Step 2 Synthesis of compound 37

To a 100 mL single-necked flask equipped with a magnetic stirrer compound 35 (3.4 g, 10 mmol) and DMF (20 mL) were added, and the mixture was stirred to form a solution. Compound 36 (1.7 g, 10 mmol) and N, N-diisopropylethylamine (DIPEA, 1.3 g, 10 mmol) were added, and the temperature of the reaction was raised to 110 °C under a nitrogen atmosphere, and the reaction was stirred at this temperature overnight. The reaction was cooled to room temperature, water (60 mL) was added, and the resulting solution was extracted with ethyl acetate (50 mLx3). The organic phases were combined, washed with water (100 mLx2), dried over anhydrous sodium sulfate, filtered, and concentrated, and the residue was passed through a silica gel column to afford 3.0 g of a white solid. Yield: 63.2%. LC-MS(APCI): m/z=475.2(M+1)⁺.

### Step 3 Synthesis of compound 38

To a 20 mL microwave tube equipped with a magnetic stirrer compound 37 (304 mg, 0.64 mmol) and anhydrous tert-butanol (10 mL) were added, and the mixture was stirred to form a solution. Compound 4 (200 mg, 0.8 mmol) and anhydrous potassium carbonate (166 mg, 1.2 mmol) were added, bubbled with nitrogen for 3 minutes, and 2-dicyclohexylphosphono-2,4,6-triisopropylbiphenyl (xphos, 30 mg) and tris (dibenzylideneacetone) dipalladium (Pd₂(dba)₃, 30 mg) were added. The microwave tube was sealed under a nitrogen atmosphere, placed in a microwave reactor and the temperature was raised to 160 °C. The reaction was stirred at this temperature for 1 hour. The reaction was cooled to room temperature, ethyl acetate (30 mL) was added for dilution, the insoluble solid was filtered off, and the filtrate was concentrated. The residue was passed through a silica gel column to afford 307 mg of a white solid. Yield: 55.8%. LC-MS(APCI): m/z=689.2(M+1)⁺.

### Step 4 Synthesis of compound T-13

To a 50 mL single-necked flask equipped with a magnetic stirrer compound 38 (300 mg, 0.44 mmol) and isopropanol were added, and the mixture was stirred to form a solution. An aqueous solution of sodium hydroxide (0.87 mL, 1.76 mmol, 2M) was added, and the reaction mixture was heated to 60 °C under a nitrogen atmosphere and the reaction was stirred at this temperature for 3 hours. The reaction was cooled to room temperature, water (20 mL) was added, and extracted with dichloromethane (30 mL × 3). The organic phases were combined, dried over anhydrous sodium sulfate, filtered, and concentrated, and the residue was passed through a silica gel column to afford 140 mg of a white solid. Yield: 60.1%. LC-MS(APCI): m/z=535.2(M+1)⁺. ¹H NMR(300 MHz, DMSO-d₆) δ (ppm): 11.59(s, 1H), 11.21(s, 1H), 8.94(dd, J=6.0 Hz, J=3.3 Hz, 1H), 8.02(d, J=6.6 Hz, 1H), 7.56(dd, J=10.2 Hz, J=5.7 Hz, 1H), 7.47-7.42(m, 2H), 7.06(t, J=6.0 Hz, 1H), 6.90(s, 1H), 4.39(d, J=10.2 Hz, 2H), 3.87(s, 3H), 3.23(s, 1H), 2.81(t, J=6.0 Hz, 2H), 2.67(s, 6H), 2.09(d, J=5.7 Hz, 2H), 1.84(d, J=10.5 Hz, 6H), 1.68-1.59(m, 2H).

### Example 14 Preparation of (2-((2-((2-methoxy-6-(4-methylpiperazin-1-yl)pyridin-3-yl)amino)-7H-pyrrolo[2,3-d]pyr imidin-4-yl)amino)phenyl)dimethylphosphine oxide (compound T-14)

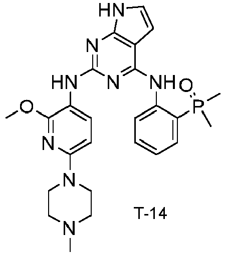

The following route was used for the synthesis:

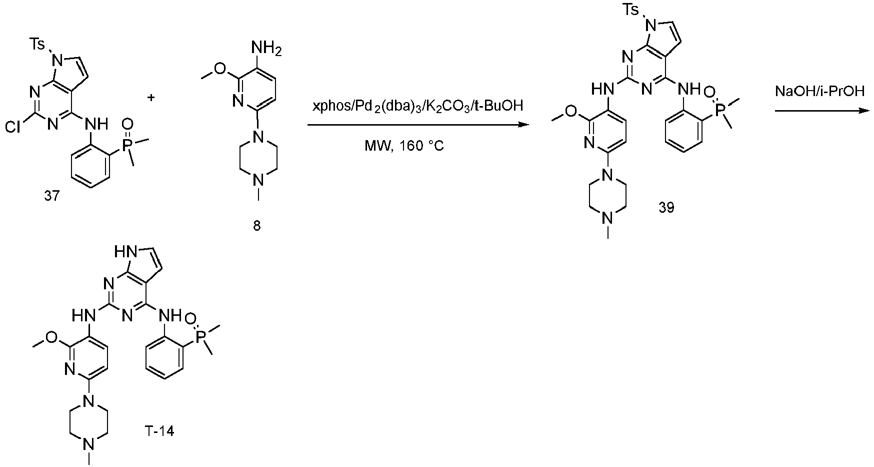

### Step 1 Synthesis of compound 39

To a 20 mL microwave tube equipped with a magnetic stirrer compound 37 (304 mg, 0.64 mmol) and anhydrous tert-butanol (10 mL) were added, and the mixture was stirred to form a solution. Compound 8 (177 mg, 0.8 mmol) and anhydrous potassium carbonate (166 mg, 1.2 mmol) were added, bubbled with nitrogen for 3 minutes, and xphos(30 mg) and Pd₂(dba)₃(30 mg) were added. The microwave tube was sealed under a nitrogen atmosphere, placed in a microwave reactor and the temperature was raised to 160 °C. The reaction was stirred at this temperature for 1 hour. The reaction was cooled to room temperature, diluted with ethyl acetate (30 mL), and the insoluble solid was filtered off. The filtrate was concentrated, and the residue was passed through a silica gel column to afford 300 mg of a white solid. Yield: 71.0%. LC-MS(APCI): m/z=661.2(M+1)⁺.

### Step 2 Synthesis of compound T-14

To a 50 mL single-necked flask equipped with a magnetic stirrer compound 39 (290 mg, 0.44 mmol) and isopropanol, and the mixture was stirred to form a solution. An aqueous solution of sodium hydroxide (0.87 mL, 1.76 mmol, 2M) was added, and the reaction mixture was heated to 60 °C under a nitrogen atmosphere and the reaction was stirred at this temperature for 3 hours. The reaction was cooled to room temperature, water (20 mL) was added, and extracted with dichloromethane (30 mL × 3). The organic phases were combined, dried over anhydrous sodium sulfate, filtered, and concentrated, and the residue was passed through a silica gel column to afford 140 mg of a white solid. Yield: 62.7%. LC-MS(APCI): m/z=507.2(M+1)⁺. ¹H NMR(300 MHz, CDCl3) δ (ppm): 11.05(s, 1H), 9.02(br s, 1H), 8.89(dd, J=6.6 Hz, J=3.3 Hz, 1H), 8.41(d, J=6.6 Hz, 1H), 7.47(d, J=6.0 Hz, 1H), 7.23-7.18(m, 1H), 7.01(t, J=5.7 Hz, 1H), 6.87(s, 1H), 6.72(d, J=2.7 Hz, 1H), 6.53(d, J=2.7 Hz, 1H), 6.17(d, J=6.6 Hz, 1H), 3.94(s, 3H), 3.56(t, J=3.6 Hz, 4H), 2.70(t, J=3.6 Hz, 4H), 2.44(s, 3H), 2.65(s, 6H), 1.81(d, J=9.6 Hz, 6H).

### Example 15 Preparation of (2-((2-((6-((2-(dimethylamino)ethyl)(methyl)amino)-2-methoxypyridin-3-yl)amino)-7H-pyrrolo[2,3-d]pyrimidin-4-yl)amino)phenyl)dimethylphosphine oxide (compound T-15)

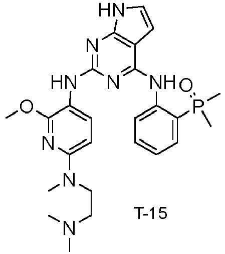

The following route was used for the synthesis:

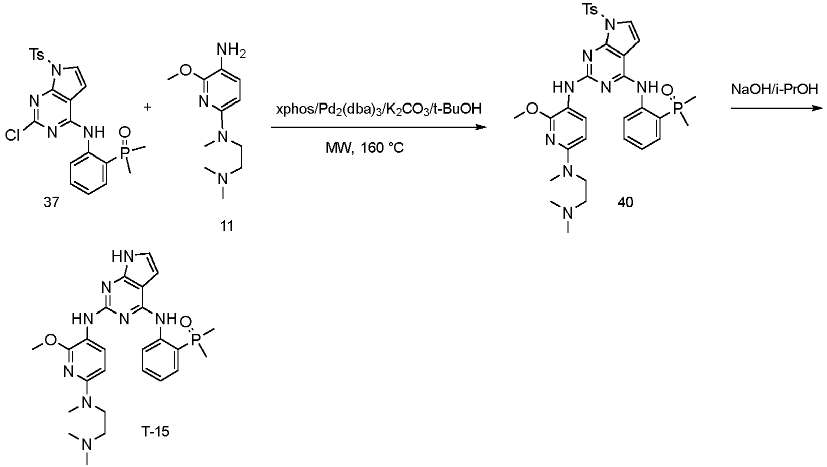

### Step 1 Synthesis of compound 40

To a 20 mL microwave tube equipped with a magnetic stirrer compound 37 (304 mg, 0.64 mmol) and anhydrous tert-butanol (10 mL) were added, and the mixture was stirred to form a solution. Compound 11 (177 mg, 0.8 mmol) and anhydrous potassium carbonate (166 mg, 1.2 mmol) were added, bubbled with nitrogen for 3 minutes, and xphos(30 mg) and Pd₂(dba)₃(30 mg) were added. The microwave tube was sealed under a nitrogen atmosphere, placed in a microwave reactor and the temperature was raised to 160 °C. The reaction was stirred at this temperature for 1 hour. The reaction was cooled to room temperature, diluted with ethyl acetate (30 mL), and the insoluble solid was filtered off. The filtrate was concentrated, and the residue was passed through a silica gel column to afford 300 mg of a white solid. Yield: 71.0%. LC-MS(APCI): m/z=663.1(M+1)⁺.

### Step 2 Synthesis of compound T-15

To a 50 mL single-necked flask equipped with a magnetic stirrer compound 40 (290 mg, 0.44 mmol) and isopropanol were added, and the mixture was stirred to form a solution. An aqueous solution of sodium hydroxide (0.87 mL, 1.76 mmol, 2M) was added, and the reaction mixture was heated to 60 °C under a nitrogen atmosphere and the reaction was stirred at this temperature for 3 hours. The reaction was cooled to room temperature, water (20 mL) was added, and the resulting solution was extracted with dichloromethane (30 mL × 3). The organic phases were combined, dried over anhydrous sodium sulfate, filtered, and concentrated, and the residue was passed through a silica gel column to afford 140 mg of a white solid. Yield: 62.7%. LC-MS(APCI): m/z=509.2(M+1)⁺. ¹H NMR(300 MHz, CDCl₃) δ (ppm): 11.07(s, 1H), 9.06(br s, 1H), 8.88(dd, J=6.3 Hz, J=3.0 Hz, 1H), 8.29(d, J=6.0 Hz, 1H), 7.44(d, J=6.0 Hz, 1H), 7.21-7.15(m, 1H), 6.98(t, J=5.7 Hz, 1H), 6.73(s, 1H), 6.66(d, J=2.7 Hz, 1H), 6.49(d, J=2.7 Hz, 1H), 6.02(d, J=6.6 Hz, 1H), 3.93(t, J=5.4 Hz, 2H), 3.89(s, 3H), 3.01(t, J=5.4 Hz, 2H), 2.98(s, 3H), 2.65(s, 6H), 1.79(d, J=10.2 Hz, 6H).

### Example 16 Preparation of (2-((2-((2-methoxy-6-(4-(4-methylpiperazin-1-yl)piperidin-1-yl)pyridin-3-yl)amino)-7H-pyrrolo[2,3-d]pyrimidin-4-yl)amino)phenyl)dimethylphosphine oxide (compound T-16)

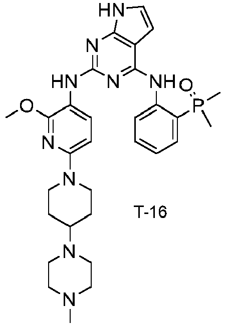

The following route was used for the synthesis:

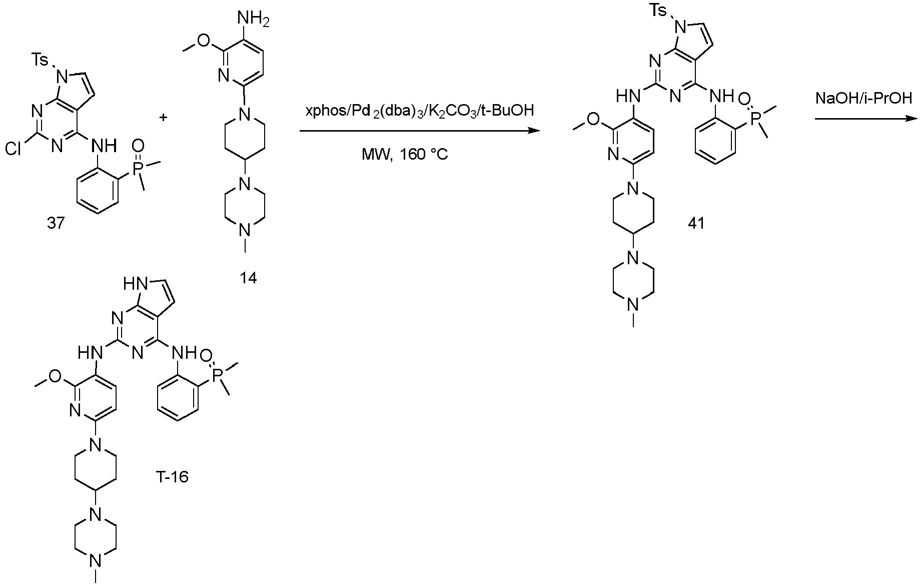

### Step 1 Synthesis of compound 41

To a 20 mL microwave tube equipped with a magnetic stirrer compound 37 (304 mg, 0.64 mmol) and anhydrous tert-butanol (10 mL) were added, and the mixture was stirred to form a solution. Compound 14 (244 mg, 0.8 mmol) and anhydrous potassium carbonate (166 mg, 1.2 mmol) were added, bubbled with nitrogen for 3 minutes, and xphos(30 mg) and Pd₂(dba)₃(30 mg) were added. The microwave tube was sealed under a nitrogen atmosphere, placed in a microwave reactor and the temperature was raised to 160 °C. The reaction was stirred at this temperature for 1 hour. The reaction was cooled to room temperature, diluted with ethyl acetate (30 mL), and the insoluble solid was filtered off. The filtrate was concentrated, and the residue was passed through a silica gel column to afford 350 mg of a white solid. Yield: 73.6%. LC-MS(APCI): m/z=744.3(M+1)⁺.

### Step 2 Synthesis of compound T-16

To a 50 mL single-necked flask equipped with a magnetic stirrer compound 41 (327 mg, 0.44 mmol) and isopropanol were added, and the mixture was stirred to form a solution. An aqueous solution of sodium hydroxide (0.87 mL, 1.76 mmol, 2M) was added, and the reaction mixture was heated to 60 °C under a nitrogen atmosphere and the reaction was stirred at this temperature for 3 hours. The reaction was cooled to room temperature, water (20 mL) was added, and the resulting solution was extracted dichloromethane (30 mL × 3). The organic phases were combined, dried over anhydrous sodium sulfate, filtered, and concentrated, and the residue was passed through a silica gel column to afford 150 mg of a white solid. Yield: 57.8%. LC-MS(APCI): m/z=590.2(M+1)⁺. ¹H NMR(300 MHz, CDCl₃) δ (ppm): 11.13(s, 1H), 9.24(br s, 1H), 8.89(dd, J=6.3 Hz, J=3.0 Hz, 1H), 8.36(d, J=6.6 Hz, 1H), 7.48(d, J=6.0 Hz, 1H), 7.26-7.21(m, 1H), 7.05(t, J=4.8 Hz, 1H), 6.98(s, 1H), 6.76(d, J=2.7 Hz, 1H), 6.54(d, J=2.1 Hz, 1H), 6.19(d, J=6.6 Hz, 1H), 4.26(d, J=10.5 Hz, 2H), 3.94(s, 3H), 3.04(s, 8H), 2.74(t, J=9.3 Hz, 3H), 2.61(s, 3H), 2.00(d, J=7.5 Hz, 2H), 1.84(d, J=9.3 Hz, 6H), 1.64(d, J=7.2 Hz, 2H).

### Example 17 Preparation of (2-((2-((6-(2-aminoethyl)-2-(2,2,2-trifluoroethoxy)pyridin-3-yl)amino)-5-chloropyrimidi n-4-yl)amino)phenyl)dimethylphosphine oxide (compound T-17)

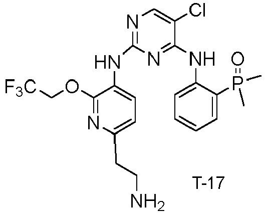

The following route was used for the synthesis:

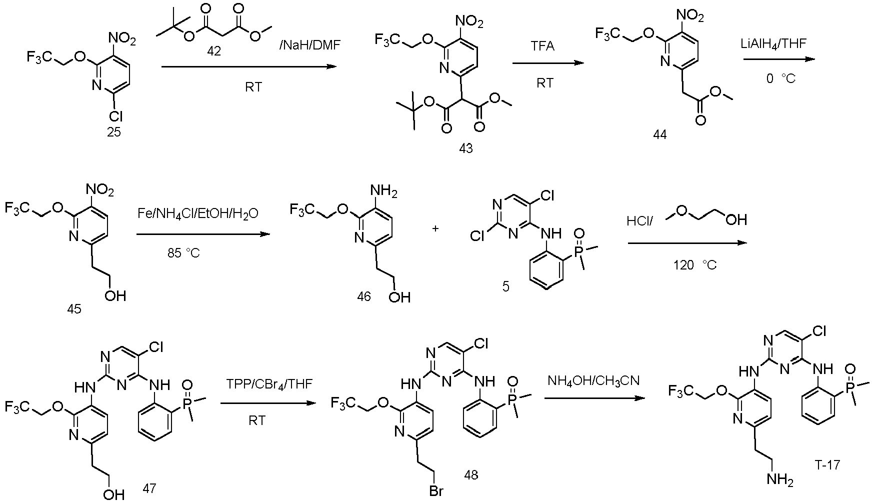

### Step 1 Synthesis of compound 43

To a 250 mL single-necked flask equipped with a magnetic stirrer compound 25 (2.43 g, 9.48 mmol) and anhydrous DMSO (30 mL) were added in sequence, and the mixture was stirred to form a solution under a nitrogen atmosphere. After the reaction was cooled to 0 °C, NaH (517 mg, 12.93 mmol, 60%) was added slowly, and the reaction was stirred at 0 °C for half an hour. A solution of compound 42 (1.5 g, 8.62 mmol) in DMSO (10 mL) was slowly added dropwise. After the addition, the ice bath was removed, and the reaction mixture was stirred at room temperature overnight. Water (50 mL) was added, and the resulting solution was extracted with ethyl acetate (50 mL × 3). The organic phases were combined, washed with water (60 mL × 2), dried over anhydrous sodium sulfate, filtered, concentrated and passed through a silica gel column to give 2.5 g of a white solid. Yield: 66.9%. LC-MS(APCI): m/z=395.1(M+1)⁺.

### Step 2 Synthesis of compound 44

To a 250 mL single-necked flask equipped with a magnetic stirrer compound 43 (2.5 g, 6.34 mmol) and TFA were added in sequence, and the reaction was stirred at room temperature for 10 minutes under a nitrogen atmosphere. Unreacted TFA was distilled off under reduced pressure. A saturated aqueous solution of sodium bicarbonate (30 mL) was added, and the resulting solution was extracted with ethyl acetate (40 mL × 3). The organic phases were combined, washed with water (60 mL × 2), dried over anhydrous sodium sulfate, filtered, concentrated and passed through a silica gel column to afford 1.77 g of a white solid. Yield: 95.0%. LC-MS(APCI): m/z=295.1(M+1)⁺.

### Step 3 Synthesis of compound 45

To a 250 mL single-necked flask equipped with a magnetic stirrer compound 44 (1.77 g, 6.0 mmol) and anhydrous THF (40 mL) were added in sequence, and the mixture was stirred to form a solution. After the reaction was cooled in an ice water bath, LiAlH₄ (228 mg, 8.4 mmol) was slowly added, and the reaction was stirred under a nitrogen atmosphere for 1 hour. The reaction was quenched by adding sodium sulfate decahydrate (20 g), stirred for 5 minutes, diluted with dichloromethane (60 mL), filtered, and the filter cake was washed with dichloromethane (10 mL). The resulting solution was dried over anhydrous sodium sulfate, filtered, and concentrated to obtain 1.24 g of a white solid. Yield: 77.7%. LC-MS(APCI): m/z=267.1(M+1)⁺.

### Step 4 Synthesis of compound 46

To a 100 mL single-necked flask equipped with a magnetic stirrer and a condenser tube compound 45 (1.24 g, 4.66 mmol) and ethanol/water (30 mL, 3/1) were added, and the mixture was stirred to form a solution. Reduced iron powder (28 mmol, 1.57 g) and ammonium chloride (4.66 mmol, 251 mg) were added, and the reaction was heated to reflux under a nitrogen atmosphere and reacted at this temperature for 1 hour. The reaction was cooled to room temperature, filtered, and the filter cake was washed with ethanol (10 mL). The resulting solution was concentrated to remove the organic solvent, and extracted with dichloromethane (30 mLx3). The organic phases were combined, dried over anhydrous sodium sulfate, filtered, and concentrated to give 800 mg of a brown solid. Yield: 72.4%. LC-MS(APCI): m/z=237.1(M+1)⁺.

### Step 5 Synthesis of compound 47

To a 50 mL single-necked flask equipped with a magnetic stirrer and a condenser tube compound 46 (800 mg, 3.39 mmol), compound 5 (1.01 g, 3.39 mmol) and ethylene glycol monomethyl ether (10 mL) were added, and the mixture was stirred to form a solution. A solution of hydrogen chloride in isopropanol (5.0 mmol, 1.0 mL, 5M) was added dropwise, and the temperature of this temperature was raised to 120 °C under a nitrogen atmosphere and the reaction was stirred at this temperature overnight. The reaction was cooled to room temperature, water (30 mL) and saturated sodium bicarbonate (20 mL) were added, and the resulting solution was extracted with dichloromethane (50 mL × 3). The organic phases were combined, washed with saturated brine, dried over anhydrous sodium sulfate, filtered, and concentrated. The residue was passed through a silica gel column to afford 1.1 g of a white solid. Yield: 62.9%. LC-MS(APCI): m/z=516.2(M+1)⁺.

### Step 6 Synthesis of compound 48

To a 100 mL single-necked flask equipped with a magnetic stirrer and a condenser tube compound 47 (1.1 g, 2.13 mmol) and anhydrous dichloromethane (20 mL) were added, and the mixture was stirred to form a solution. CBr₄(3.2 mmol, 1.04 g) was added to the solution, which was cooled to 0 °C under a nitrogen atmosphere. Triphenylphosphine (0.84 g, 3.2 mmol) was added, the ice bath was removed after addition, and the reaction was stirred at room temperature for 1 h. The solvent was distilled off under reduced pressure, and the residue was passed through a silica gel column to afford 900 mg of a white solid. Yield: 73.1 %. LC-MS(APCI): m/z=578.1, 580.1(M+1)⁺.

### Step 7 Synthesis of compound T-17

To a 50 mL single-necked flask equipped with a magnetic stirrer compound 48 (115 mg, 0.2 mmol) and acetonitrile (2 mL) were added, and the mixture was stirred to form a solution. Ammonia (3 mL) was added, and the reaction was stirred at room temperature overnight under a nitrogen atmosphere. The solvent was distilled off under reduced pressure, and the residue was passed through a silica gel column to afford 60 mg of a white solid. Yield: 58.2 %. LC-MS(APCI): m/z=515.1(M+1)⁺. ¹H NMR(400 MHz, DMSO-D₆) δ ppm: 11.19(s, 1H), 8.49-8.46(m, 1H), 8.10(s, 1H), 7.96(br s, 2H), 7.77(d, J=8.0Hz, 1H), 7.61-7.55(m, 1H), 7.45(t, J=8.0Hz, 1H), 7.16(t, J=8.0Hz, 1H), 6.97(s, 1H), 6.78(d, J=8.0Hz, 1H), 3.35-3.32(m, 2H), 3.06(t, J=8.0Hz, 2H), 2.88(t, J=8.0Hz, 2H), 1.80(s, 3H), 1.76(s, 3H).

### Example 18 Preparation of (2-((5-chloro-2-((6-(2-(methylamino)ethyl)-2-(2,2,2-trifluoroethoxy)pyridin-3-yl)amino) pyrimidin-4-yl)amino)phenyl)dimethylphosphine oxide (compound T-18)

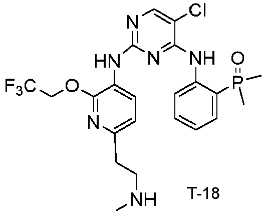

The following route was used for the synthesis:

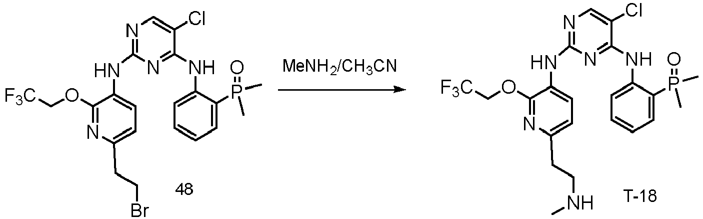

To a 50 mL single-necked flask equipped with a magnetic stirrer compound 48 (115 mg, 0.2 mmol) and acetonitrile (2 mL) were added, and the mixture was stirred to form a solution. A solution of methylamine in tetrahydrofuran (3 mL, 2M) was added, and the reaction was stirred at room temperature overnight under a nitrogen atmosphere. The solvent was distilled off under reduced pressure, and the residue was passed through a silica gel column to afford 60 mg of a white solid. Yield: 56.8 %. LC-MS(APCI): m/z=529.1(M+1)⁺. ¹H NMR(400 MHz, DMSO-D₆) δ ppm: 11.19(s, 1H), 8.49-8.46(m, 1H), 8.10(s, 1H), 7.96(br s, 2H), 7.77(d, J=8.0Hz, 1H), 7.61-7.55(m, 1H), 7.45(t, J=8.0Hz, 1H), 7.16(t, J=8.0Hz, 1H), 6.97(s, 1H), 6.78(d, J=8.0Hz, 1H), 3.35-3.32(m, 2H), 3.06(t, J=8.0Hz, 2H), 2.88(t, J=8.0Hz, 2H), 2.67(s, 3H), 1.80(s, 3H), 1.76(s, 3H).

### Example 19 Preparation of (2-((5-chloro-2-((6-(2-(dimethylamino)ethyl)-2-(2,2,2-trifluoroethoxy)pyridin-3-yl)amin o)pyrimidin-4-yl)amino)phenyl)dimethylphosphine oxide (compound T-19)

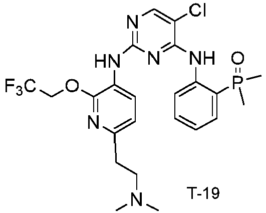

The following route was used for the synthesis:

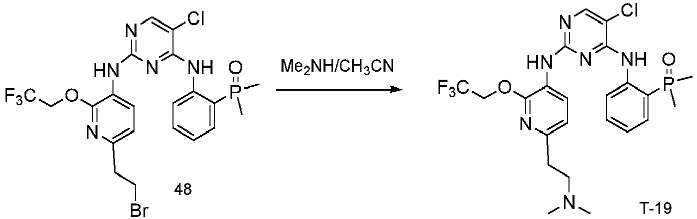

To a 50 mL single-necked flask equipped with a magnetic stirrer compound 48 (115 mg, 0.2 mmol) and acetonitrile (2 mL) were added, and the mixture was stirred to form a solution. A solution of dimethylamine in tetrahydrofuran (3 mL, 2M) was added, and the reaction was stirred at room temperature overnight under a nitrogen atmosphere. The solvent was distilled off under reduced pressure, and the residue was passed through a silica gel column to afford 60 mg of a white solid. Yield: 55.1 %. LC-MS(APCI): m/z=543.1(M+1)⁺. ¹H NMR(400 MHz, DMSO-D₆) δ ppm: 11.19(s, 1H), 8.49-8.46(m, 1H), 8.10(s, 1H), 7.96(br s, 2H), 7.77(d, J=8.0Hz, 1H), 7.61-7.55(m, 1H), 7.45(t, J=8.0Hz, 1H), 7. 16(t, J=8.0Hz, 1H), 6.97(s, 1H), 6.78(d, J=8.0Hz, 1H), 3.35-3.32(m, 2H), 3.06(t, J=8.0Hz, 2H), 2.88(t, J=8.0Hz, 2H), 2.67(s, 6H), 1.80(s, 3H), 1.76(s, 3H).

### Biological evaluation of compounds.

The compounds of the present disclosure are evaluated in multiple assays to determine their biological activity. For example, the compounds of the present disclosure may be tested for their ability to inhibit various protein kinases of interest. Some tested compounds showed potent inhibitory activity against ALK kinase.

### (1) Evaluation of kinase inhibition

Compound preparation: The test compounds were dissolved in DMSO to make 20 mM stock solutions. The compounds were diluted in DMSO to 0.1 mM (a dilution with 100 times the final concentration) before use, and a 3-fold series gradient dilution with 11 concentrations was made. Dilutions with 4 times of the final concentration were prepared by diluting with buffer when dosing.

Kinase assay: after preparing the buffer solution, the enzyme was mixed with pre-diluted compounds with different concentrations, and placed at room temperature for 30 minutes in duplicate. The corresponding substrate and ATP were added thereto and reacted at room temperature for 60 minutes (both a negative and a positive control were set). After the reaction, antibody was added for detection, and after incubation at room temperature for 60 minutes, Envision detection was carried out, and data was collected. Data was analyzed and fit according to XI,fitS software. IC₅₀ was calculated by the following formula: (IC₅₀= [(ABS test- ABS begin)/(ABS control- ABS begin)] × 100). Wherein A represents IC₅₀ ≤ 5 nM, B represents IC₅₀ of 5-10 nM, C represents IC₅₀ of 10-50 nM, and D represents IC₅₀ of 50-100 nM.

The compounds of the present disclosure were tested in the above kinase inhibition assay, and were found to have potent activity against ALK and ALK[L1196M]. The results of the representative example compounds are summarized in Table 1 below.

**Table 1:**

| Example No. | IC₅₀(nM) | | Example No. | IC₅₀(nM) | |
|---|---|---|---|---|---|
| | ALK [WT] | ALK [L1196M] | | ALK [WT] | ALK [L1196M] |
| 1 | A | B | 11 | A | C |
| 2 | A | A | 12 | B | C |
| 3 | A | B | 13 | B | A |
| 4 | B | B | 14 | A | A |
| 5 | B | A | 15 | B | A |
| 6 | B | A | 16 | A | B |
| 7 | C | B | 17 | C | D |
| 8 | B | B | 18 | C | D |
| 9 | A | B | 19 | C | D |
| 10 | A | B | | | |

### (2) Cytotoxicity test

The in vitro anti-proliferative activity of the compounds of the present disclosure against three types of cells that are cultured in vitro was tested by CellTiter-Glo method. The experimental results show that the compounds of the present disclosure have potent inhibition on the in vitro proliferation of EML4-ALK and EML4-ALK L1196M mutant cells that cultured in vitro.

Cell lines: Ba/F3 parental; Ba/F3 [EML4-ALK] (From WuXi PharmaTech) and Ba/F3 [EML4-ALK L1196M] (From WuXi PharmaTech); wherein, Ba/F3 parental was cultured in RPMI1640 medium containing 10 ng/ml IL-3, 10% fetal bovine serum, 100 U/ml penicillin, and 100 µg/ml streptomycin, Ba/F3 [EMI,4-ALK] and Ba/F3 [EML4-ALK L1196M] were cultured in RPMI1640 medium containing 10% fetal bovine serum, 100 U/ml penicillin, and 100 µg/ml streptomycin.

Reagents and materials: RPMI-1640 (GIBCO, Cat. No. A10491-01); fetal bovine serum (GIBCO, Cat. No. 10099141); 0.25% trypsin-EDTA (GIBCO, Cat. No. 25200); penicillin-streptomycin, liquid (GIBCO, Cat. No. 15140-122); DMSO (Sigma, Cat. No. D2650); CellTiter-Glo Test Kit (Promega, Cat. No. G7572), and 96-well plate (Corning, Cat. No. 3365).

Specific experimental procedures:
1. The test compounds were dissolved in DMSO to make stock solutions and subjected to a series gradient dilution to give solutions with 10-fold working concentration.
2. The cells in the logarithmic growth phase were diluted with the culture solution to adjust to the specific cell concentration, 90 µl of cell suspension was added to the 96-well plate to make the cell density reach the specified concentration. The plate was incubated in a incubator at 37 °C, containing 5% carbon dioxide gas overnight.
3. 10 µl drug solutions were added to each well of the 96-well plate inoculated with cells. 10 concentrations of the compounds were tested starting from the highest concentration of 20 µM with a 3-fold series gradient dilution in duplicate.
4. After continuously cultured for 72 hours, the cells were detected by CellTiter-Glo for the cell viability. The dose-effect curve was made using GraphPad Prism software and IC₅₀ was calculated.

The compounds of the present disclosure were tested in the above-mentioned cytotoxicity assay, finding out that the compounds of the present disclosure have potent activity against Ba/F3 ALK and Ba/F3 ALK[L1196M], and superior selectivity over Ba/F3 parental cells. The results of the inhibition effects on the proliferation of cancer cells in vitro by the representative examples compounds of the present disclosure were summarized in Table 2 below, wherein, A represents IC₅₀ ≤ 50 nM, B represents IC₅₀ of 50-100 nM, C represents IC₅₀ of 100-400 nM, D represents IC₅₀ of 400-1000 nM, and E represents IC₅₀ ≥ 1000 nM.

**Table 2**

| Example No. | IC₅₀(nM) | | | Selectivity | |
|---|---|---|---|---|---|
| | parental (Ba/F3) | ALK (Ba/F3) | ALK[L1196M] (Ba/F3) | parental / ALK | parental / ALK[L1196M] |
| 1 | E | A | B | 50-100 | 0-50 |
| 2 | E | A | B | 50-100 | 0-50 |
| 3 | E | A | B | 100-200 | 50-100 |
| 4 | E | A | B | 50-100 | 0-50 |
| 5 | E | A | B | 100-200 | 50-100 |
| 6 | E | A | C | 200-400 | 50-100 |
| 7 | E | A | B | 200-400 | 50-100 |
| 8 | E | A | C | 200-400 | 50-100 |
| 9 | E | A | B | 200-400 | 50-100 |
| 10 | E | A | C | 100-200 | 50-100 |
| 11 | E | B | C | 100-200 | 50-100 |
| 12 | E | A | B | 111.30 | 0-50 |
| 13 | D | A | B | 0-50 | 0-50 |
| 14 | D | A | A | 50-100 | 0-50 |
| 15 | E | A | B | 50-100 | 0-50 |
| 16 | D | A | B | 50-100 | 0-50 |
| 17 | E | C | D | 50-100 | 0-50 |
| 18 | E | C | C | 50-100 | 0-50 |
| 19 | E | B | C | 100-200 | 50-100 |

The above is a further detailed description of the present disclosure in conjunction with specific embodiments. For ordinary artisan in the technical field to which the present disclosure belongs, without deviating from the concept of the present disclosure, various simple deductions or replacements may be made, which should be regarded as falling within the protection scope of the present disclosure.

## Claims

1. A compound of formula (I): 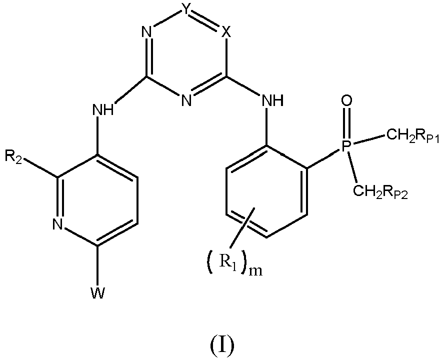 wherein,
X is selected from CRx or NR_{X1};
Y is selected from CR_{Y} or NR_{Y1};
m is selected from 0, 1, 2, 3 or 4;
R₁, R_{X} and R_{Y} are independently selected from:
1) H, halogen, -CN, -OH, -NO₂ or -NH₂;
2) C₁-C₆ alkyl, C₁-C₆ haloalkyl, C₁-C₆ alkoxy, C₁-C₆ haloalkoxy, C₁-C₆ alkylthio, C₁-C₆ alkylamino, C₁-C₆ alkanoyl, 3- to 7-membered carbocyclyl or 3- to 8-membered heterocyclyl; wherein the said group is optionally substituted by one or more Rₐ; or
3) Rxi and R_{Y1} are absent; or two adjacent substituents selected from Rx, Rxi, R_{Y} and R_{Y1}, or two adjacent Ri groups, together with the atoms to which they are attached may form a fused 5- to 7-membered ring, which contains 0 to 4 heteroatoms selected from N, O and S and may be substituted by one or more Rₐ;
R_{P1} and R_{P2} are each independently H;
R₂ is selected from C₁-C₆ alkoxy, C₁-C₆ haloalkoxy, C₂-C₆ alkenyloxy or 3- to 7-membered cycloalkyloxy; wherein the said group is optionally substituted by one or more Rₐ;
W is selected from -R₃, or -NR₃R₄, or 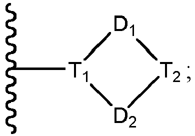
T₁ is selected from N or CR₃;
T₂ is selected from -NR₃, -CR₃(NR₃R₄), O or S;
D₁ and D₂ are independently selected from -(CR₃R₄)₁₋₃-;
each R₃ and R₄ is independently selected from H, C₁-C₆ alkyl, C₁-C₆ alkoxy, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S; wherein the said group is optionally substituted by one or more Rₐ;
each Rₐ is independently selected from H, -R, -NRR, or -OR, as long as the chemistry permits; wherein each R is independently H, C₁₋₆ alkyl, C₁₋₆ haloalkyl, C₁₋₆ alkoxy, C₁₋₆ haloalkoxy, 3- to 10-membered carbocyclyl, 3- to 10-membered heterocyclyl; two adjacent R could be taken together to form optionally substituted 3- to 10-membered carbocyclyl or optionally substituted 5- to 8-membered heterocyclyl;
or pharmaceutically acceptable salts, crystal forms, hydrates, solvates, stereoisomers or isotopic derivatives thereof.

2. The compound according to claim 1, which is the compound of formula (II): 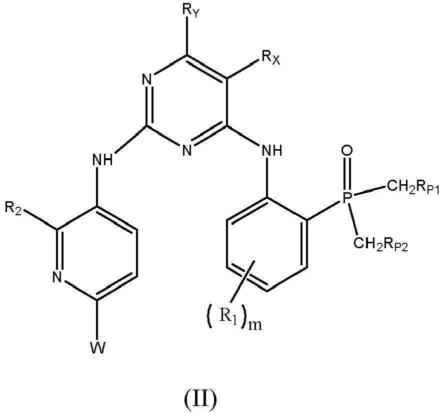 wherein,
R_{X} and R_{Y} are each independently selected from H, halogen or C₁-C₆ alkyl, or R_{X} and R_{Y} together with the carbon atom to which they are attached may form a fused 5- to 7-membered aryl ring, which contains 0 to 2 heteroatoms selected from N, O and S;
m is selected from 0, 1 or 2;
R₁ is selected from H, halogen or C₁-C₆ alkyl, wherein the C₁-C₆ alkyl is optionally substituted by one or more Rₐ;
R_{P1} and R_{P2} are each independently H;
R₂ is selected from C₁-C₆ alkoxy, C₁-C₆ haloalkoxy or 3- to 7-membered cycloalkyloxy; wherein the said group is optionally substituted by one or more Rₐ;
W is selected from -R₃, -NR₃R₄ or 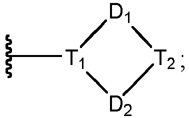
T₁ is selected from N or CR₃;
T₂ is selected from -NR₃, -CR₃(NR₃R₄), O or S;
D₁ and D₂ are independently selected from -(CR₃R₄)₁₋₃-;
R₃ and R₄ are independently selected from H, C₁-C₆ alkyl, C₁-C₆ alkoxy, C₁-C₆ alkylamino, 3- to 7-membered carbocyclyl or 5- to 8-membered heterocyclyl; or R₃ and R₄ together with the atom to which they are attached form a 3- to 7-membered ring, which contains 0 to 3 heteroatoms selected from N, O or S; wherein the said group is optionally substituted by one or more Rₐ;
each Rₐ is independently selected from H, -R, -NRR, or -OR, as long as the chemistry permits; wherein each R is independently H, C₁₋₆ alkyl, C₁₋₆ haloalkyl, C₁₋₆ alkoxy, C₁₋₆ haloalkoxy, 3- to 10-membered carbocyclyl, 3- to 10-membered heterocyclyl; two adjacent R could be taken together to form optionally substituted 3- to 10-membered carbocyclyl, or optionally substituted 5- to 8-membered heterocyclyl; or pharmaceutically acceptable salts, crystal forms, hydrates, solvates, stereoisomers or isotopic derivatives thereof.

3. The compound according to claim 2, which is selected from the compound of the following formulae: 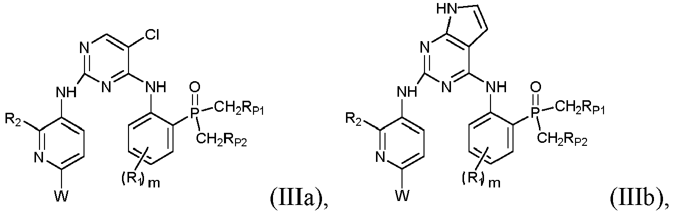 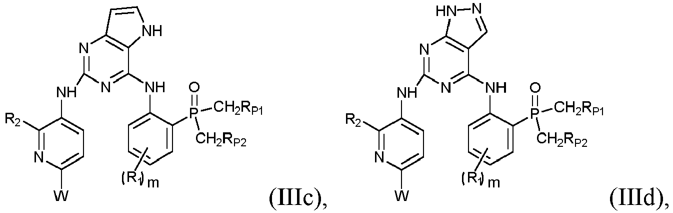 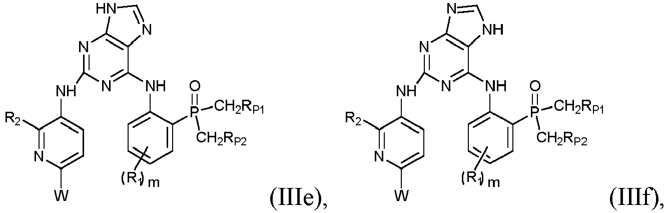 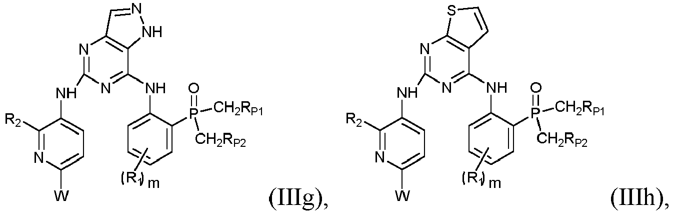 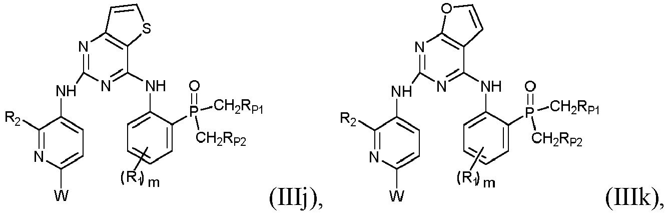 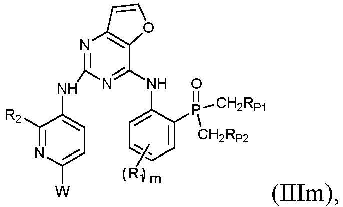
wherein, R₁, R_{P1}, R_{P2}, R₂, Wand m are as defined in claim 2;
or pharmaceutically acceptable salts, crystal forms, hydrates, solvates, stereoisomers or isotopic derivatives thereof.

4. The compound according to any one of claims 1 to 3, wherein the W is selected from the following groups: 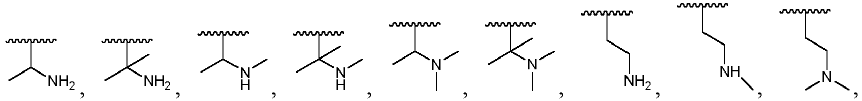 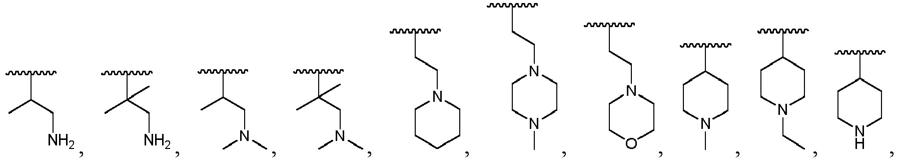 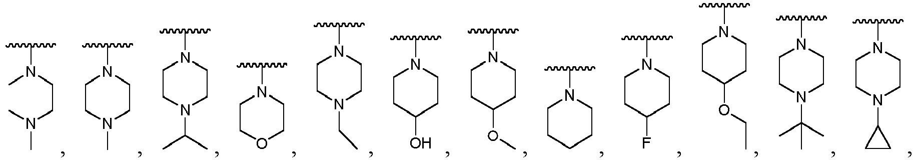 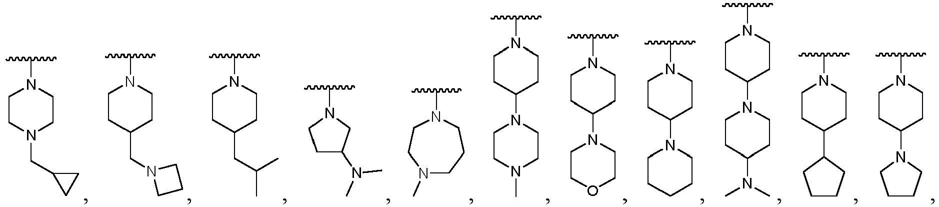 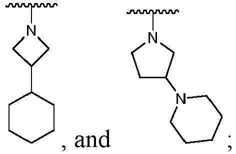 alternatively, W is selected from the following groups:

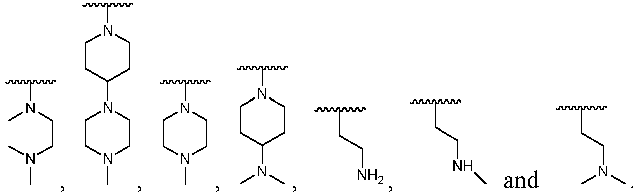

5. The compound according to any one of claims 1 to 4, wherein R₂ is selected from C₁-C₆ alkoxy or C₁-C₆ haloalkoxy;
alternatively, R₂ is selected from -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, -OCF₃, -OCHF₂, -OCH₂F, -OCH₂CF₃, -OCHF₂, -OCH₂F, -OCCl₃, -OCHCl₂, -OCH₂Cl, -OCH₂CCl₃, -OCHCl₂, -OCH₂Cl;
yet alternatively, R₂ is selected from -OCH₃, -OCH(CH₃)₂ or -OCH₂CF₃.

6. The compound according to any one of claims 1 to 5, wherein R₁ is selected from H, halogen or C₁-C₄ alkyl.

7. The compound according to claim 1, wherein the compound is selected from: 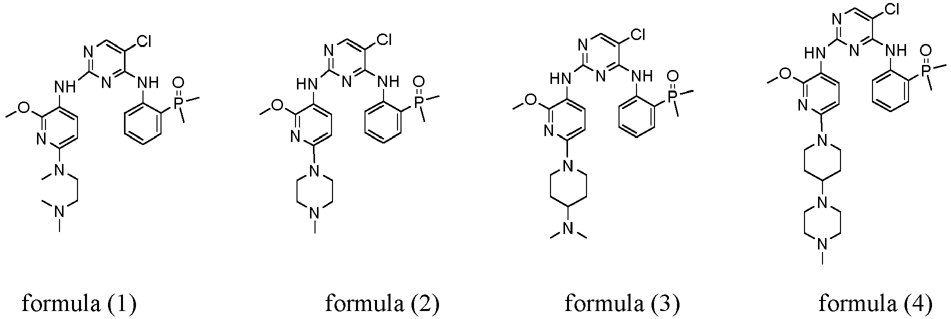 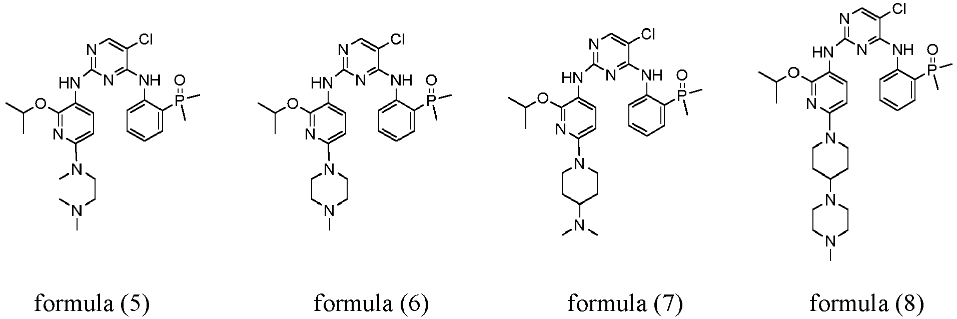 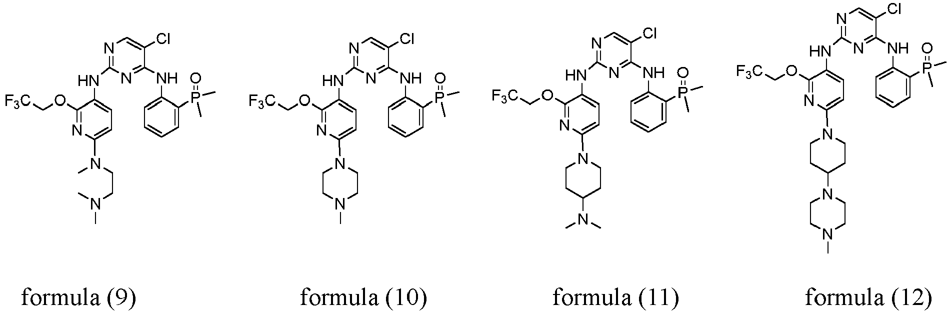 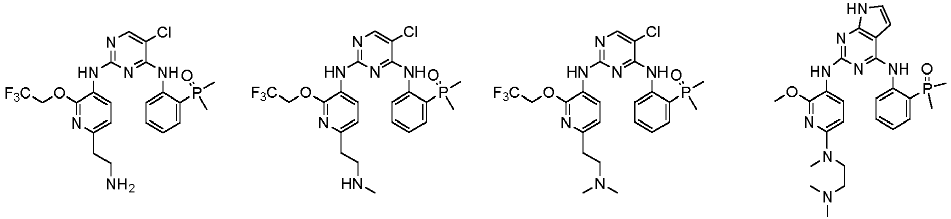 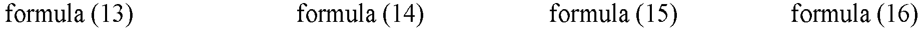 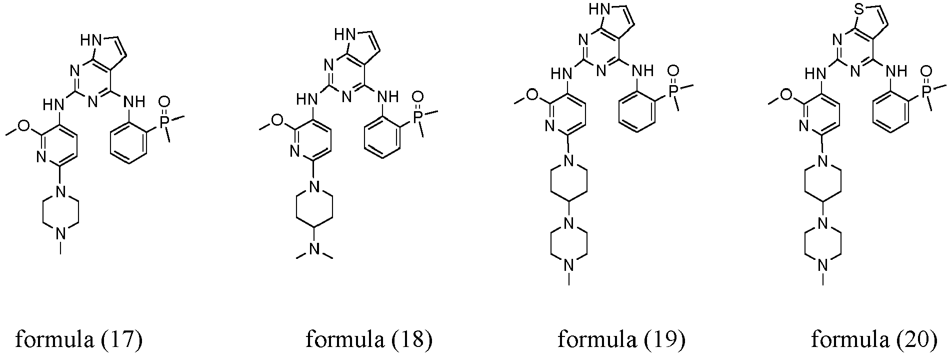 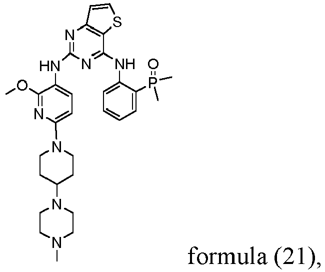 or pharmaceutically acceptable salts, crystal forms, hydrates, solvates, stereoisomers or isotopic derivatives thereof.

8. A pharmaceutical composition, comprising a compound according to any one of claims 1-7 or pharmaceutically acceptable salts, crystal forms, hydrates, solvates, stereoisomers or isotopic derivatives thereof, and pharmaceutically acceptable excipient(s).

9. A compound according to any one of claims 1-7 or pharmaceutically acceptable salts, crystal forms, hydrates, solvates, stereoisomers or isotopic derivatives thereof, or a pharmaceutical composition according to claim 8, for use in treating an ALK-mediated cancer.

10. The compound or composition for use according to claim 9, wherein the cancer is selected from non-small cell lung cancer, breast cancer, neuroma, esophageal cancer, soft tissue cancer, lymphoma or leukemia.

11. The compound or composition for use according to claim 10, wherein the non-small cell lung cancer is ALK positive non-small cell lung cancer; wherein the lymphoma is anaplastic large cell lymphoma.

## Patentansprüche

1. Verbindung der Formel (I): 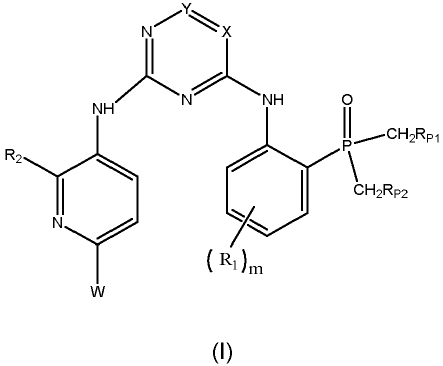 wobei
X aus CRx oder NRxi ausgewählt ist;
Y aus CR_{Y} oder NR_{Y1} ausgewählt ist;
m aus 0, 1, 2, 3 oder 4 ausgewählt ist;
R₁, Rx und R_{Y} unabhängig ausgewählt sind aus:
1) H, Halogen, -CN, -OH, -NO₂ oder -NH₂;
2) C₁-C₆-Alkyl, C₁-C₆-Haloalkyl, C₁-C₆-Alkoxy, C₁-C₆-Haloalkoxy, C₁-C₆-Alkylthio, C₁-C₆-Alkylamino, C₁-C₆-Alkanoyl, 3- bis 7-gliedrigem Carbocyclyl oder 3- bis 8-gliedrigem Heterocyclyl; wobei die Gruppe wahlweise mit einem oder mehreren Rₐ substituiert ist; oder
3) Rxi und R_{Y1} nicht vorhanden sind; oder zwei benachbarte Substituenten, die aus Rx, Rxi, R_{Y} und R_{Y1} ausgewählt sind, oder benachbarte R₁-Gruppen gemeinsam mit den Atomen, an die sie angebunden sind, einen kondensierten 5- bis 7-gliedrigen Ring bilden können, der 0 bis 4 Heteroatome enthält, die aus N, O und S ausgewählt sind, und die durch ein oder mehrere Rₐ substituiert sein können;
R_{P1} und R_{P2} jeweils unabhängig H sind;
R₂ aus C₁-C₆-Alkoxy, C₁-C₆-Haloalkoxy, C₂-C₆-Alkenyloxy oder 3- bis 7-gliedrigem Cycloalkyloxy ausgewählt ist; wobei die Gruppe wahlweise mit einem oder mehreren Rₐ substituiert ist;
W ausgewählt ist aus -R₃ oder -NR₃R₄ oder 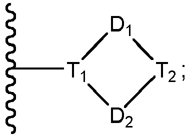
T₁ aus N oder CR₃ ausgewählt ist;
T₂ aus -NR₃, -CR₃(NR₃R₄), O oder S ausgewählt ist;
D₁ und D₂ unabhängig aus -(CR₃R₄)₁₋₃- ausgewählt sind;
R₃ und R₄ jeweils unabhängig aus H, C₁-C₆-Alkyl, C₁-C₆-Alkoxy, C₁-C₆-Alkylamino, 3- bis 7-gliedrigem Carbocyclyl oder 5- bis 8-gliedrigem Heterocyclyl ausgewählt sind; oder R₃ und R₄ zusammen mit dem Atom, an das sie angebunden sind, einen 3- bis 7-gliedrigem Ring bilden, der 0 bis 3 Heteroatome enthält, die aus N, O oder S ausgewählt sind; wobei die Gruppe wahlweise mit einem oder mehreren Rₐ substituiert ist;
Rₐ jeweils unabhängig aus H, -R, -NRR oder -OR ausgewählt ist, solange die Chemie dies zulässt; wobei R jeweils unabhängig H, C₁₋₆-Alkyl, C₁₋₆-Haloalkyl, C₁₋₆-Alkoxy, C₁₋₆-Haloalkoxy, 3- bis 10-gliedriges Carbocyclyl, 3- bis 10-gliedriges Heterocyclyl ist; zwei benachbarte R zusammengenommen werden könnten, um wahlweise substituiertes 3- bis 10-gliedriges Carbocyclyl oder wahlweise substituiertes 5- bis 8-gliedriges Heterocyclyl zu bilden;
oder pharmazeutisch unbedenkliche Salze, Kristallformen, Hydrate, Solvate, Stereoisomere oder isotope Derivate davon.

2. Verbindung nach Anspruch 1, bei der es sich um die Verbindung der Formel (II) handelt: 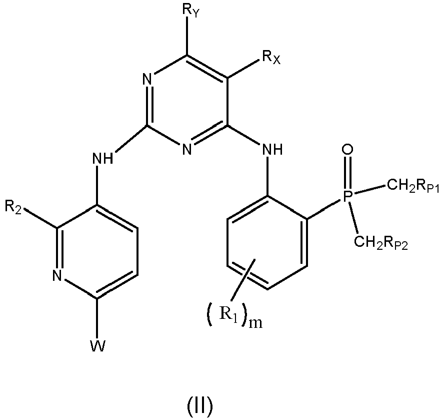 wobei
Rx und R_{Y} jeweils unabhängig aus H, Halogen oder C₁-C₆-Alkyl ausgewählt sind oder Rx und R_{Y} zusammen mit dem Kohlenstoffatom, an das sie angebunden sind, einen kondensierten 5- bis 7-gliedrigen Arylring bilden, der 0 bis 2 Heteroatome enthält, die aus N, O und S ausgewählt sind;
m aus 0, 1 oder 2 ausgewählt ist;
R₁ aus H, Halogen oder C₁-C₆-Alkyl ausgewählt ist, wobei das C₁-C₆-Alkyl wahlweise durch ein oder mehrere Rₐ substituiert ist;
R_{P1} und R_{P2} jeweils unabhängig H sind;
R₂ aus C₁-C₆-Alkoxy, C₁-C₆-Haloalkoxy oder 3- bis 7-gliedrigem Cycloalkyloxy ausgewählt ist; wobei die Gruppe wahlweise mit einem oder mehreren Rₐ substituiert ist;
W ausgewählt ist aus -R₃, -NR₃R₄ oder 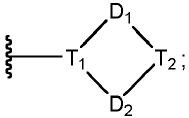
T₁ aus N oder CR₃ ausgewählt ist;
T₂ aus -NR₃, -CR₃(NR₃R₄), O oder S ausgewählt ist;
D₁ und D₂ unabhängig aus -(CR₃R₄)₁₋₃- ausgewählt sind;
R₃ und R₄ unabhängig aus H, C₁-C₆-Alkyl, C₁-C₆-Alkoxy, C₁-C₆-Alkylamino, 3- bis 7-gliedrigem Carbocyclyl oder 5- bis 8-gliedrigem Heterocyclyl ausgewählt sind; oder R₃ und R₄ zusammen mit dem Atom, an das sie angebunden sind, einen 3- bis 7-gliedrigem Ring bilden, der 0 bis 3 Heteroatome enthält, die aus N, O oder S ausgewählt sind; wobei die Gruppe wahlweise mit einem oder mehreren Rₐ substituiert ist;
Rₐ jeweils unabhängig aus H, -R, -NRR oder -OR ausgewählt ist, solange die Chemie dies zulässt; wobei R jeweils unabhängig H, C₁₋₆-Alkyl, C₁₋₆-Haloalkyl, C₁₋₆-Alkoxy, C₁₋₆-Haloalkoxy, 3- bis 10-gliedriges Carbocyclyl, 3- bis 10-gliedriges Heterocyclyl ist; zwei benachbarte R zusammengenommen werden könnten, um wahlweise substituiertes 3- bis 10-gliedriges Carbocyclyl oder wahlweise substituiertes 5- bis 8-gliedriges Heterocyclyl zu bilden; oder pharmazeutisch unbedenkliche Salze, Kristallformen, Hydrate, Solvate, Stereoisomere oder isotope Derivate davon.

3. Verbindung nach Anspruch 2, die aus der Verbindung der folgenden Formeln ausgewählt ist: 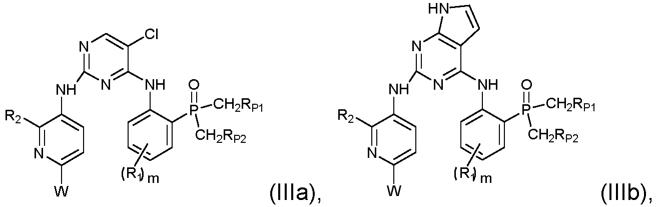 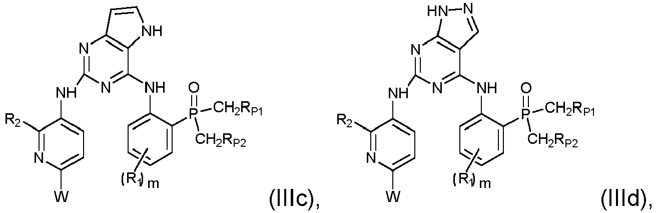 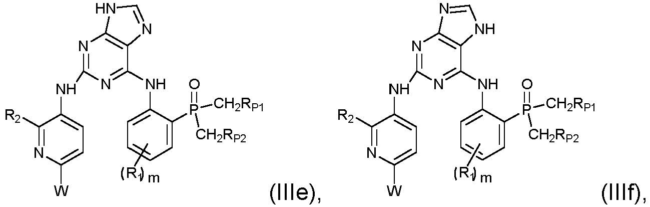 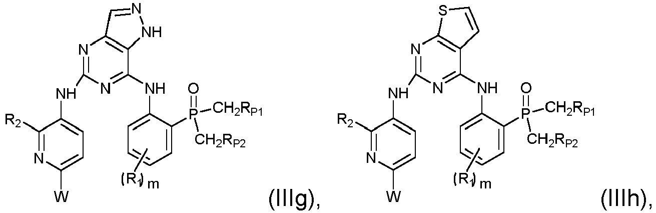 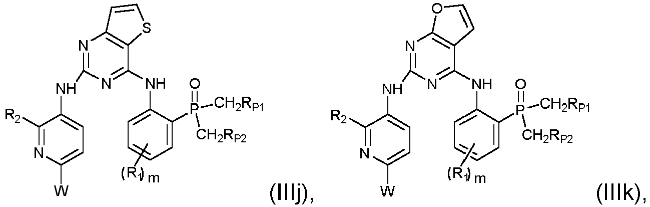 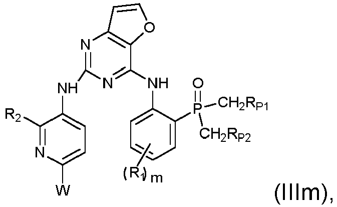
wobei R₁, R_{P1}, R_{P2}, R₂, W und m wie in Anspruch 2 definiert sind;
oder pharmazeutisch unbedenkliche Salze, Kristallformen, Hydrate, Solvate, Stereoisomere oder isotope Derivate davon.

4. Verbindung nach einem der Ansprüche 1 bis 3, wobei W aus den folgenden Gruppen ausgewählt ist: 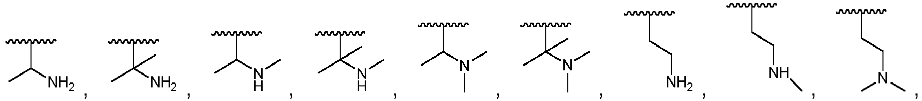 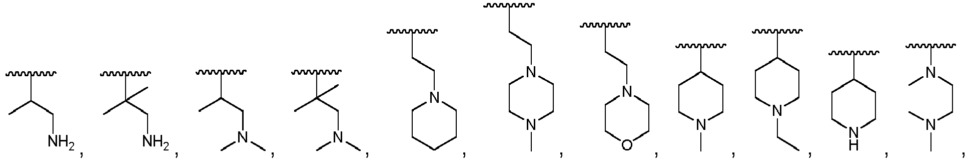 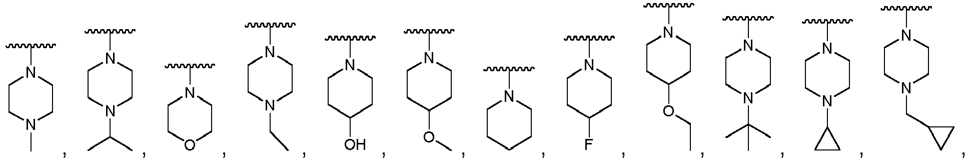 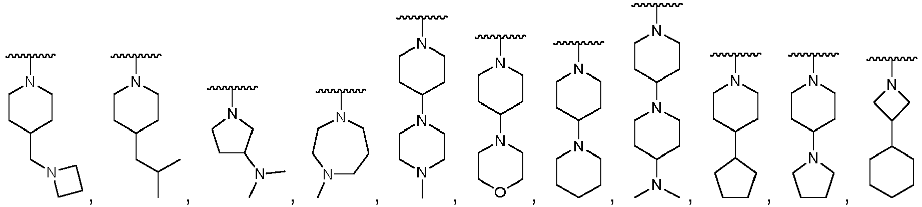 und 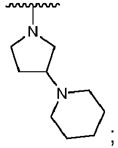 W alternativ aus den folgenden Gruppen ausgewählt ist:

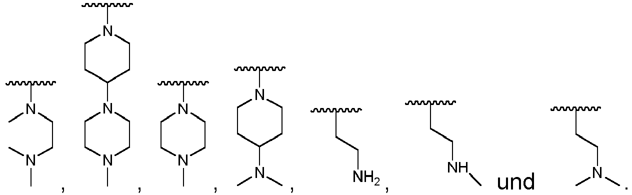

5. Verbindung nach einem der Ansprüche 1 bis 4, wobei R₂ aus C₁-C₆-Alkoxy oder C₁-C₆-Haloalkoxy ausgewählt ist;
alternativ ist R₂ aus -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, -OCF₃, -OCHF₂, -OCH₂F, -OCH₂CF₃, -OCHF₂, -OCH₂F, -OCCl₃, -OCHCl₂, -OCH₂Cl, -OCH₂CCl₃, -OCHCl₂, -OCH₂Cl ausgewählt;
ferner alternativ ist R₂ aus -OCH₃, -OCH(CH₃)₂ oder -OCH₂CF₃ ausgewählt.

6. Verbindung nach einem der Ansprüche 1 bis 5, wobei R₁ aus H, Halogen oder C₁-C₄-Alkyl ausgewählt ist.

7. Verbindung nach Anspruch 1, wobei die Verbindung ausgewählt ist aus: 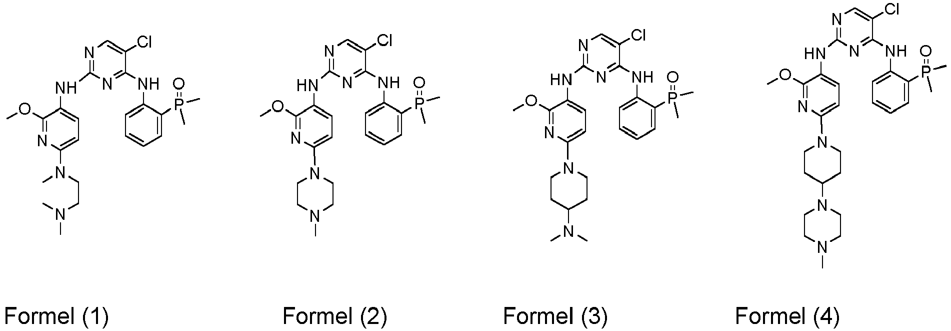 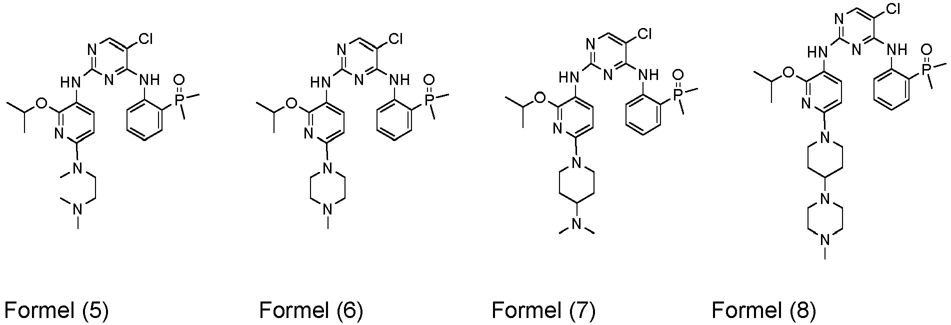 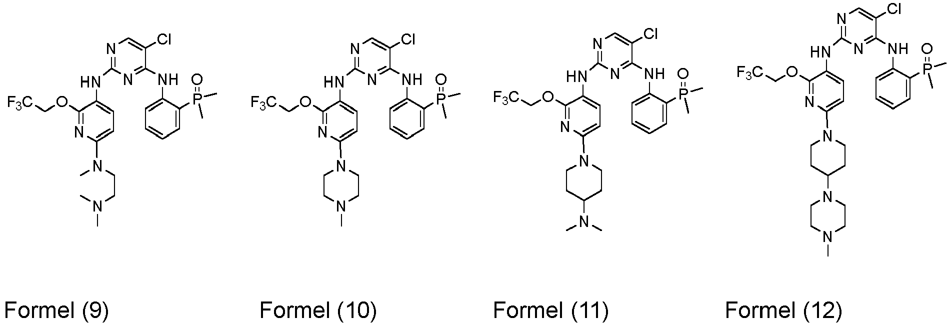 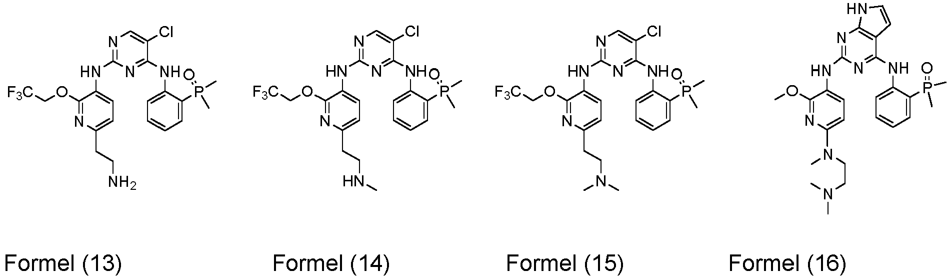 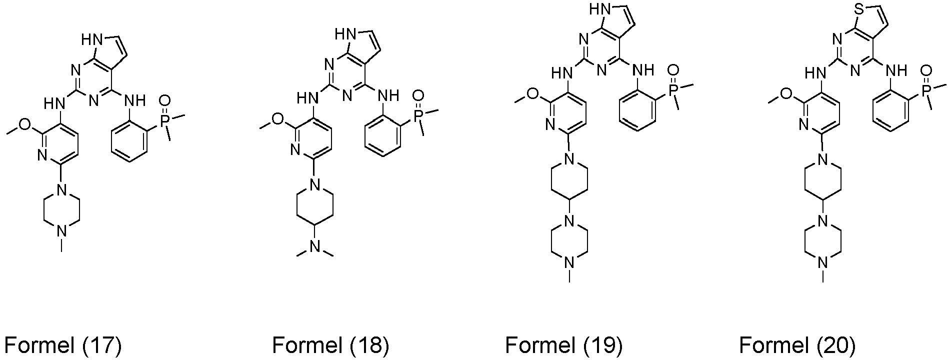 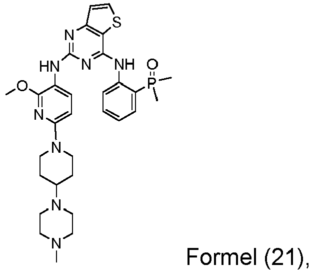 oder pharmazeutisch unbedenkliche Salze, Kristallformen, Hydrate, Solvate, Stereoisomere oder isotope Derivate davon.

8. Pharmazeutische Zusammensetzung, die eine Verbindung nach einem der Ansprüche 1 bis 7 oder pharmazeutisch unbedenkliche Salze, Kristallformen, Hydrate, Solvate, Stereoisomere oder isotope Derivate davon und einen oder mehrere pharmazeutisch unbedenkliche Hilfsstoffe umfasst.

9. Verbindung nach einem der Ansprüche 1 bis 7 oder pharmazeutisch unbedenkliche Salze, Kristallformen, Hydrate, Solvate, Stereoisomere oder isotope Derivate davon oder pharmazeutische Zusammensetzung nach Anspruch 8 zur Verwendung bei der Behandlung von ALK-vermitteltem Krebs.

10. Verbindung oder Zusammensetzung zur Verwendung nach Anspruch 9, wobei der Krebs aus nichtkleinzelligem Lungenkrebs, Brustkrebs, Neurom, Speiseröhrenkrebs, Weichgewebekrebs, Lymphom oder Leukämie ausgewählt ist.

11. Verbindung oder Zusammensetzung zur Verwendung nach Anspruch 10, wobei es sich bei dem nichtkleinzelligen Lungenkrebs um ALK-positiven nichtkleinzelligen Lungenkrebs handelt; wobei das Lymphom anaplastisches großzelliges Lymphom ist.

## Revendications

1. Composé de formule (I) : 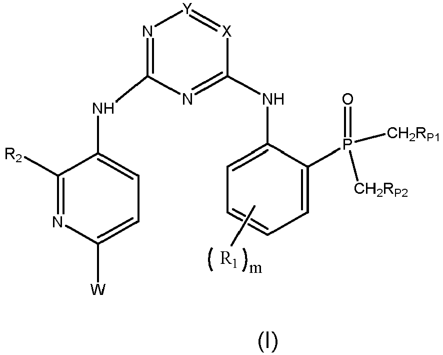 dans laquelle
X est choisi parmi CR_{X} ou NR_{X1} ;
Y est choisi parmi CR_{Y} ou NR_{Y1} ;
m est choisi parmi 0, 1, 2, 3 ou 4 ;
R₁, R_{X} et R_{Y} sont indépendamment choisis parmi :
1) H, un halogéno, -CN, -OH, -NO₂ ou -NH₂ ;
2) un alkyle en C₁ à C₆, un halogénoalkyle en C₁ à C₆, un alkoxy en C₁ à C₆, un halogénoalkoxy en C₁ à C₆, un alkylthio en C₁ à C₆, un alkylamino en C₁ à C₆, un alkanoyle en C₁ à C₆, un carbocyclyle tri- à heptagonal ou un hétérocyclyle tri- à octogonal ; ledit groupe étant facultativement substitué par un ou plusieurs Rₐ ; ou
3) R_{X1} et R_{Y1} sont absents ; ou deux substituants adjacents choisis parmi R_{X}, R_{X1}, R_{Y} et R_{Y1}, ou deux groupes R₁ adjacents, conjointement avec les atomes auxquels ils sont fixés, peuvent former un noyau penta- à heptagonal fusionné, qui contient 0 à 4 hétéroatomes choisis parmi N, O ou S et peut être substitué par un ou plusieurs Rₐ ;
R_{P1} et R_{P2} représentent chacun indépendamment H ;
R₂ est choisi parmi un alkoxy en C₁ à C₆, un halogénoalkoxy en C₁ à C₆, un alcényloxy en C₂ à C₆ ou un cycloalkyloxy tri- à heptagonal ; ledit groupe étant facultativement substitué par un ou plusieurs Rₐ ;
W est choisi parmi -R₃ ou -NR₃R₄, ou 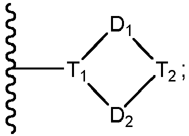
T₁ est choisi parmi N or CR₃ ;
T₂ est choisi parmi -NR₃, -CR₃(NR₃R₄), O ou S ;
D₁ et D₂ sont indépendamment choisis parmi -(CR₃R₄)₁₋₃- ;
R₃ et R₄ sont chacun indépendamment choisis parmi H, un alkyle en C₁ à C₆, un alkoxy en C₁ à C₆, un alkylamino en C₁ à C₆, un carbocyclyle tri- à heptagonal ou un hétérocyclyle tri- à octogonal ; ou R₃ et R₄, conjointement avec l'atome auquel ils sont fixés, forment un noyau tri- à heptagonal, qui contient 0 à 3 hétéroatomes choisis parmi N, O ou S ; ledit groupe étant facultativement substitué par un ou plusieurs Rₐ ;
chaque Rₐ est indépendamment choisi parmi H, -R, -NRR, ou -OR, tant que la chimie le permet ; chaque R représentant indépendamment H, un alkyle en C₁ à C₆, un halogénoalkyle en C₁ à C₆, un alkoxy en C₁ à C₆, un halogénoalkoxy en C₁ à C₆, un carbocyclyle tri- à décagonal ou un hétérocyclyle tri- à décagonal ; deux R adjacents pouvant être pris conjointement pour former un carbocyclyle tri- à décagonal facultativement substitué ou un hétérocyclyle tri- à pentagonal facultativement substitué ;
ou un de ses sels, formes cristallines, hydrates, solvates, stéréoisomères ou dérivés isotopiques, pharmaceutiquement acceptables.

2. Composé selon la revendication 1, qui est le composé de formule (II) : 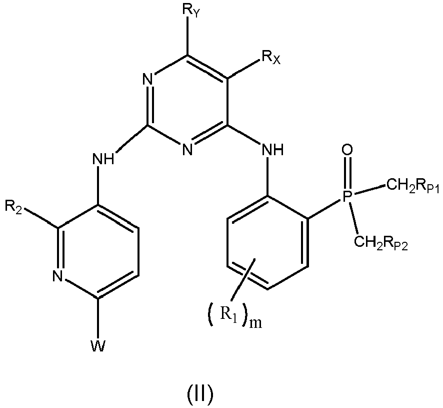 dans laquelle
R_{X} et R_{Y} sont chacun indépendamment choisis parmi H, un halogéno ou un alkyle en C₁ à C₆, ou R_{X} et R_{Y} conjointement avec l'atome de carbone auquel ils sont fixés, peuvent former un noyau arylique penta- à heptagonal, qui contient 0 à 2 hétéroatomes choisis parmi N, O ou S ;
m est choisi parmi 0, 1 ou 2 ;
R₁ est choisi parmi H, un halogéno ou un alkyle en C₁ à C₆, où l'alkyle en C₁ à C₆ est facultativement substitué par un ou plusieurs Rₐ ;
R_{P1} et R_{P2} représentent chacun indépendamment H ;
R₂ est choisi parmi un alkoxy en C₁ à C₆, un halogénoalkoxy en C₁ à C₆ ou un cycloalkyloxy tri- à heptagonal ; ledit groupe étant facultativement substitué par un ou plusieurs Ra ;
W est choisi parmi -R₃, -NR₃R₄ ou 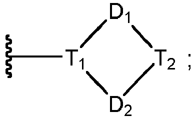
T₁ est choisi parmi N or CR₃ ;
T₂ est choisi parmi -NR₃, -CR₃(NR₃R₄), O ou S ;
D₁ et D₂ sont indépendamment choisis parmi -(CR₃R₄)₁₋₃- ;
R₃ et R₄ sont indépendamment choisis parmi H, un alkyle en C₁ à C₆, un alkoxy en C₁ à C₆, un alkylamino en C₁ à C₆, un carbocyclyle tri- à heptagonal ou un hétérocyclyle penta- à octogonal ; ou R₃ et R₄, conjointement avec l'atome auquel ils sont fixés, forment un noyau tri- à heptagonal, qui contient 0 à 3 hétéroatomes choisis parmi N, O ou S ; ledit groupe étant facultativement substitué par un ou plusieurs Rₐ ;
chaque Rₐ est indépendamment choisi parmi H, -R, -NRR, ou -OR, tant que la chimie le permet ; chaque R représentant indépendamment H, un alkyle en C₁ à C₆, un halogénoalkyle en C₁ à C₆, un alkoxy en C₁ à C₆, un halogénoalkoxy en C₁ à C₆, un carbocyclyle tri- à décagonal ou un hétérocyclyle tri- à décagonal ; deux R adjacents pouvant être pris conjointement pour former un carbocyclyle tri- à décagonal facultativement substitué ou un hétérocyclyle penta- à octogonal facultativement substitué ; ou un de ses sels, formes cristallines, hydrates, solvates, stéréoisomères ou dérivés isotopiques, pharmaceutiquement acceptables.

3. Composé selon la revendication 2, qui est choisi parmi le composé des formules suivantes : 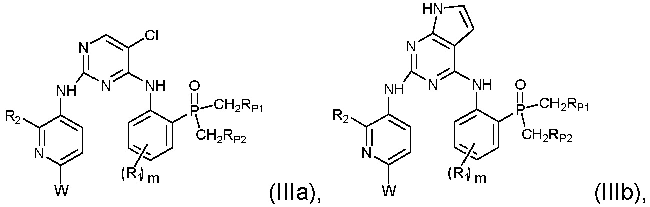 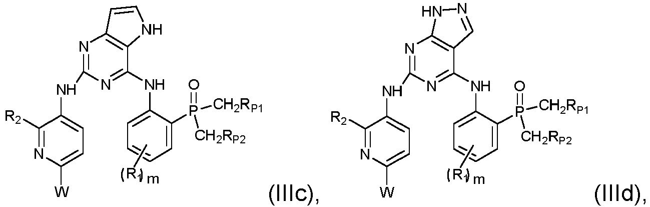 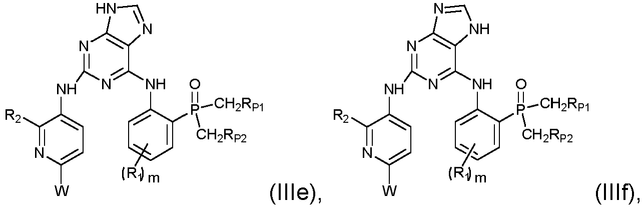 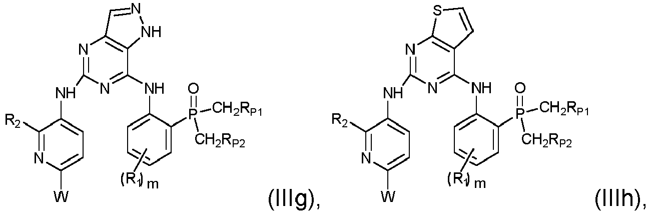 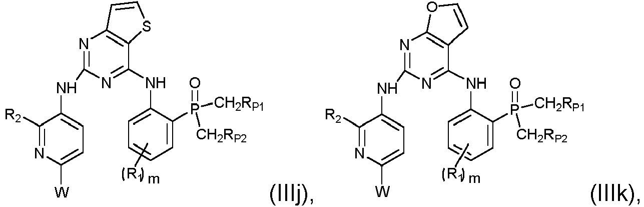 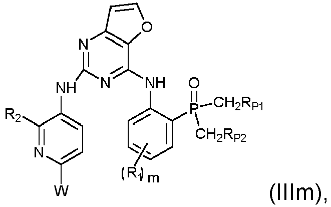
dans lesquelles, R₁, R_{P1}, R_{P2}, R₂, W et m sont tels que définie dans la revendication 2 ;
ou un de ses sels, formes cristallines, hydrates, solvates, stéréoisomères ou dérivés isotopiques, pharmaceutiquement acceptables.

4. Composé selon l'une quelconque des revendications 1 à 3, dans lequel le groupe W est choisi parmi les groupes suivants : 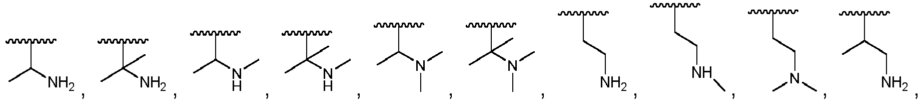 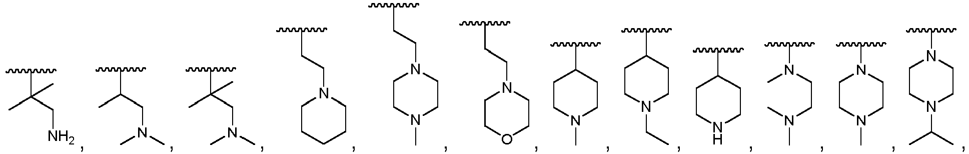 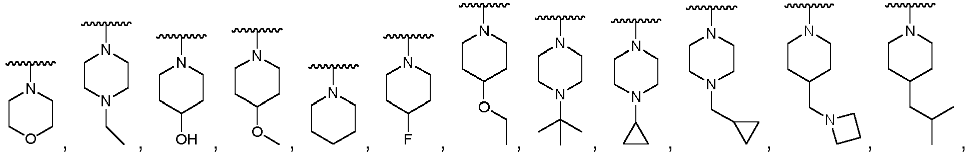 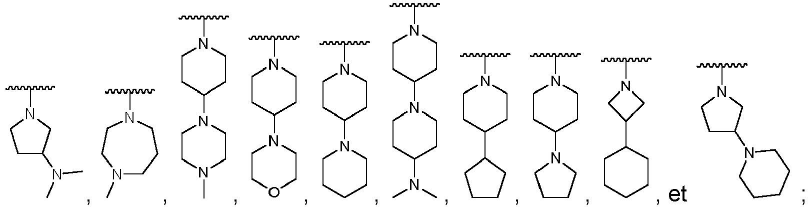 en variante, W est choisi parmi les groupes suivants :

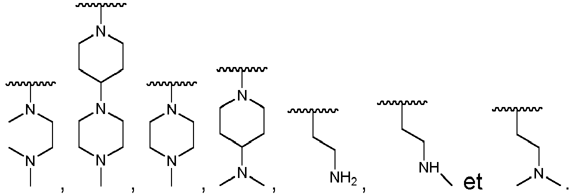

5. Composé selon l'une quelconque des revendications 1 à 4, dans R₂ est choisi parmi un alkoxy en C₁ à C₆ ou un halogénoalkoxy en C₁ à C₆ ;
en variante, R₂ est choisi parmi -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, -OCF₃, -OCHF₂, -OCH₂F, -OCH₂CF₃, -OCHF₂, -OCH₂F, -OCCl₃, -OCHCl₂, -OCH₂Cl, -OCH₂CCl₃, -OCHCl₂, -OCH₂Cl ;
en variante encore, R₂ est choisi parmi -OCH₃, -OCH(CH₃)₂ ou -OCH₂CF₃.

6. Composé selon l'une quelconque des revendications 1 à 5, dans R₁ est choisi parmi H, un halogéno ou un alkyle en C₁ à C₄.

7. Composé selon la revendication 1, où le composé est choisi parmi : 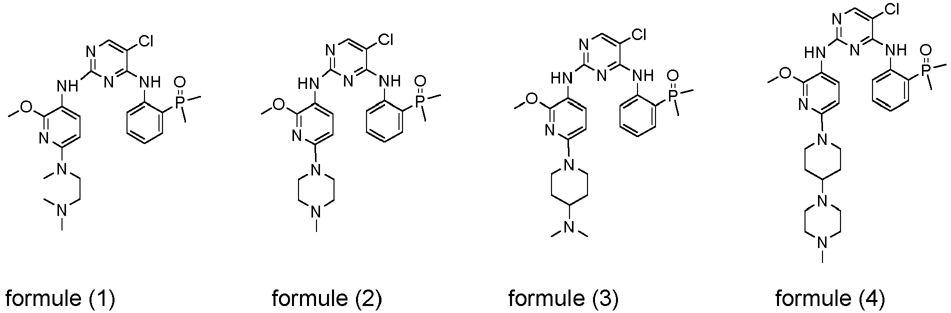 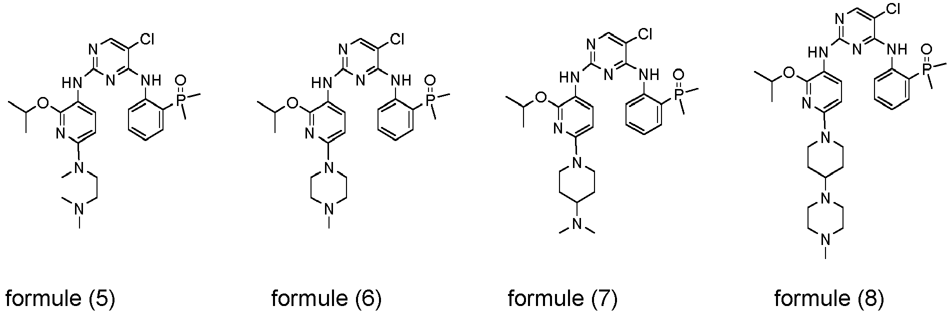 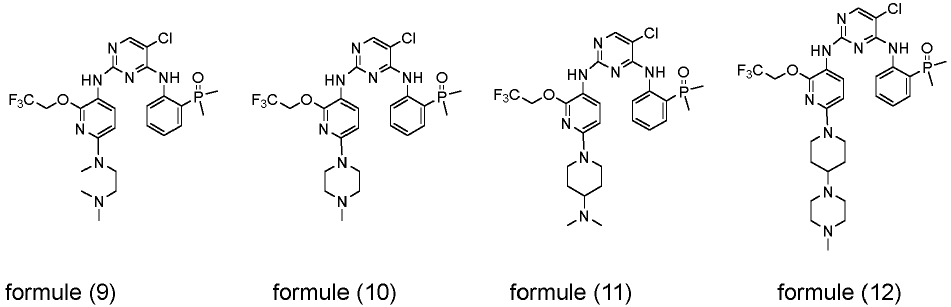 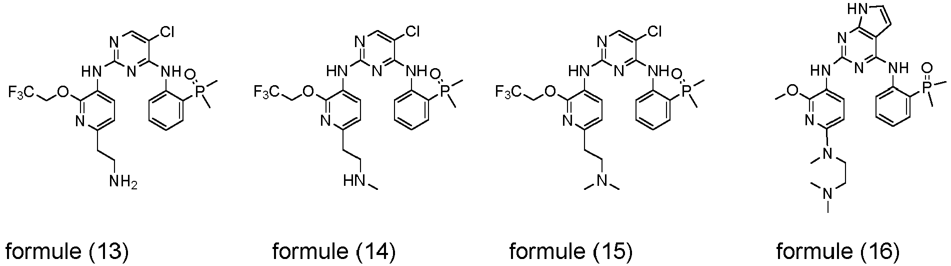 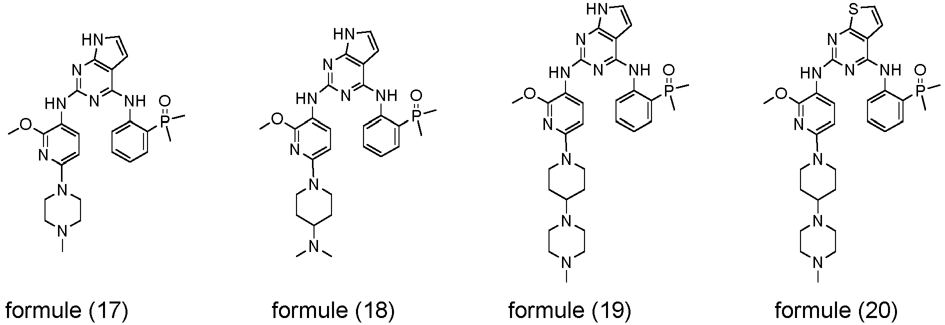 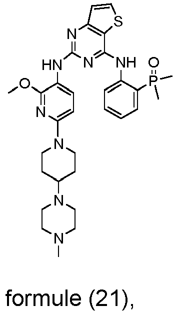 ou un de ses sels, formes cristallines, hydrates, solvates, stéréoisomères ou dérivés isotopiques, pharmaceutiquement acceptables.

8. Composition pharmaceutique, comprenant un composé selon l'une quelconque des revendications 1 à 7 ou un de ses sels, formes cristallines, hydrates, solvates, stéréoisomères ou dérivés isotopiques, pharmaceutiquement acceptables, et un ou plusieurs excipients pharmaceutiquement acceptables.

9. Composé selon l'une quelconque des revendications 1 à 7 ou un de ses sels, formes cristallines, hydrates, solvates, stéréoisomères ou dérivés isotopiques, pharmaceutiquement acceptables, ou composition pharmaceutique selon la revendication 8, pour une utilisation dans le traitement d'un cancer à médiation parALK.

10. Composé ou composition pour une utilisation selon la revendication 9, le cancer étant choisi parmi un cancer du poumon non à petites cellules, un cancer du sein, un neurinome, un cancer de l'oesophage, un cancer des tissus mous, un lymphome ou une leucémie.

11. Composé ou composition pour une utilisation selon la revendication 10, le cancer du poumon non à petites cellules étant un cancer du poumon non à petites cellules ALK positif ; le lymphome étant un lymphome anaplasique à grandes cellules.
